# EUROPEAN PATENT APPLICATION

(11) **EP 4 488 260 A1**
(43) Date of publication of application: **08.01.2025**
(21) Application number: 23762973.8
(22) Date of filing: 02.03.2023
(51) Int. Cl.: C07C 225/20, C07C 225/00, A61K 31/215, C07C 221/00, C07C 253/30, A61K 31/135, A61K 31/277, A61P 25/24, A61P 25/00, C07D 307/68

(54) **NEW AMINOKETONE COMPOUND, AND PREPARATION METHOD THEREFOR AND USE THEREOF**

(30) Priority: 04.03.2022 CN 202210210856
(71) Applicant: Shanghai Zhigen Pharmaceutical & Technology Co. Ltd., Shanghai 201318 (CN)
(72) Inventor: XUE, Tao, Shanghai 201318 (CN); CHEN, Yilang, Shanghai 201318 (CN)
(74) Representative: Mewburn Ellis LLP
(86) International application number: PCT/CN2023/079389
(87) International publication number: WO 2023/165575

(57) **Abstract**

The present invention relates to a new aminoketone compound, and a preparation method therefor and the use thereof. Specifically, the compound of the present invention has a structure as shown in formula (I), wherein the definition of each group and substituent are as described in the description. Also disclosed in the present invention are a preparation method for the compound and the use thereof against depression.

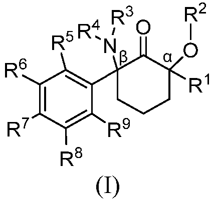

## Description

### TECHNICAL FIELD

The present invention relates to the field of pharmaceuticals, specifically to novel aminoketone compound, and preparation method therefor and use thereof.

### BACKGROUND

Depression usually refers to mood disorders, an affective psychosis characterized by low mood, sadness, despair and frustration, accompanied by symptoms such as sleep disorders, anxiety and physical discomfort, etc. As a common and serious mental illness, it seriously affects the patient's ability to work, quality of life, and can even be life-threatening. With the exacerbation of many stress factors, depression has become a common and highly incidence disease in modern society, and its incidence rate is rapidly climbing year by year. According to the World Health Organization, there are about 350 million patients with depression globally, and depression is currently the fourth largest disease burden in the world and one of the main causes of functional disability in patients, and it is expected that the disease burden of depression will rise to the first place globally by 2030.

Currently, the main antidepressant treatment remains pharmacologic. Antidepressants have been on the market for more than 60 years, ranging from tricyclic antidepressants (TCAs, promethazine, clomipramine, amitriptyline, etc.) and monoamine oxidase inhibitors (MAOIs, moclobemide, etc.), to selective 5-HT reuptake inhibitors (SSRIs, fluoxetine, paroxetine, citalopram, sertraline, fluvoxamine, etc.), s elective norepinephrine (NE) reuptake inhibitors (NaRIs, reboxetine), 5-HT and NE dual reuptake inhibitors (SNRIs, venlafaxine, duloxetine, etc.), and NE and specific 5-HT dual reuptake inhibitors (NaSSAs, mirtazapine), etc., play an important role in the prevention and treatment of depression. However, these medications also have serious drawbacks: 1. slow onset of action, usually taking 2-4 weeks or even longer to exert a more pronounced efficacy; 2. low treatment response rate, with only about 1/3 of patients responding to the first treatment medication, and about 2/3 responding only after trying multiple antidepressant medications. In particular, for suicidal patients with major depressive disorder (MDD), the shortcomings of the slow onset of action of all existing antidepressants (requiring 2-4 weeks of medication time before significant efficacy) are to the disadvantage of patients with a high risk of suicide.

Ketamine (ketamine) has been in clinical use as a traditional anesthetic for more than fifty years. However, in later studies (Arch Gen Psychiatry, 2006; 63(8): 856-864), it was found that a subanesthetic dose of ketamine had a rapid (a few hours), significant, and relatively long-lasting (about a week) antidepressant effect in patients with treatment-refractory depression (TRD), and it began to gain clinical attention. Currently, the FDA has approved Johnson & Johnson's esketamine nasal spray (esketamine, spravato) for the treatment of treatment resistant depression (TRD) and major depression with suicidal thoughts/behavior, ushering in a new era of rapid treatment of depression.

Although ketamine has shown rapid and sustained antidepressant effects, there are some problems that may affect its clinical use. 1. side effects: including psychotic-like, dissociative side effects, increased blood pressure, respiratory depression and other side effects; long-term application may cause urinary tract dysfunction and even renal failure; 2. oral bioavailability is low, difficult to give the drug orally, and is currently given as a nasal spray; 3. causing euphoria, hallucinations, addictive; these side effects of ketamine lead to the need for its use in medical institutions with the appropriate qualifications and under regulatory conditions, causing many inconveniences to the daily life of patients.

In May 2016, Zanos et al. published research (Nature, 2016, 533, 481-486) showing that the rapid and sustained antidepressant effects of ketamine are primarily derived from its metabolite (2R,6R)-HNK, rather than ketamine itself. (2R,6R)-HNK is currently in clinical phase I studies.

(2R,6R)-HNK has the highly desirable characteristic of being able to treat depression with rapid onset of action, but there are still many shortcomings: for example, the compound has weak antidepressant activity; the brain tissue exposure is low, and the brain/plasma ratio is low; due to its chemical structure, (2R,6R)-HNK is not chemically stable and is prone to degradation and impurities, which makes it more demanding for storage conditions. It is currently administered intravenously during clinical studies, resulting in poor patient compliance.

Therefore, there is an urgent clinical need for novel antidepressants that are fast-acting, efficacious, have high brain exposure, high brain/plasma ratios, safe, chemically stable, and can be administered orally.

### SUMMARY OF THE INVENTION

The purpose of the present invention is to provide a class of compounds that are structurally novel, have rapid and long-lasting antidepressant activity, a high brain/plasma ratio, high safety, and good chemical stability.

The first aspect of the present invention provided is a compound shown in formula (I), or a tautomer, an enantiomer, a diastereoisomer, a racemate, or a mixture thereof, or a pharmaceutically acceptable salt thereof, 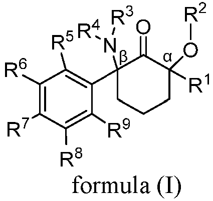 wherein,
R¹ is selected from the group consisting of deuterium and C₁-C₆ alkyl;
R², R³ and R⁴ are each independently selected from the group consisting of hydrogen, C₁-C₆ alkyl, deuterated C₁-C₆ alkyl and -(C=O)-C₁-C₆ alkyl;
R⁵, R⁶, R⁷, R⁸ and R⁹ are each independently selected from the group consisting of hydrogen, C₁-C₆ alkyl, halogenated C₁-C₆ alkyl, C₁-C₆ alkoxy, halogenated C₁-C₆ alkoxy, halogen, nitro and cyano; and at least 2 of R⁵, R⁶, R⁷, R⁸ and R⁹ are hydrogen;
the stereo configuration of the α-position or β-position carbon atom is each independently selected from the group consisting of: R, S and (R, S).

In another preferred embodiment, the additional condition is that R⁵ and/or R⁹ are not Cl when R⁶, R⁷ and R⁸ are hydrogen.

In another preferred embodiment, the compound is a compound according to formula (I-A), (I-B), (I-C), (I-D), (I-E), (I-F), or (I-G):

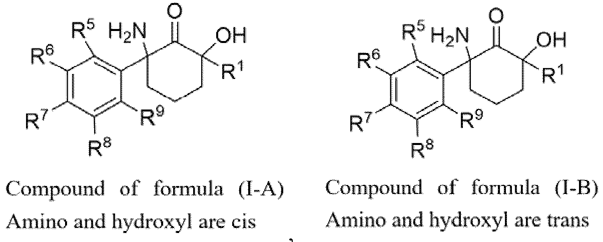

In formula (I-A), (I-B), R¹, R⁵, R⁶, R⁷, R⁸ and R⁹ are defined above;

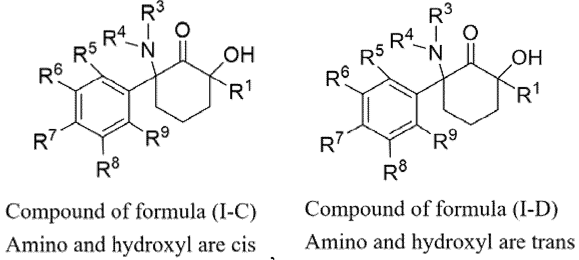

In formula (I-C), (I-D), R¹, R⁵, R⁶, R⁷, R⁸ and R⁹ are defined above, R³ and R⁴ are independently hydrogen, C₁-C₆ alkyl, or deuterated C₁-C₆ alkyl, and R³ and R⁴ are not both hydrogen at the same time;

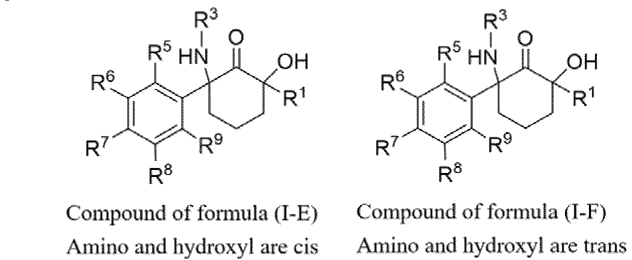

In formula (I-E), (I-F), R¹, R⁵, R⁶, R⁷, R⁸ and R⁹ are as defined above and R³ is - (C=O)-C₁-C₆ alkyl;

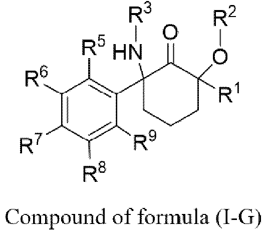

In formula (I-G), R¹, R⁵, R⁶, R⁷, R⁸ and R⁹ are defined above; R² is selected from the group consisting of C₁-C₆ alkyl, deuterated C₁-C₆ alkyl and -(C=O)-Ci-Ce alkyl; R³ is selected from the group consisting of hydrogen, C₁-C₆ alkyl and deuterated C₁-C₆ alkyl.

In another preferred embodiment, 2, 3 or 4 of R⁵, R⁶, R⁷, R⁸ and R⁹ are hydrogen.

In another preferred embodiment, R¹ is deuterium or methyl.

In another preferred embodiment, R², R³ and R⁴ are independently hydrogen, methyl, deuteromethyl, ethyl, isopropyl, acetyl or propionyl.

In another preferred embodiment, R² is hydrogen; R³ is hydrogen;

R⁴ is methyl, deuteromethyl, ethyl, isopropyl, acetyl or propionyl.

In another preferred embodiment, R⁵, R⁶, R⁷, R⁸ and R⁹ are independently hydrogen, fluorine, chlorine, methoxy, trifluoromethyl, trifluoromethoxy or cyano.

In another preferred embodiment, the compound is a compound shown in formula (I-C) or (I-D): 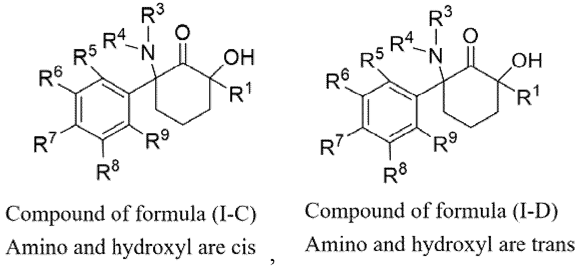 wherein,
R¹, R⁵, R⁶, R⁷, R⁸ and R⁹ are defined above;
R³ is hydrogen;
R⁴ is selected from the group consisting of: C₁-C₆ alkyl, deuterated C₁-C₆ alkyl and -(C=O)-C₁-C₆ alkyl.

In another preferred embodiment, the compound is selected from the group consisting of:

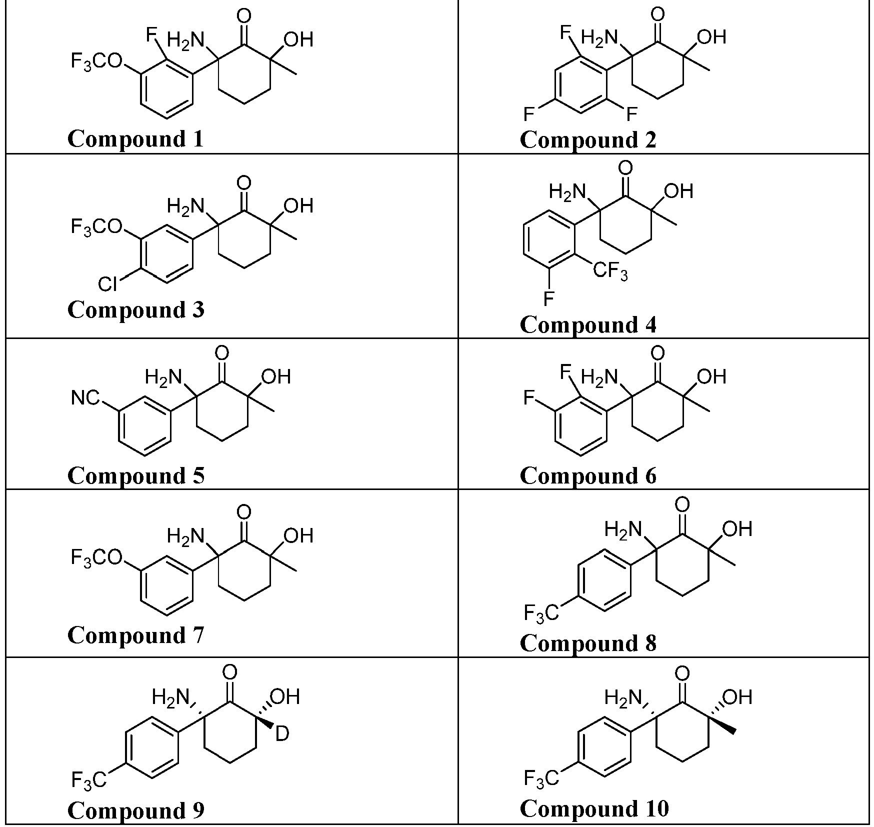

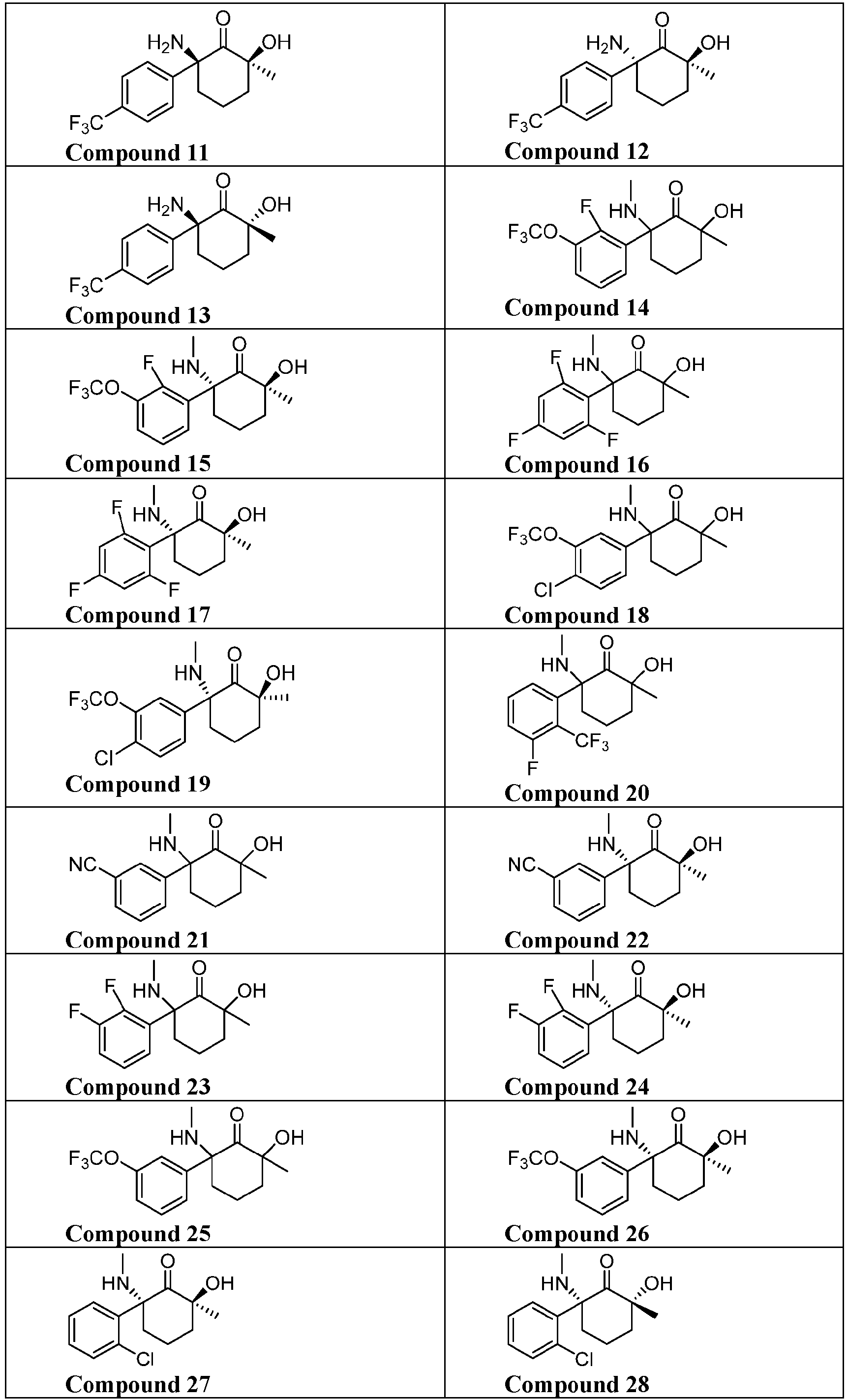

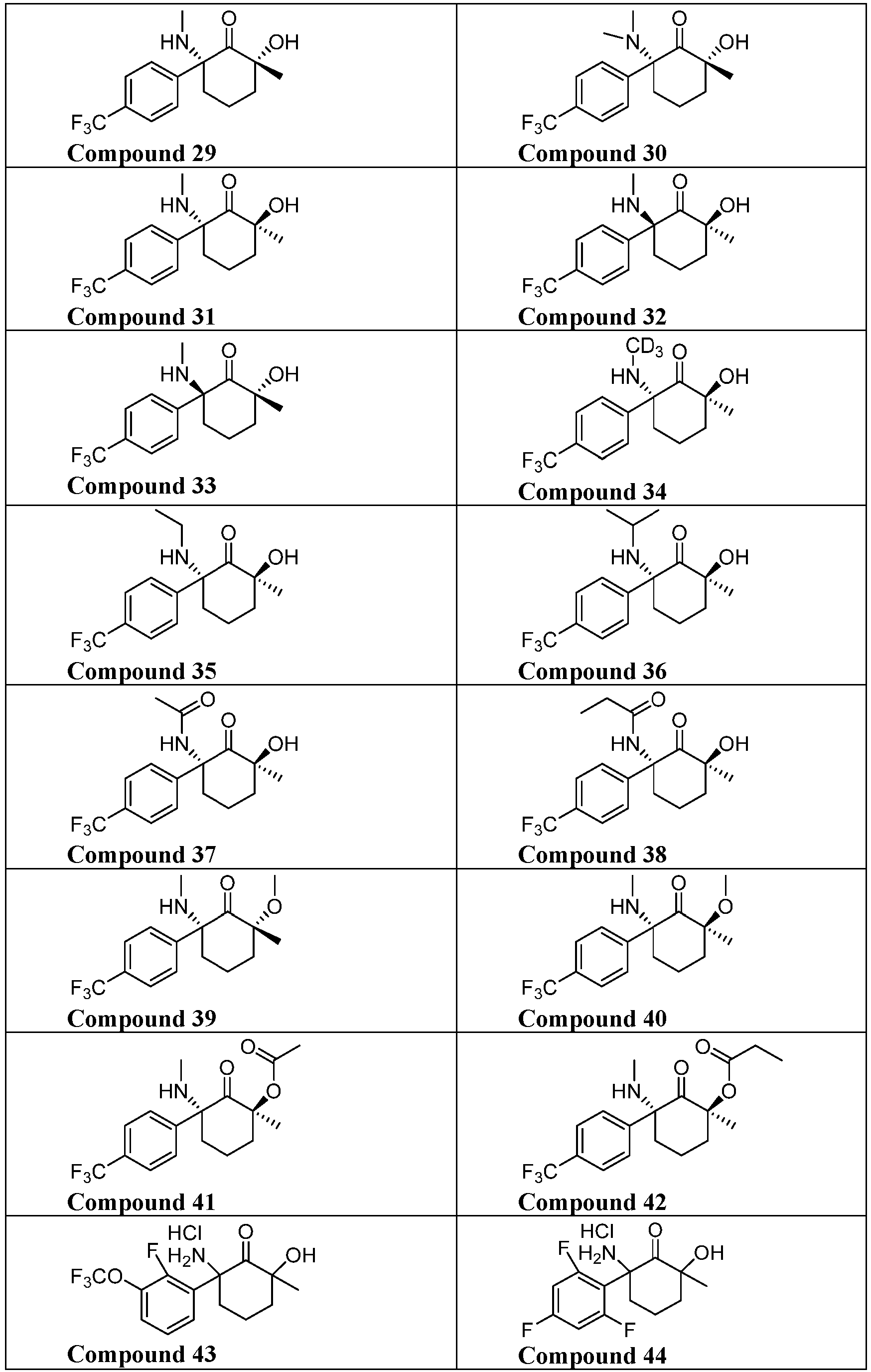

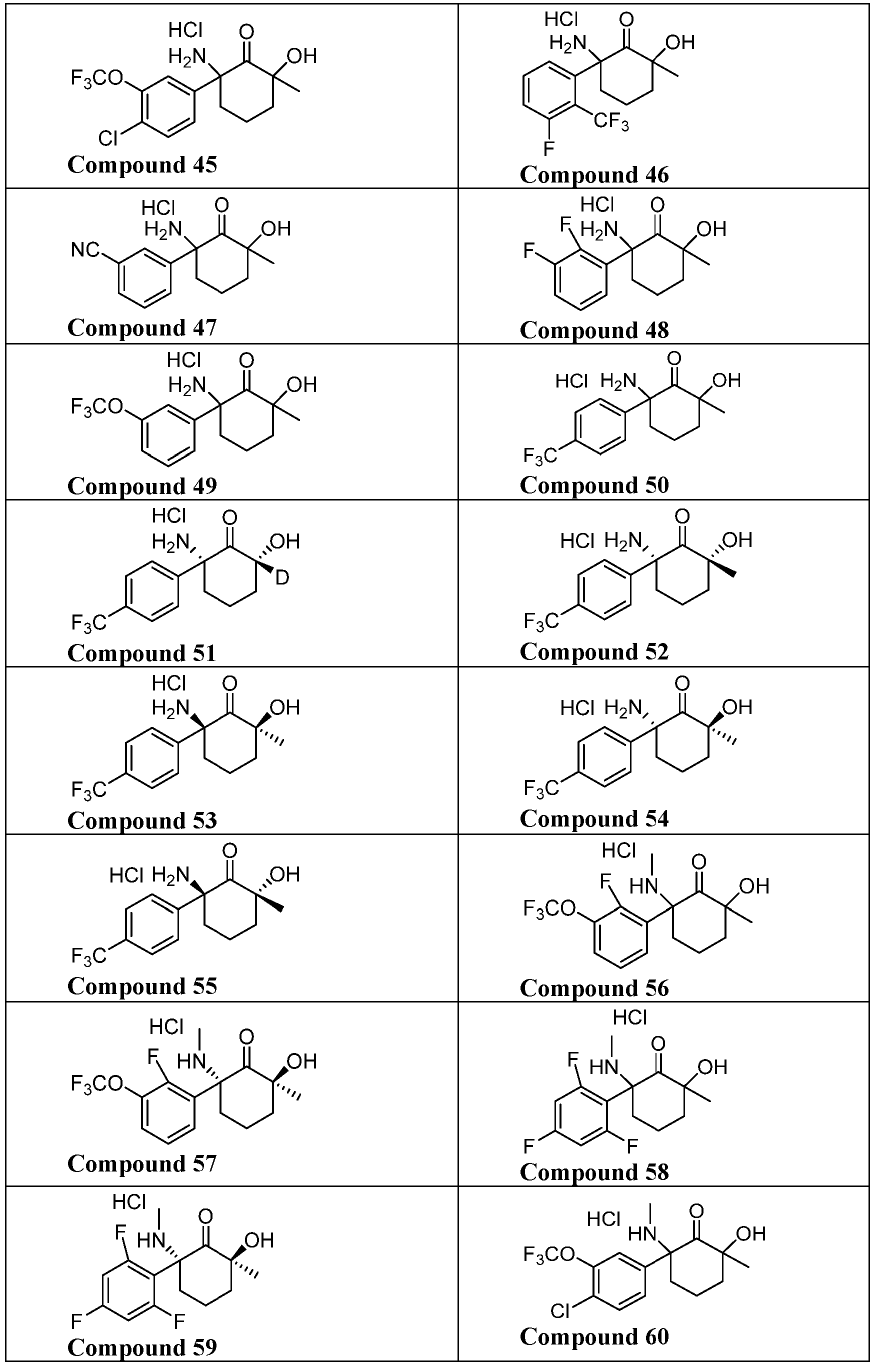

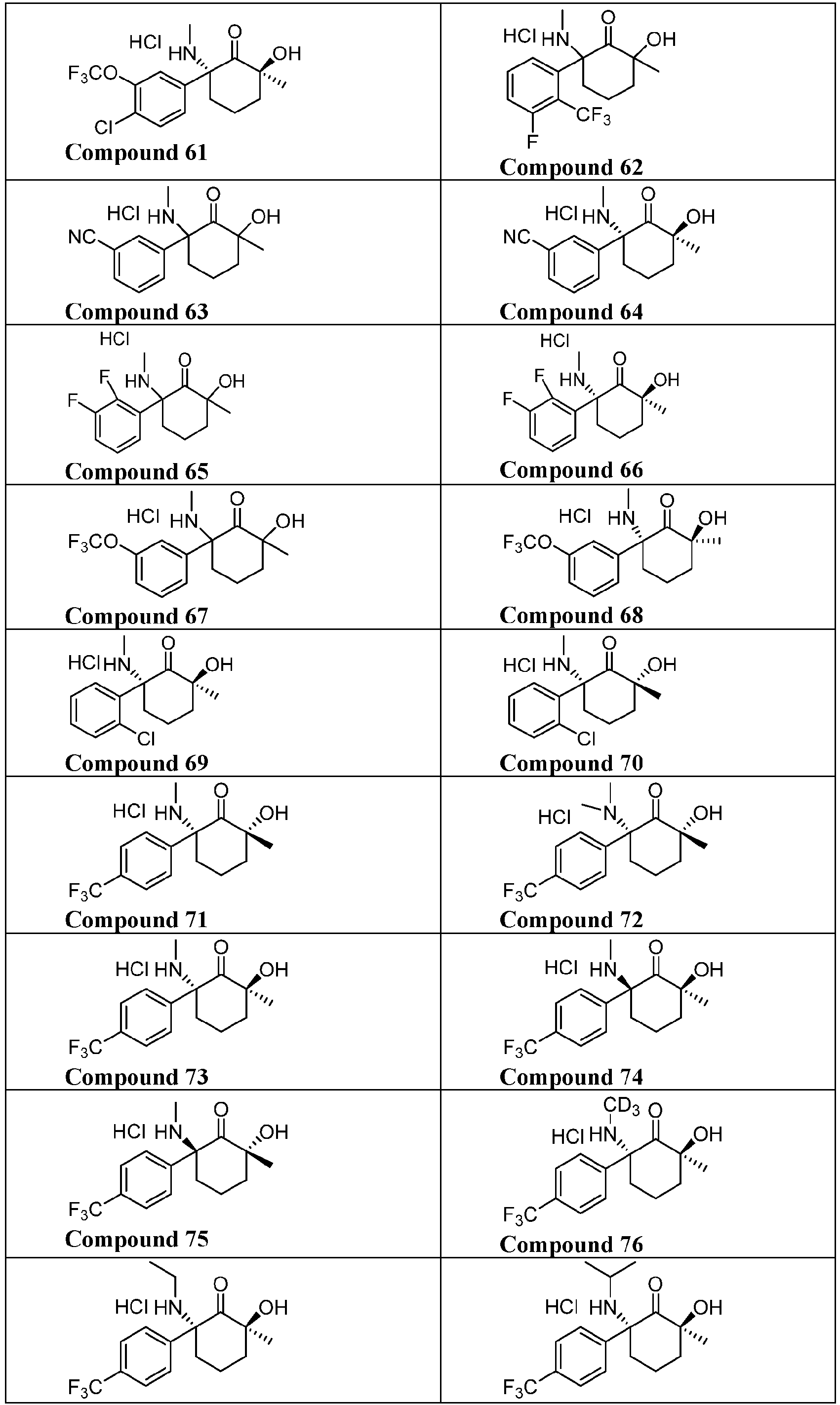

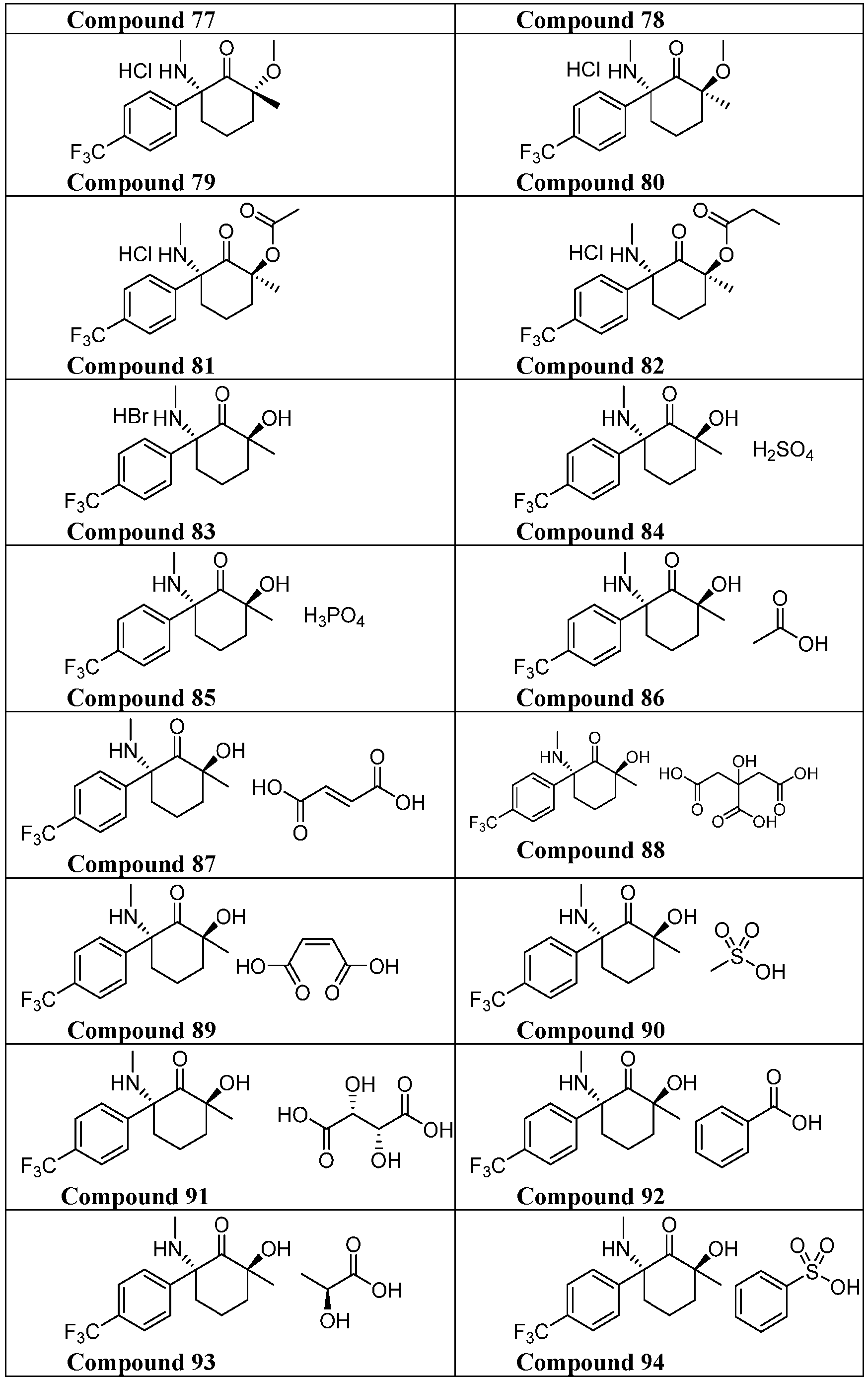

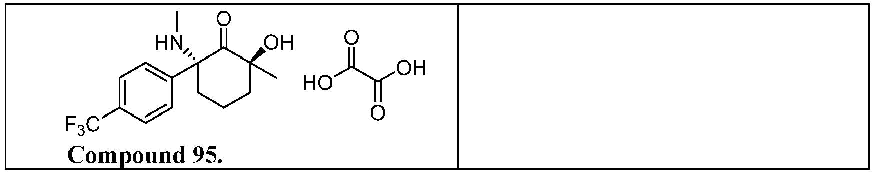

The second aspect of the present invention, provided is a pharmaceutical composition comprising:
1) one or more safe and effective amounts of the compound of the first aspect of the present invention, or a tautomer, an enantiomer, a diastereoisomer, a racemate, or a mixture thereof, or a pharmaceutically acceptable salt thereof; and
2) a pharmaceutically acceptable carrier or excipient.

In another preferred embodiment, the pharmaceutical composition is an oral formulation.

The third aspect of the present invention provided is a method for the preparation of the compound of the first aspect of the present invention, or a tautomer, an enantiomer, a diastereoisomer, a racemate or a mixture thereof, or a pharmaceutically acceptable salt thereof,
(1) the method comprises steps of: 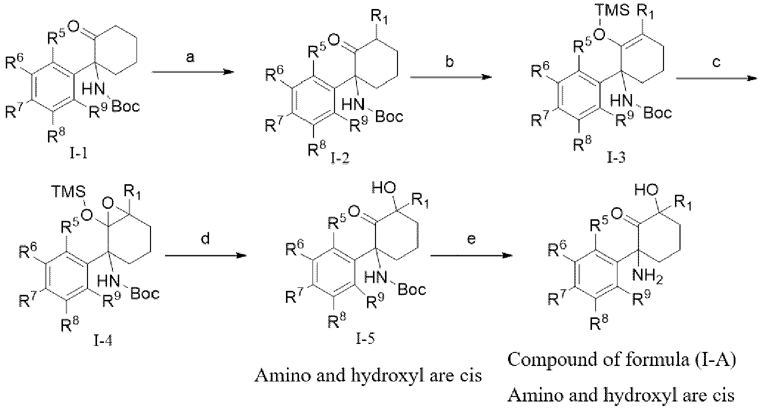 wherein, R¹, R⁵, R⁶, R⁷, R⁸ and R⁹ are defined according to the first aspect of the present invention;
   a. in a non-protonic solvent, compound I-1 reacts with a deuterated reagent or an alkylating reagent to form compound I-2;
   b. in a non-protonic solvent, compound I-2 reacts with a trimethylchlorosilane to form compound I-3;
   c. in a non-protonic solvent, compound I-3 and an oxidizing agent undergo oxidation to form compound I-4;
   d. in a non-protonic solvent, compound I-4 deprotects the trimethylsilyl protecting group to form compound I-5;
   e. in a polar non-protonic solvent, compound I-5 deprotects the tert-butoxycarbonyl protecting group to form compound I-A;
or (2) the method comprises steps of: 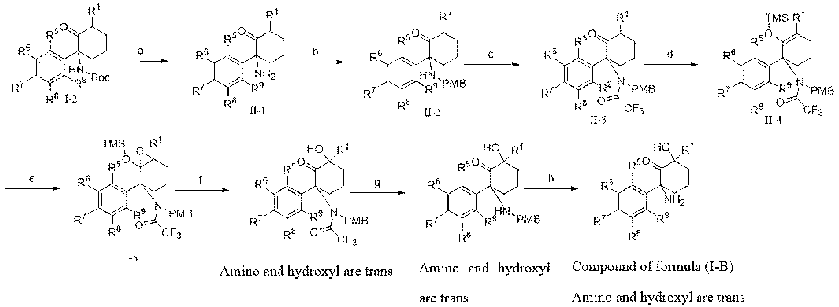 wherein, R¹, R⁵, R⁶, R⁷, R⁸ and R⁹ are defined according to the first aspect of the present invention;
   a. in a polar non-protonic solvent, compound I-2 deprotects the tert-butoxycarbonyl protecting group to form compound II-1;
   b. in a non-protonic solvent, compound II-1 reacts with p-methoxybenzyl chloride to form compound II-2;
   c. in a non-protonic solvent, Compound II-2 reacts with trifluoroacetic anhydride to form Compound II-3;
   d. in a non-protonic solvent, Compound II-3 reacts with trimethylchlorosilane to form Compound II-4;
   e. in a non-protonic solvent, Compound II-4 and an oxidizing agent undergo oxidation to form Compound II-5;
   f. in a non-protonic solvent, compound II-5 deprotects the trimethylsilyl protecting group to form compound II-6;
   g. in proton solvent, compound II-6 removes trifluoroacetyl group to form compound II-7;
   h. in acidic conditions, Compound II-7 removes p-methoxybenzyl to form Compound I-B;
or (3) the method comprising steps of: 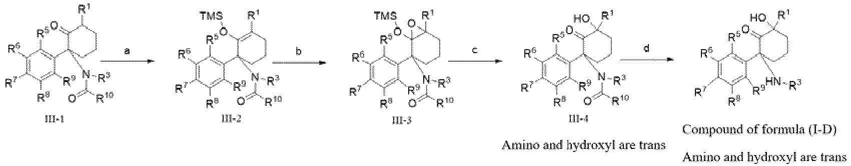 wherein, R¹, R⁵, R⁶, R⁷, R⁸ and R⁹ are defined according to the first aspect of the present invention, R³ is C₁-C₆ alkyl or deuterated C₁-C₆ alkyl, and R¹⁰ is C₁-C₆ alkyl, halogenated C₁-C₆ alkyl, C₁-C₆ alkoxy or benzyloxy;
   a. in a non-protonic solvent, compound III-1 reacts with trimethylchlorosilane to form compound III-2;
   b. in a non-protonic solvent, Compound III-2 and an oxidizing agent undergo oxidation to form Compound III-3;
   c. in a non-protonic solvent, compound III-3 deprotects the trimethylsilyl protecting group to form compound III-4;
   d. in a proton solvent or a non-proton solvent, Compound III-4 deprotects the R¹⁰ carbonyl protecting group to form Compound I-D;
or (4) the method comprises steps of: 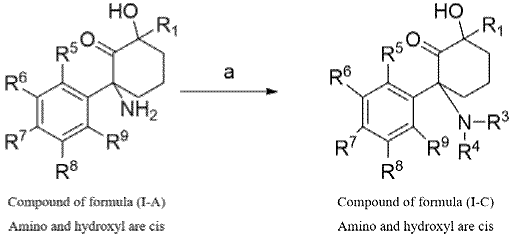 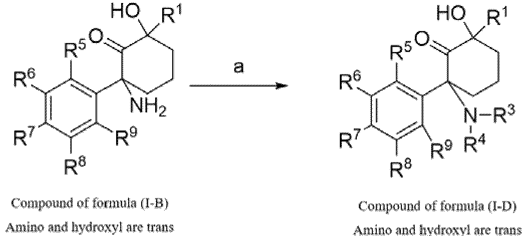 Wherein, R¹, R⁵, R⁶, R⁷, R⁸ and R⁹ are defined according to the first aspect of the present invention, and R³ and R⁴ are independently hydrogen, C₁-C₆ alkyl or deuterated C₁-C₆ alkyl, and R³ and R⁴ are not both hydrogen at the same time;
   a. in a protonated solvent or a non-protonated solvent, compound I-A or I-B react with a corresponding aldehyde or ketone to form the corresponding compound I-C or I-D;
or (5) the method comprises steps of: 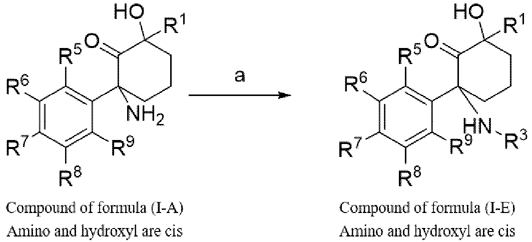 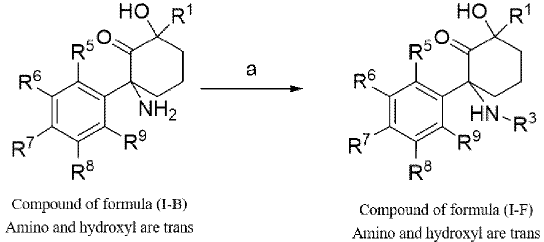 wherein, R¹, R⁵, R⁶, R⁷, R⁸ and R⁹ are defined according to the first aspect of the present invention, and R³ is -(C=O)-C₁-C₆ alkyl;
   a. in a non-protonic solvent, compound I-A or I-B react with a corresponding acyl chloride or anhydride to form the corresponding compound I-E or I-F;
or (6) the method comprises steps of: 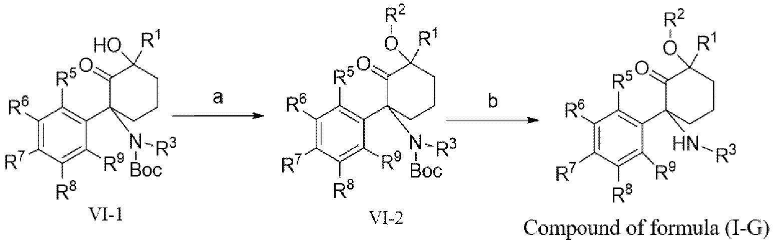 wherein, R¹, R⁵, R⁶, R⁷, R⁸ and R⁹ are defined according to the first aspect of the present invention; R² is C₁-C₆ alkyl, deuterated C₁-C₆ alkyl or -(C=O)-Ci-Ce alkyl; R³ is hydrogen, C₁-C₆ alkyl or deuterated C₁-C₆ alkyl;
   a. in a non-protonic solvent, compound VI-1 reacts with a corresponding alkylating reagent, deuteroalkylating reagent, acyl chloride or anhydride to form the corresponding compound VI-2;
   b. in a polar non-protonic solvent, compound VI-2 deprotects the tert-butoxycarbonyl protecting group to form compound I-G.

In another preferred embodiment,
the method for the preparation of compound III-1 comprises steps of: 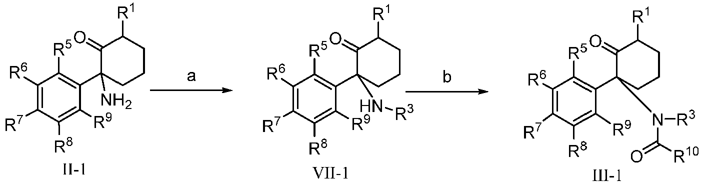
Wherein, R¹, R⁵, R⁶, R⁷, R⁸ and R⁹ are defined according to the first aspect of the present invention, R³ is C₁-C₆ alkyl or deuterated C₁-C₆ alkyl, and R¹⁰ is C₁-C₆ alkyl, halogenated C₁-C₆ alkyl, C₁-C₆ alkoxy and benzyloxy;
   a. in a protonated or non-protonated solvent, compound II-1 reacts with the corresponding aldehyde or ketone to form compound VII-1;
   b. in a non-protonic solvent, compound VII-1 reacts with an anhydride or an acyl chloride to form compound III-1;
or the method for the preparation of compound III-1 comprising steps of: 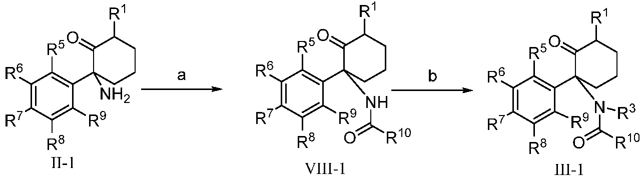
wherein, R¹, R⁵, R⁶, R⁷, R⁸ and R⁹ are defined according to the first aspect of the present invention, R³ is C₁-C₆ alkyl or deuterated C₁-C₆ alkyl, and R¹⁰ is C₁-C₆ alkyl, halogenated C₁-C₆ alkyl, C₁-C₆ alkoxy and benzyloxy;
   a. in a non-protonic solvent, compound II-1 reacts with anhydride or acyl chloride to form compound VIII-1;
   b. in a protonated or non-protonated solvent, compound VIII-1 reacts with the corresponding alkylating reagent or deuterated alkylating reagent to form compound III-1.

The fourth aspect of the present invention provided is a use of the compound of the first aspect of the present invention, or a tautomer, an enantiomer, a diastereoisomer, a racemate, or a mixture thereof, or a pharmaceutically acceptable salt thereof in the preparation of a medication for the treatment of neurological related disorders.

In another preferred embodiment, the neurological related disorder is selected from the group consisting of depression, post-traumatic stress disorder, obsessive-compulsive disorder, anxiety, and pain.

It should be understood that in the present invention, any of the technical features specifically described above and below (such as in the Examples) can be combined with each other, so as to constitute new or preferred technical solutions. Due to space limitations, it will not be repeated here.

### DETAILED DESCRIPTION OF THE INVENTION

After extensive experimental studies on drug design, chemical synthesis and structural testing, the present inventors have synthesized a series of structurally novel compounds as shown in formula (I), and have found for the first time that the compounds represented by the following formula (I) have rapid onset of action in oral administration, potent and long-lasting antidepressant activity , high brain exposure, high brain/plasma ratio, good chemical stability and other excellent drug-forming properties through the most classical antidepressant efficacy experiments -forced swimming experiments on mice, metabolism studies, etc., which are particularly suitable for use as antidepressant drugs for the treatment of depression and neurological related disorders. On this basis, the inventors have completed the present invention.

### TERMS

In the present invention, unless otherwise indicated, the terms used have the ordinary meaning known to those skilled in the art.

In the present invention, the term "halogen" refers to F, Cl, Br or I.

In the present invention, the term "C1-C6 alkyl" or "C₁-C₆ alkyl" refers to a straight or branched alkyl group comprising 1-6 carbon atoms, such as methyl, ethyl, propyl, isopropyl, butyl, isobutyl, tert-butyl, neopentyl, tert-amyl, or similar groups.

In the present invention, the term "C1-C6 alkoxy" or "C₁-C₆ alkoxy" refers to a straight or branched alkoxy group with 1-6 carbon atoms, and non-limitingly includes methoxy, ethoxy, propoxy, isopropoxy, and butoxy, etc. Preferably C1-C4 alkoxy.

In the present invention, the term "halogenated" means substituted with a halogen.

In the present invention, the term "deuterated" means substituted with deuterium.

In the present invention, the term "substituted" refers to one or more hydrogen atoms on a particular group substituted by a particular substituent. The particular substituent is a substituent described above accordingly, or a substituent in the examples. Unless indicated specifically, a substituted group may have a substituent selected from a particular group at any substitutable site of the group, and the substituents can be identical or different at each site. It should be understood by those skilled in the art that the combinations of substituents expected in the present invention are ones that are stable or chemically realizable.

It should be understood that when a certain group is present at a number of different sites in a compound, its definition at each site is independent and may be the same or different. That is, the term "selected from the groups consisting of:" has the same meaning as the term "independently selected from the groups consisting of:".

### Compounds

The present invention provides a compound shown in formula (I), or a tautomer, an enantiomer, a diastereoisomer, a racemate, or a mixture thereof, or a pharmaceutically acceptable salt thereof; 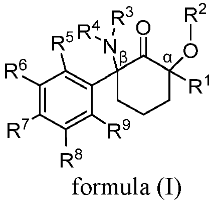 wherein, the groups are as described above.

In another preferred embodiment, any one of R¹, R², R³, R⁴, R⁵, R⁶, R⁷, R⁸ and R⁹ of the compound is independently a corresponding group in the specific compound described herein.

In another preferred embodiment, the compound is preferably a compound prepared in the examples.

As used herein, the term "pharmaceutically acceptable salt" refers to a salt of a compound herein formed with an acid or base suitable for use as a drug. Pharmaceutically acceptable salts include inorganic and organic salts. A preferred class of salts are salts formed by the compounds of the present invention with acids. Acids suitable for forming salts include (but are not limited to): inorganic acids such as hydrochloric, hydrobromic, hydrofluoric, sulfuric, nitric, and phosphoric acids; organic acids such as formic, acetic, trifluoroacetic, propionic, oxalic, malonic, succinic, fumaric, maleic, lactic, malic, tartaric, citric, picric acid, benzoic, methanesulfonic, ethylsulfonic, p-toluenesulfonic, phenylsulfonic, and naphthalenesulfonic acids; and proline, phenylalanine, aspartic acid, glutamic acid and other amino acids.

### Methods of Preparation

Methods of preparing the compounds of the structure of formula (I) of the present invention are described more specifically below, but these specific methods do not constitute any limitation herein. The compounds of the present invention may also be conveniently made by an optional combination of various synthetic methods described in this specification or known in the art, and the combinations can be readily carried out by those skilled in the art.

Typically, the process flow for the preparation of the compounds of the present invention is as follows, wherein the raw materials and reagents used are commercially available if not otherwise specified.

The compounds of the present invention may be produced by the following methods, however the conditions of the methods, such as reactants, solvents, acids, bases, amounts of compounds used, reaction temperatures, reaction times, and the like, are not limited to the following description. The compounds of the present invention may also be conveniently made by an optional combination of various synthetic methods described in this specification or known in the art.

The compounds of the present invention of formula (I) can be prepared according to the preferred methods of reaction formula (1), reaction formula (2), reaction formula (3), reaction formula (4), reaction formula (5), or reaction formula (6). 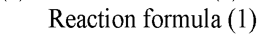 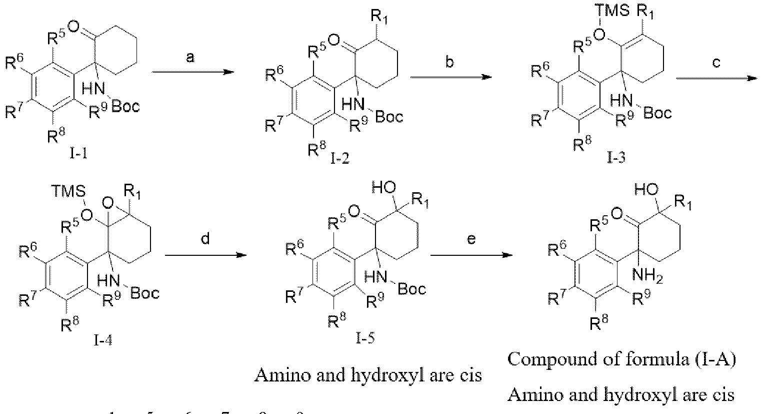 Wherein, R¹, R⁵, R⁶, R⁷, R⁸, R⁹ are defined above;
a. Compound I-1 is reacted with heavy water or an alkylating reagent in the presence of an inorganic base or an organic base, protected by an inert gas, in a non-protonic solvent at -80°C to room temperature to give Compound I-2. The alkylating reagent may be a C₁-C₆ iododoalkane, a C₁-C₆ bromoalkane, or an C₁-C₆ alkyl sulphate; the inorganic or organic base may be sodium hydrogen, lithium diisopropylamino (LDA), lithium hexamethyldisilamido (LHMDS), potassium hexamethyldisilamido (KHMDS), butyllithium; the non-protonic solvent may be toluene, tetrahydrofuran, 2-methyltetrahydrofuran, 1,4-dioxane, N,N-dimethylformamide; the inert gas may be nitrogen or argon.
b. Compound I-2 is reacted with trimethylchlorosilane under the protection of an inert gas in a non-protonic solvent at -80°C to room temperature and in the presence of an organic base to obtain Compound I-3. The non-protonic solvent can be tetrahydrofuran, 2-methyltetrahydrofuran; the organic base can be lithium diisopropylamino, lithium hexamethyldisilamido, potassium hexamethyldisilanylamino, butyllithium; the inert gas may be nitrogen or argon.
c. Compound I-3 is oxidized by an oxidizing agent in a non-protonic solvent at - 40°C to room temperature to obtain compound I-4. The non-protonic solvent may be tetrahydrofuran, 2-methyltetrahydrofuran, dichloromethane, chloroform; the oxidizing agent may be m-chloroperoxybenzoic acid, hydrogen peroxide.
d. Compound I-4 is deprotected by a fluorine-containing reagent to remove the trimethylsilyl protecting group in a non-protonic solvent at 0°C to room temperature to obtain compound I-5. The non-protonic solvent may be tetrahydrofuran, 2-methyltetrahydrofuran, dichloromethane, chloroform; the fluorine-containing reagent may be tetrabutylammonium fluoride.
e. Compound I-5 is deprotected in the presence of an organic or inorganic acid to remove the tert-butoxycarbonyl protecting group in a polar non-protonic solvent at 0° C to room temperature to give compound I-A. The organic or inorganic acid may be hydrochloric acid, sulfuric acid, hydrobromic acid, trifluoroacetic acid; the polar non-protonic solvent may be tetrahydrofuran, dimethyltetrahydrofuran, dichloromethane or chloroform. 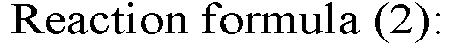 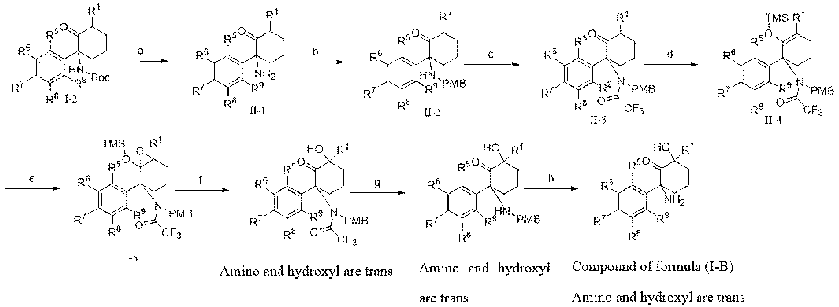 Wherein, R¹, R⁵, R⁶, R⁷, R⁸, R⁹ are defined above;
   a. Compound I-2 is deprotected in the presence of an organic or inorganic acid to remove a tert-butoxycarbonyl protecting group in a polar non-protonic solvent at 0°C to room temperature to obtain compound II-1. The organic or inorganic acid may be hydrochloric acid, sulfuric acid, hydrobromic acid, trifluoroacetic acid; the polar non-protonic solvent may be tetrahydrofuran, dimethyltetrahydrofuran, dichloromethane, or chloroform.
   b. Compound II-1 is reacted with p-methoxybenzaldehyde in a protonated or non-protonated solvent or a mixture thereof in the presence of a reducing agent at 0°C to room temperature to obtain Compound II-2. The protonated or non-protonated solvent may be methanol, ethanol, tetrahydrofuran, ethyl acetate, dichloromethane, 1,4-dioxane; the reducing agent may be sodium cyanoborohydride, sodium borohydride acetate.
   c. Compound II-2 is reacted with trifluoroacetic anhydride in a non-protonic solvent at 0°C to room temperature and in the presence of an organic base to obtain compound II-3. The non-protonic solvent may be tetrahydrofuran, 1,4-dioxane, dichloromethane; the organic base may be triethylamine, diisopropylethylamine, 4-dimethylaminopyridine, imidazole.
   d. Compound II-3 is reacted with trimethylchlorosilane under the protection of an inert gas in a non-protonic solvent at -80°C to room temperature and under the condition of an organic base to obtain Compound II-4. The non-protonic solvent can be tetrahydrofuran, 2-methyltetrahydrofuran; and the organic base can be lithium diisopropylamino, lithium hexamethyldisilanylamino, potassium hexamethyldisilanylamino, and butyllithium; the inert gas may be nitrogen or argon.
   e. Compound II-4 is oxidized by an oxidizing agent in a non-protonic solvent at - 40°C to room temperature to obtain compound II-5. The non-protonic solvent may be tetrahydrofuran, 2-methyltetrahydrofuran, dichloromethane, chloroform; the oxidizing agent may be m-chloroperoxybenzoic acid, hydrogen peroxide.
   f. Compound II-5 is deprotected the trimethylsilyl protecting group by a fluorine-containing reagent in a non-protonic solvent at 0°C to room temperature to obtain compound II-6. The non-protonic solvent may be tetrahydrofuran, 2-methyltetrahydrofuran, dichloromethane, chloroform; the fluorine-containing reagent may be tetrabutylammonium fluoride.
   g. Compound II-6 is deprotected under the action of an inorganic base to remove the trifluoromethanesulfonyl protecting group in a proton solvent at 0°C to room temperature to obtain compound II-7. The inorganic base may be sodium hydroxide, potassium hydroxide, sodium carbonate, potassium carbonate, cesium carbonate; the proton solvent may be methanol, ethanol, water or a mixture thereof.
   h. Compound II-7 is deprotected under the action of organic or inorganic acid to remove p-methoxybenzyl protecting group at room temperature to 100°C to obtain Compound I-B. The organic or inorganic acid can be hydrochloric acid, sulfuric acid, acetic acid, hydrobromic acid, trifluoroacetic acid 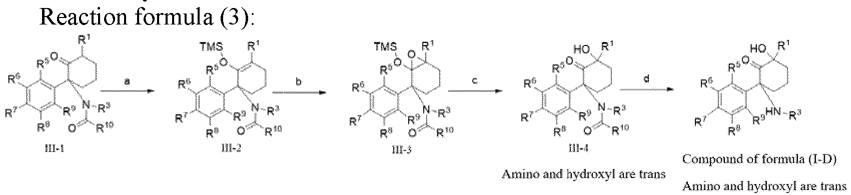
   Wherein, R¹, R⁵, R⁶, R⁷, R⁸, R⁹ are defined above, R³ is C₁-C₆ alkyl or deuterated C₁-C₆ alkyl, and R¹⁰ is C₁-C₆ alkyl, halogenated C₁-C₆ alkyl, C₁-C₆ alkoxy, or benzyloxy;
   a. Compound III-1 is reacted with trimethylchlorosilane under the protection of an inert gas in a non-protonic solvent at -80°C to room temperature and under the organic base condition to obtain Compound III-2. The non-protonic solvent can be tetrahydrofuran, 2-methyltetrahydrofuran; the organic base can be lithium diisopropylamino, lithium hexamethyldisilanylamo, potassium hexamethyldisilanylamino, butyllithium; the inert gas may be nitrogen or argon.
   b. Compound III-2 is oxidized by an oxidizing agent in a non-protonic solvent at -40°C to room temperature to obtain Compound III-3. The non-protonic solvent may be tetrahydrofuran, 2-methyltetrahydrofuran, dichloromethane, chloroform; the oxidizing agent may be m-chloroperoxybenzoic acid, hydrogen peroxide.
   c. Compound III-3 is deprotected by fluorine-containing reagent trimethylsilyl protecting group in a non-protonic solvent at 0°C to room temperature to obtain compound III-4. The non-protonic solvent can be tetrahydrofuran, 2-methyltetrahydrofuran, dichloromethane, chloroform; the fluorine-containing reagent can be tetrabutylammonium fluoride.
   d. Compound III-4 is deprotected under the action of inorganic base, inorganic acid or organic acid to remove the carbonyl protecting group of R¹⁰ in a protonated solvent or a non-protonated solvent at 0°C to room temperature to obtain Compound I-D. The inorganic base can be sodium hydroxide, potassium hydroxide, sodium carbonate, potassium carbonate, cesium carbonate; the inorganic acid or organic acid can be hydrochloric acid, sulfuric acid, acetic acid, hydrobromic acid, trifluoroacetic acid; the protonated solvent can be methanol, ethanol, water or a mixture thereof; the non-protonated solvent can be tetrahydrofuran, 2-methyltetrahydrofuran, dichloromethane, chloroform. 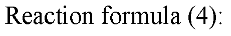 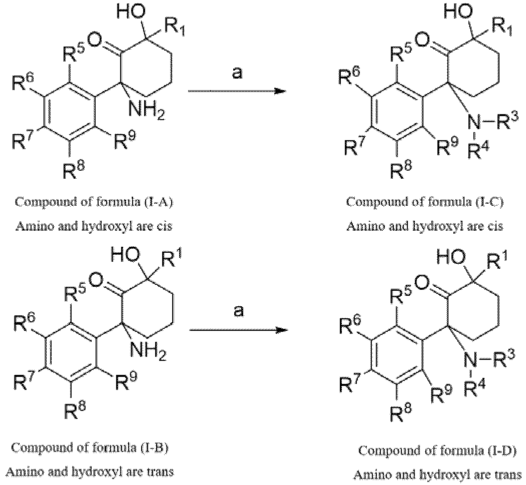
   Wherein, R¹, R⁵, R⁶, R⁷, R⁸, R⁹ are defined above, and R³, R⁴ are independently hydrogen, C₁-C₆ alkyl, deuterated C₁-C₆ alkyl, and R³, R⁴ are not both hydrogen;
   a. Compounds I-A or I-B are reacted with aldehydes or ketones in a proton or non-proton solvent or a mixture thereof in the presence of a hydrogen source with or without a catalyst to obtain compounds I-C or I-D with substituents on the amino groups. The aldehydes or ketones can be deuterated or non-deuterated C₁-C₆ alkyl aldehydes, deuterated or non-deuterated C₁-C₆alkyl ketones; the proton or non-proton solvent may be methanol, ethanol, water, tetrahydrofuran, ethyl acetate, dichloromethane, 1,4-dioxane; the catalyst may be Pd/C, Pd(OH)₂/C; and the hydrogen source may be hydrogen, formic acid, sodium borohydride acetate, sodium cyanoborohydride. 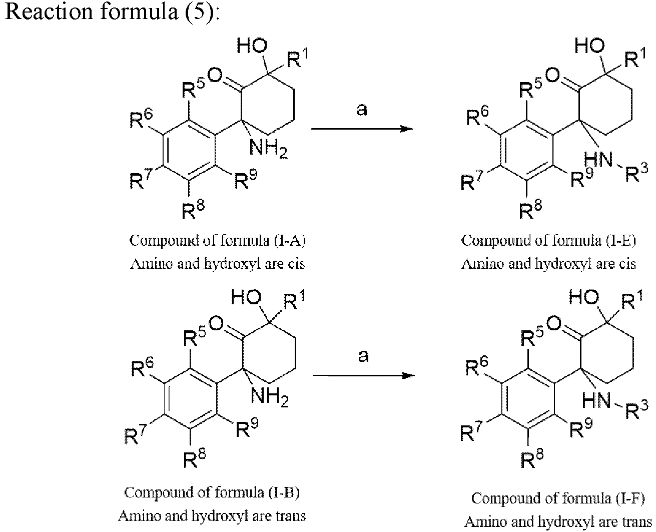
   Wherein, R¹, R⁵, R⁶, R⁷, R⁸, R⁹ are defined above and R³ is -(C=O)-C₁-C₆ alkyl;
   a. Compounds I-A or I-B are reacted with an acyl chloride or anhydride, in a non-protonic solvent, at 0°C to room temperature and with an organic base to give compounds I-E or I-F. The non-protonic solvent may be tetrahydrofuran, 2-methyltetrahydrofuran, dichloromethane, chloroform, ethyl acetate; The organic base may be triethylamine, imidazole, 4-dimethylaminopyridine, 1,8-diazabicyclo[5.4.0]undec-7-ene, pyridine; the acyl chlorides may be C₂-C₆ alkyl acyl chlorides; the anhydrides may be C₄-C₁₂alkyl anhydrides. 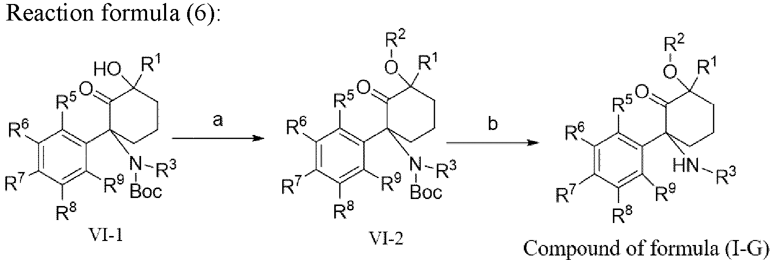
   Wherein, R¹, R⁵, R⁶, R⁷, R⁸, R⁹ are defined above; R² is C₁-C₆ alkyl, deuterated C₁-C₆ alkyl, -(C=O)-C₁-C₆ alkyl; and R³ is hydrogen, C₁-C₆ alkyl, deuterated C₁-C₆ alkyl;
   a. Compound VI-1 is reacted with an alkylating reagent, a deuterated alkylating reagent, an acyl chloride or an anhydride in a non-protonic solvent at 0°C to room temperature and in the presence of an organic base to obtain Compound VI-2. Said non-protonic solvent may be tetrahydrofuran, 2-methyltetrahydrofuran, 1,4-dioxane, methylene chloride; said organic base may be triethylamine, diisopropylethylamine, 4-dimethylaminopyridine, imidazole; said alkylating reagent or deuterated alkylating reagent may be a deuterated or non-deuterated C₁-C₆ iodoalkane, a deuterated or non-deuterated C₁-C₆ bromoalkane, a deuterated or non-deuterated C₁-C₆ alkyl sulfate; said acyl chloride may be a C₂-C₆ alkyl acyl chlorine; and said anhydride may be an C₄-C₁₂ alkyl anhydride.
   b. Compound VI-2 is deprotected in the presence of an organic or inorganic acid to remove the tert-butoxycarbonyl protecting group in a polar non-protonic solvent at 0°C to room temperature to obtain Compound I-G. The organic or inorganic acid may be hydrochloric acid, sulphuric acid, hydrobromic acid, trifluoroacetic acid; and the polar non-protonic solvent may be tetrahydrofuran, dimethyl tetrahydrofuran, dichloromethane or chloroform.

Intermediate III-1 can be prepared according to the method of reaction formula (7) or reaction formula (8). 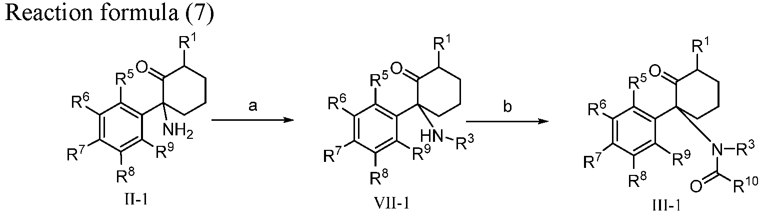 Wherein, R¹, R⁵, R⁶, R⁷, R⁸, R⁹ are defined above, R³ is C₁-C₆ alkyl or deuterated C₁-C₆ alkyl, and R¹⁰ is C₁-C₆ alkyl, halogenated C₁-C₆ alkyl, C₁-C₆ alkoxy or benzyloxy;
a. Compound II-1 is reacted with an aldehyde or ketone in a protonated or non-protonated solvent or a mixture thereof in the presence of a hydrogen source with or without a catalyst to obtain Compound VII-1. The aldehyde or ketone may be a deuterated or non-deuterated C₁-C₆alkyl aldehyde, a deuterated or non-deuterated C₁-C₆alkyl ketone; the protonated or non-protonated solvent may be methanol, ethanol, water, tetrahydrofuran, ethyl acetate, dichloromethane, 1,4-dioxane; said catalyst may be Pd/C, Pd(OH)₂/C; the hydrogen source may be hydrogen, formic acid, sodium borohydride acetate, sodium cyanoborohydride.
b. Compound VII-1 is reacted with an acyl chloride or anhydride, in a non-protonic solvent, at 0°C to room temperature and with an organic base to obtain compound III-1. The non-protonic solvent may be tetrahydrofuran, 2-methyltetrahydrofuran, methylene chloride, chloroform, ethyl acetate; the organic base may be triethylamine, imidazole, 4-dimethylaminopyridine, 1,8-diazabicyclo[5.4.0]undec-7-ene, pyridine; said acyl chloride may be C₂-C₆alkyl acyl chlorides, benzyl chloroformate; the anhydride may be C₄-C₁₂ alkyl anhydride, di-tert-butyl dicarbonate, trifluoroacetic anhydride. 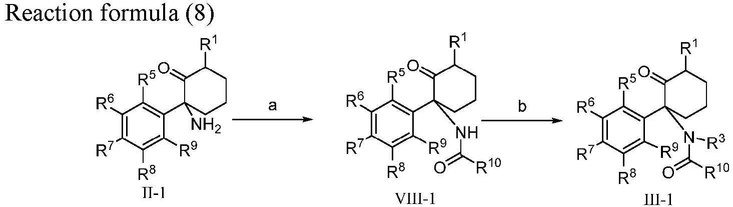 Wherein, R¹, R⁵, R⁶, R⁷, R⁸, R⁹ are defined according to the first aspect of the present invention, R³ is C₁-C₆ alkyl or deuterated C₁-C₆ alkyl, and R¹⁰ is C₁-C₆ alkyl, halogenated C₁-C₆ alkyl, C₁-C₆ alkoxy or benzyloxy;
   a. Compound II-1 is reacted with an acyl chloride or anhydride, in a non-protonic solvent, at 0°C to room temperature and with an organic base to obtain compound VIII-1. The non-protonic solvent may be tetrahydrofuran, 2-methyltetrahydrofuran, dichloromethane, chloroform, ethyl acetate; the organic base may be triethylamine, imidazole, 4-dimethylaminopyridine, 1,8-diazabicyclo[5.4.0]undec-7-ene, pyridine; said acyl chloride may be C₂-C₆alkyl acyl chlorides, benzyl chloroformate; the anhydride may be C₄-C₁₂ alkyl anhydride, di-tert-butyl dicarbonate, trifluoroacetic anhydride.
   b. Compound VIII-1 is reacted with an alkylating reagent or a deuterated alkylating reagent in the presence of an inorganic base or an organic base under the protection of an inert gas in a non- protonic solvent at -80°C to room temperature to obtain Compound III-1. The alkylating reagent or deuterated alkylating reagent may be a deuterated or non-deuterated C₁-C₆ iodoalkane, a deuterated or non-deuterated C₁-C₆ bromoalkanes, deuterated or undeuterated C₁-C₆ alkyl sulfates; the inorganic or organic base may be sodium hydrogen, lithium diisopropylammonium, lithium hexamethyldisilammonium, potassium hexamethyldisilammonium, butyllithium; the non- protonic solvents may be toluene, tetrahydrofuran, 2-methyltetrahydrofuran, 1,4-dioxane, N,N-dimethylformamide; the inert gas may be nitrogen or argon.

The pharmaceutically acceptable salt is one formed by a compound of general formula (I) (e.g. a compound of general formula (I-A), a compound of general formula (I-B), a compound of general formula (I-C), a compound of general formula (I-D), a compound of general formula (I-G)) with an inorganic acid or an organic acid, etc.; preferably the inorganic acid suitable for forming the salt is hydrochloric, hydrobromic, hydrofluoric, sulfuric, trifluoroacetic, nitric, or phosphoric acid; the organic acids suitable for forming the salt are formic, acetic, propionic, oxalic, benzoic, malic, succinic, fumaric, maleic, lactic, malic acid, tartaric acid, citric acid, picric acid, methanesulfonic acid, ethanesulfonic acid, benzenesulfonic acid, p-toluenesulfonic acid, naphthalenesulfonic acid, proline, phenylalanine, glutamic acid, aspartic acid.

The pharmaceutically acceptable salts of Compound I may be prepared by the following method: compounds of Formula I (e.g., Compound I-A, I-B, I-C, I-D, or I-G) or of General Formulas I-5, II-7, or VI-2 are reacted with a variety of organic or inorganic acids in a non-protonic solvent or a protonic solvent, or a mixture of solvents thereof, to obtain the corresponding organic or inorganic acid salts of Compound I.

The non-protonic solvent or protonic solvent may be methanol, ethanol, water, dichloromethane, tetrahydrofuran, ethyl acetate, dioxane; the organic or inorganic acid may be hydrochloric acid, hydrobromic acid, hydrofluoric acid, sulfuric acid, trifluoroacetic acid, nitric acid, or phosphoric acid, formic acid, acetic acid, propionic acid, oxalic acid, benzoic acid, malonate, succinic acid, fumaric acid, maleic acid, lactic acid, malic acid, tartaric acid, citric acid, picric acid, methanesulfonic acid, ethanesulfonic acid, benzenesulfonic acid, p-toluenesulfonic acid, naphthalenesulfonic acid, proline, phenylalanine, aspartic acid, glutamic acid.

### Pharmaceutical compositions and methods of administration

The present invention also provides a pharmaceutical composition, comprising:
1) one or more safe and effective amounts of the compound, or a tautomer, an enantiomer, a diastereomer, a racemate, or a mixture thereof, or a pharmaceutically acceptable salt thereof; and
2) a pharmaceutically acceptable carrier or excipient.

In another preferred embodiment, the pharmaceutical composition is an oral formulation.

The pharmaceutical compositions of the present invention comprise a safe and effective amount of a compound of the present invention or a pharmaceutically acceptable salt thereof and a pharmaceutically acceptable excipient or carrier. The term "safe and effective amount" means that the amount of the compound is sufficient to significantly improve the condition without causing serious side effects. Typically, the pharmaceutical compositions contain 1-2000 mg of the compound of the invention/dose, preferably 10-1000 mg of the compound of the invention/dose. Preferably, the "dose" is a capsule or tablet.

"Pharmaceutically acceptable carrier" means one or more compatible solid or liquid fillers or gel substances which are suitable for human use and which must be of sufficient purity and sufficiently low toxicity. "Compatibility" herein refers to the ability of the components of the composition to be admixed with and between the compounds of the invention without appreciably reducing the efficacy of the compounds. Examples of pharmaceutically acceptable carrier portions are cellulose and derivatives thereof (e.g., sodium carboxymethylcellulose, sodium ethylcellulose, cellulose acetate, etc.), gelatin, talc, solid lubricants (e.g., stearic acid, magnesium stearate), calcium sulfate, vegetable oils (e.g., soybean oil, sesame oil, peanut oil, olive oil, etc.), polyols (e.g., propylene glycol, glycerol, mannitol, sorbitol, etc.), emulsifying agents (e.g., Tween^{®}), Wetting agent (e.g. sodium dodecyl sulfate), coloring agent, flavoring agent, stabilizer, antioxidant, preservative, pyrogen-free water, etc.

The pharmaceutical compositions are in the form of injections, capsules, tablets, pills, dispersions or granules.

There are no particular limitations to the mode of administration of the compounds or pharmaceutical compositions of the present invention, representative modes of administration include (but are not limited to): oral, parenteral (intravenously, intramuscularly, nasal spray, or subcutaneously), and topical administration.

Solid dosage forms for oral administration include capsules, tablets, pills, dispersions and granules. In these solid dosage forms, the active compound is mixed with at least one conventional inert excipient (or carrier) such as sodium citrate or dicalcium phosphate, or mixed with: (a) fillers or bulking agents, such as, for example, starch, lactose, sucrose, dextrose, mannitol, and silicic acid; (b) binders, such as, for example, hydroxymethylcellulose, alginate, gelatin, polyvinylpyrrolidone, sucrose, and gum Arabic; (c) humectants, e.g., glycerin; (d) disintegrants, e.g., agar, calcium carbonate, potato starch or tapioca starch, alginate, certain complex silicates, and sodium carbonate; (e) retardants, e.g., paraffin waxes; (f) absorption accelerators, e.g., quaternary amine compounds; (g) wetting agents, e.g., cetearyl alcohols and glycerol monostearate; (h) adsorbents, e.g., kaolin; and (i) lubricants, e.g., talc, calcium stearate, magnesium stearate, solid polyethylene glycol, sodium dodecyl sulfate, or mixtures thereof. In capsules, tablets and pills, the dosage form may also contain a buffering agent.

Solid dosage forms such as tablets, sugar pills, capsules, pills and granules may be prepared using coatings and shell materials such as enteric coats and other materials well known in the art. They may comprise opacifying agents, and the release of the active compound or compounds in such compositions may be released in a delayed manner in a portion of the digestive tract. Examples of encapsulated components that may be employed are polymeric substances and wax-like substances. If necessary, the active compound may also be formed in the form of a microcapsule with one or more of the excipients above.

Liquid dosage forms for oral administration include pharmaceutically acceptable emulsions, solutions, suspensions, syrups or tinctures. In addition to the active compound, the liquid dosage form may comprise inert diluents routinely employed in the art, such as water or other solvents, solubilizers and emulsifiers, such as ethanol, isopropanol, ethyl carbonate, ethyl acetate, propylene glycol, 1,3-butylene glycol, dimethylformamide, as well as oils, in particular, cottonseed oil, peanut oil, corn embryo oil, olive oil, castor oil, and sesame oil, or mixtures thereof, etc.

In addition to these inert diluents, the compositions may also comprise auxiliaries such as wetting agents, emulsifiers and suspending agents, sweeteners, flavoring agents and fragrances.

In addition to the active compounds, the suspensions may comprise suspending agents, such as ethoxylated isooctadecanol, polyoxyethylene sorbitol and dehydrated sorbitan esters, microcrystalline cellulose, methanolic aluminum and agar, or mixtures thereof, etc.

Compositions for parenteral administration may comprise physiologically acceptable sterile aqueous or non-aqueous solutions, dispersions, suspensions, or emulsions, and sterile powders for re-dissolution into sterile injectable solutions or dispersions. Suitable aqueous and non-aqueous carriers, diluents, solvents or excipients include water, ethanol, polyols and suitable mixtures thereof.

Dosage forms of the compounds of the present invention for topical administration include ointments, dispersions, patches, sprays, and inhalers. The active ingredient is mixed under sterile conditions with a physiologically acceptable carrier and any preservatives, buffers, or, if necessary, propellants that may be required.

The compounds of the present invention may be administered alone or in combination with other pharmaceutically acceptable other drugs (e.g., antidepressants).

The therapeutic methods of the present invention may be administered alone or in combination with other therapeutic means or therapeutic drugs.

The use of the pharmaceutical compositions involves the application of a safe and effective amount of the compounds of the present invention to a mammal (e.g., human) in need of treatment, wherein the dose at the time of administration is the pharmaceutically considered effective dose to be administered, and for a person of 60 kg body weight, the daily dose to be administered is usually from 1 to 2,000 mg, and preferably from 20 to 1,000 mg. Of course, the specific dose should also take into account the route of administration, the health of the patient, and other factors, which are within the skill of a skilled practitioner.

Compared with the prior art, the present invention has the following main advantages:
(1) The present invention provides a class of compounds with novel structure, fast and long-lasting antidepressant activity, and excellent druggability.
(2) The compounds of the present invention have more excellent antidepressant activity and more excellent chemical stability, and are expected to be developed into new antidepressant drugs with fast onset of action and long-lasting efficacy.
(3) Compared with (2R,6R)-HNK, the compounds of the present invention have higher brain tissue exposure and higher brain/plasma ratios, indicating that the compounds of the present invention have a lower risk of peripheral side effects.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the instructions of manufacturer. Unless indicated otherwise, percentage and parts are calculated by weight.

Unless otherwise defined, all professional and scientific terminology used in the text have the same meanings as known to these skilled in the art. In addition, any methods and materials similar or equal with the recorded content can apply to the methods of the invention. The preferred embodiments and the materials described herein are only for demonstration purposes.

In all examples, 1H-NMR was recorded with a Varian Mercury 300 or Varian Mercury 400 NMR instrument, with chemical shifts expressed in δ (ppm); silica gel was used for separation, 200-300 mesh in all cases not specified, and eluent was dispensed in the volume ratio.

### Preparation Examples

### Example 1: Preparation of 2-amino-2-(2-fluoro-3-(trifluoromethoxy)phenyl)-6-hydroxy-6-methylcyclohexan-1-one (Compound 1)

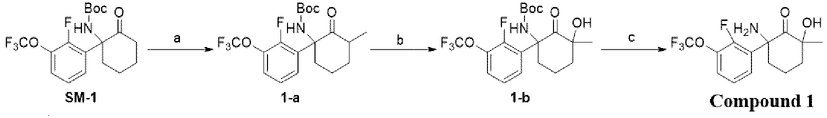

### Step a: Preparation of 1-a

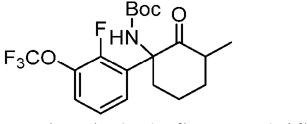

Tert-butyl (1-(2-fluoro-3-(trmuoromethoxy)phenyl)-2-oxocyclohexyl)carbamate (5 g, 12.78 mmol) was dissolved in anhydrous THF (70 mL), cooled to -78 °C, protected by Ar gas, dropwise addition of LDA (9.6 mL, 19.2 mmol), and stirred at -78 °C for 1 h, then MeI (2.36 g, 16.63 mmol) was added, the temperature was slowly raised to 0 °C, and stirred at 0 °C for 3 h. TLC (PE/EA=10/1) detected the raw material was reacted completely, the reaction was quenched with saturated NH4Cl aqueous solution (10 mL), diluted with water (150 mL), and extracted with ethyl acetate (60 mL × 3). The combined organic phases were washed with saturated aqueous NaCl, dried over anhydrous sodium sulfate, and filtered. The filtrate was concentrated to dry, subjected to a column chromatography (PE/EA=20/1-10/1), and then rotatory evaporated to remove the low-boiling solvent to give a colorless oil 2.57 g, yield: 49.6%. ¹H NMR (400 MHz, CDCl₃) δ 7.71 (s, 1H), 7.25 - 7.18 (m, 2H), 6.62 (s, 1H), 3.79 (m, 1H), 2.46 (m, 1H), 2.01 (m, 1H), 1.85 - 1.63 (m, 3H), 1.43 (m, 1H), 1.31 (s, 9H), 1.06 (d, *J =* 6.2 Hz, 3H). MS[M+H]⁺: 406.

### Step b: Preparation of 1-b

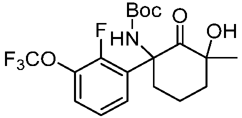

Step 1: Compound 1-a (3.54 g, 8.73 mmol) was dissolved in dry THF (60 mL), cooled to -78°C, protected by Ar gas, LDA (17.5 mL, 35.0 mmol) was added dropwise, and stirred at -78°C for 1 h after dropwise addition, then TMSCl (3.79 g, 34.88 mmol) was added, the reaction was naturally raised to room temperature for 1 h after continued stirring for 10 min. TLC (PE/EA=10/1) detected the raw material was reacted completely, the reaction was quenched with saturated NH4Cl aqueous solution (10 mL), diluted with water (100 mL), and extracted with ethyl acetate (50 mL×3), the organic phases were combined, washed with saturated aqueous NaCl, dried over anhydrous sodium sulfate, filtered, and the filtrate was rotatory evaporated to remove the low-boiling solvent to obtain the crude product.

Step 2: The obtained crude product was dissolved in DCM (50 mL), cooled to - 20°C, protected by Ar, m-CPBA (3.54 g, 17.44 mmol) was added, and the reaction was stirred at -20°C for 10 min and then raised to room temperature and stirred for 1 h. TLC (PE/EA=10/1) detected the raw material was reacted completely, and the reaction was quenched with saturated aqueous Na2SO3 solution (50 mL) and saturated NaHCO3 aqueous solution (50 mL), and extracted with DCM (60 mL×3), the organic phases were combined, and spun-dried to remove the solvent to give a crude product.

Step 3: The obtained crude product was dissolved in THF (50 mL), cooled in an ice bath, and a solution of TBAF·3H2O (4.13 g, 13.09 mmol) in THF (10 mL) was added dropwise, and after stirring for 30 min, TLC (PE/EA=10/1) detected the raw material was reacted completely, then the reaction was quenched with saturated aqueous NaHCO₃ solution (100 mL), extracted with ethyl acetate (60 mL×3), the combined organic phases was washed with saturated NaCl aqueous solution, dried over anhydrous sodium sulfate, filtered, the filtrate was concentrated to dryness, and subjected to a column chromatography (PE/EA=10/1-5/1) to obtain 2.58 g of light yellow solid, yield: 70.1%. ¹H NMR (400 MHz, CDCl₃) δ 7.65 (s, 1H), 7.33 - 7.28 (m, 1H), 7.23 (m, 1H), 6.71 (s, 1H), 3.82 (s, 1H), 2.13 (d, *J =* 9.2 Hz, 1H), 1.90 - 1.68 (m, 4H), 1.29 (s, 9H), 0.88 (s, 3H). MS [M+H]⁺: 422.

### Step c: Preparation of compound 1

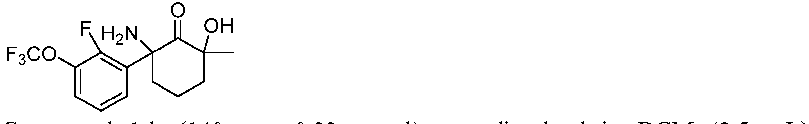

Compound 1-b (140 mg, 0.33 mmol) was dissolved in DCM (2.5 mL), HCl/dioxane solution (0.7 mL, 4 M) was added under an ice bath and stirred at room temperature for 1 h. TLC (PE/EA=10/1) detected the raw material was reacted completely, then the reaction was added saturated NaHCO₃ aqueous solution to adjust pH about 10, extracted with ethyl acetate (10 mL×3), the organic phases were combined, washed with saturated NaCl aqueous solution, dried over anhydrous sodium sulfate, filtered, the filtrate was concentrated to dryness, and then subjected to a column chromatography (DCM/MeOH=100/1-50/1) to obtain 95 mg of white solid, yield: 88.8%. ¹H NMR (400 MHz, CDCl₃) δ 7.41 (t, *J* = 6.9 Hz, 1H), 7.31 (t, *J* = 7.5 Hz, 1H), 7.23 (t, *J* = 8.1 Hz, 1H), 2.72 - 2.61 (m, 1H), 2.06 - 1.95 (m, 2H), 1.94 - 1.81 (m, 2H), 1.80 - 1.70 (m, 1H), 1.08 (s, 3H). MS [M+H]⁺:322.

### Example 2: Preparation of 2-amino-6-hydroxy-6-methyl-2-(2,4,6-trifluorotolyl)cyclohexan-1-one (Compound 2)

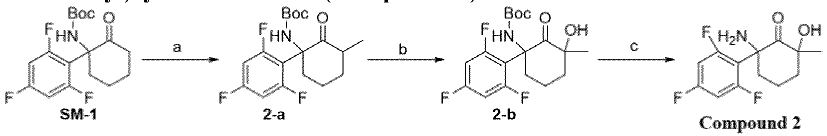

### Step a: Preparation of 2-a

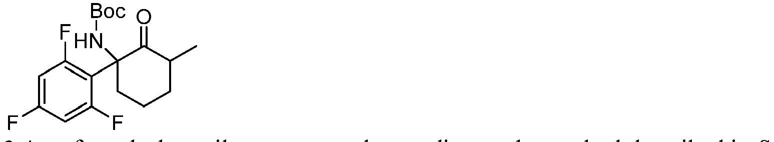

2.4 g of a colorless oil was prepared according to the method described in Step a of Example 1 with Tert-butyl (2-oxo-1-(2,4,6-trifluorophenyl)cyclohexyl)carbamate (2.9 g, 8.45 mmol) as a raw material, yield: 79.5%. ¹H NMR (400 MHz, CDCl₃) δ 6.67 (t, *J* = 9.1 Hz, 2H), 6.55 (s, 1H), 3.82 (d, *J* = 7.8 Hz, 1H), 2.57 (m, 1H), 2.04 (m, 1H), 1.89 - 1.64 (m, 3H), 1.49 - 1.41 (m, 1H), 1.34 (s, 9H), 1.06 (d, *J* = 6.1 Hz, 3H). MS [M+H]⁺:358.

### Step b: Preparation of 2-b

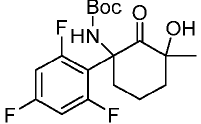

1.71 g of yellow oil was prepared according to the method described in step b in Example 1 with compound 2-a (2.4 g, 6.72 mmol) as a raw material, yield: 68.2%. MS(M+Na)⁺: 396.

### Step c: Preparation of compound 2

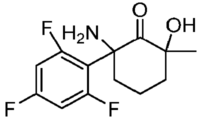

0.93 g of light yellow oil was prepared according to the method described in step c of Example 1 with compound 2-b (1.5 g, 4.02 mmol) as a raw material, yield: 84.7%. ¹H NMR (400 MHz, CDCl₃) δ 6.67 (t, *J* = 9.5 Hz, 2H), 2.83 - 2.72 (m, 1H), 2.12 (m, 1H), 1.92 (m, 3H), 1.71 (m, 1H), 1.19 (s, 3H). MS(M+H)⁺: 274.

### Example 3: Preparation of 2-amino-2-(4-chloro-3-(trifluoromethoxy)phenyl)-6-hydroxy-6-methylcyclohexan-1-one (Compound 3)

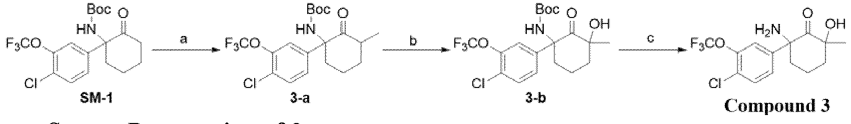

### Step a: Preparation of 3-a

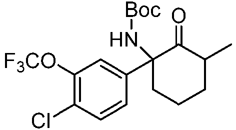

0.9 g of light yellow solid was prepared according to the method described in Step a of Example 1 with Tert-butyl (1-(4-chloro-3-(trifluoromethoxy)phenyl)-2-oxocyclohexyl)carbamate (1.5 g, 3.68 mmol) as a raw material, yield: 58.0%. ¹H NMR (400 MHz, CDCl₃) δ 7.82 (d, *J =* 7.4 Hz, 1H), 7.40 (t, *J* = 8.2 Hz, 1H), 7.32 (d, *J =* 8.3 Hz, 1H), 6.67 (s, 1H), 3.82 (d, *J =* 11.9 Hz, 1H), 2.47 - 2.35 (m, 1H), 2.02 (m, 1H), 1.82 - 1.59 (m, 3H), 1.45 (dt, *J =* 15.7, 11.5 Hz, 1H), 1.31 (s, 9H), 1.06 (d, *J* = 6.4 Hz, 3H). MS (M+H)⁺: 422.

### Step b: Preparation of 3-b

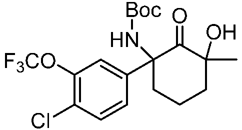

297 mg of yellow oil was prepared according to the method described in step b in Example 1 with compound 3-a (888 mg, 2.10 mmol) as raw material, yield: 32.2%. ¹H NMR (400 MHz, CDCl₃) δ 7.77 (d, *J* = 7.1 Hz, 1H), 7.41 (t, *J* = 8.1 Hz, 1H), 7.34 (d, *J* = 9.1 Hz, 1H), 6.85 (s, 1H), 3.87 (d, *J =* 11.6 Hz, 1H), 2.14 (d, *J =* 12.9 Hz, 1H), 1.80 (m, 2H), 1.71 (m, 2H), 1.29 (s, 9H), 0.87 (s, 3H). MS (M+H)⁺: 438.

### Step c: Preparation of compound 3

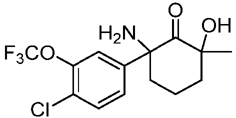

170 mg of white solid was prepared according to the method described in step c of Example 1 with Compound 3-b (290 mg, 0.66 mmol) as raw material, yield: 75.9%. ¹H NMR (400 MHz, CDCl₃) δ 7.55 (d, *J =* 7.6 Hz, 1H), 7.39 (t, *J =* 8.0 Hz, 1H), 7.34 (d, *J* = 8.3 Hz, 1H), 2.68 (dd, *J* = 11.7, 7.0 Hz, 1H), 2.02 (m, 2H), 1.92 - 1.73 (m, 3H), 1.14 (s, 3H). MS (M+H)⁺: 338.

### Example 4: Preparation of 2-amino-2-(3-fluoro-2-(trifluoromethyl)phenyl)-6-hydroxy-6-methylcyclohexan-1-one (Compound 4)

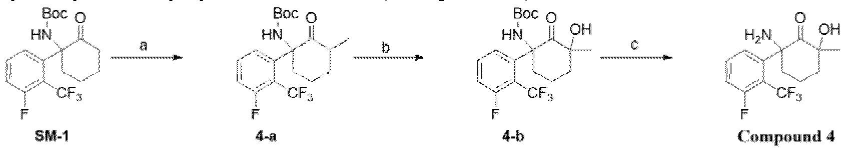

### Step a: Preparation of 4-a

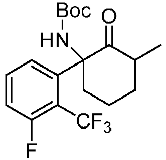

2.0 g of white solid was prepared according to the method described in Step a of Example 1 with Tert-butyl (1-(1-(3-fluoro-2-(trifluoromethyl)phenyl)-2-oxocyclohexyl) carbamate (4.0 g, 10.66 mmol) as a raw material, yield: 48.2%. ¹H NMR (400 MHz, CDCl₃) δ 7.88 (d, *J* = 7.1 Hz, 1H), 7.62 (q, *J* = 7.7 Hz, 1H), 7.24 - 7.17 (m, 1H), 6.48 (s, 1H), 3.89 (d, *J* = 12.7 Hz, 1H), 2.42 (m, 1H), 2.03 (m, 1H), 1.73 (m, 3H), 1.48 (m, 1H), 1.32 (s, 9H), 1.02 (d, *J* = 6.3 Hz, 3H). MS (M+Na)⁺: 412.

### Step b: Preparation of 4-b

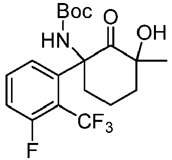

1.0 g of white solid was prepared according to the method described in step b of Example 1 with compound 4-a (2.0 g, 5.14 mmol) as a raw material, yield: 48.1%. ¹H NMR (400 MHz, CDCl₃) δ 7.56 (m, 2H), 7.19 (t, *J =* 7.5 Hz, 1H), 3.13 (m, 1H), 2.37 (m, 1H), 2.04 (m, 1H), 1.95 (m, 2H), 1.85 (m, 1H), 1.29 (s, 9H), 1.13 (s, 3H). MS (M+H)⁺: 406.

### Step c: Preparation of compound 4

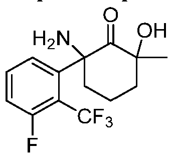

100 mg of white solid was prepared according to the method described in step c of Example 1 with compound 4-b (200 mg, 0.49 mmol) as a raw material, yield: 66.2%. ¹H NMR (400 MHz, CDCl₃) δ 7.58 - 7.49 (m, 2H), 7.18 (t, *J =* 9.2 Hz, 1H), 2.38 - 2.17 (m, 2H), 2.09 (dd, *J =* 22.9, 13.5 Hz, 2H), 1.85 (t, *J =* 14.1 Hz, 1H), 1.74 (d, *J =* 13.4 Hz, 1H), 1.36 (s, 3H). MS (M+H)⁺: 306.

### Example 5: Preparation of 3-(1-amino-3-hydroxy-3-methyl-2-oxocyclohexyl)benzonitrile (Compound 5)

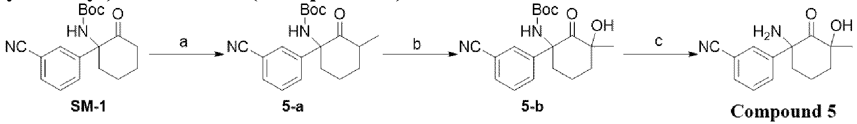

### Step a: Preparation of 5-a

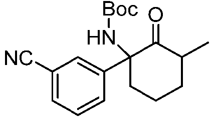

1.2 g of white solid was prepared according to the method described in step a of Example 1 with Tert-butyl (1-(3-cyanophenyl)-2-oxocyclohexyl)carbamate (3.6 g, 11.45 mmol) as a raw material, yield: 31.9%. ¹H NMR (400 MHz, CDCl₃) δ 7.61 (m, 3H), 7.47 (t, *J =* 7.5 Hz, 1H), 6.59 (s, 1H), 3.74 (m, 1H), 2.30 (m, 1H), 2.03 (d, *J =* 12.4 Hz, 1H), 1.86 (m, 3H), 1.51 - 1.41 (m, 1H), 1.30 (s, 9H), 1.02 (d, *J* = 6.1 Hz, 3H). MS [M+H]⁺: 329.

### Step b: Preparation of 5-b

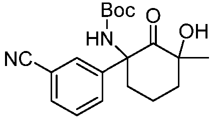

208 mg of white solid was prepared according to the method described in step b of Example 1 with compound 5-a (1.0 g, 3.04 mmol) as a raw material, yield: 19.8%. ¹H NMR (400 MHz, CDCl₃) δ 7.68 (m, 1H), 7.60 (m, 2H), 7.49 (t, *J* = 7.7 Hz, 1H), 6.47 (s, 1H), 3.66 (m, 1H), 2.11 (d, *J* = 8.9 Hz, 1H), 1.99 (m, 2H), 1.83 (q, *J* = 13.9 Hz, 2H), 1.30 (s, 9H), 0.73 (s, 3H). MS [M+H]⁺: 345.

### Step c: Preparation of compound 5

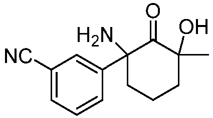

200 mg of colorless oil was prepared according to the method described in step c of Example 1 with compound 5-b (300 mg, 0.87 mmol) as a raw material, yield: 93.9%. ¹H NMR (400 MHz, CDCb) δ 7.63 (m, 2H), 7.51 (d, *J* = 3.5 Hz, 2H), 2.80 (d, *J =* 14.0 Hz, 1H), 2.06 (d, *J =* 11.3 Hz, 1H), 1.98 (d, *J =* 14.3 Hz, 1H), 1.88 (t, *J =* 13.3 Hz, 2H), 1.75 (dd, *J* = 24.6, 12.0 Hz, 1H), 0.85 (s, 3H). MS [M+H]⁺: 245.

### Example 6: Preparation of 2-amino-2-(2,3-difluorophenyl)-6-hydroxy-6-methylcyclohexan-1-one (Compound 6)

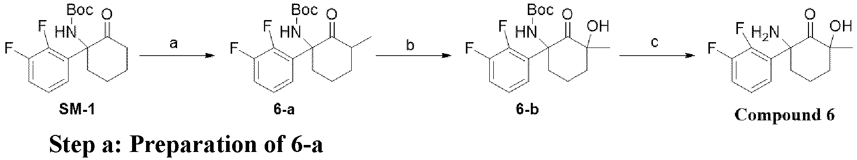

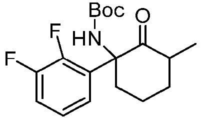

2.98 g of white solid was prepared according to the method described in step a of Example 1 with Tert-butyl (1-(2,3-difluorophenyl)-2-oxocyclohexyl)carbamate (4.2 g, 12.91 mmol) as a raw material, yield: 68.0 %. ¹H NMR (400 MHz, CDCl₃) δ 7.50 (s, 1H), 7.14 (m, 2H), 6.61 (s, 1H), 3.81 (d, *J =* 12.3 Hz, 1H), 2.49 (m, 1H), 2.02 (m, 1H), 1.80 - 1.72 (m, 2H), 1.65 (m, 1H), 1.49 - 1.38 (m, 1H), 1.32 (s, 9H), 1.06 (d, *J =* 6.3 Hz, 3H). MS[M+H]⁺: 340.

### Step b: Preparation of 6-b

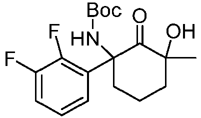

1.8 g of white solid was prepared according to the method described in step b of Example 1 with compound 6-a (2.91 g, 8.57 mmol) as a raw material, yield: 59.1%. ¹H NMR (400 MHz, CDCl₃) δ 7.42 (s, 1H), 7.15 (t, *J* = 6.1 Hz, 2H), 6.69 (s, 1H), 3.83 (s, 1H), 2.13 (d, *J* = 11.8 Hz, 1H), 1.86 - 1.67 (m, 4H), 1.31 (s, 9H), 0.91 (s, 3H). MS[M+H]⁺: 356.

### Step c: Preparation of compound 6

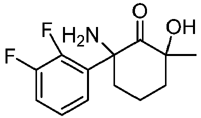

405 mg of white solid in according to the method described in step c of Example 1 with compound 6-b (600 mg, 1.69 mmol) as a raw material, yield: 94.0%. ¹H NMR (400 MHz, CDCl₃) δ 7.22 - 7.12 (m, 3H), 2.72 - 2.61 (m, 1H), 2.00 (m, 2H), 1.94 - 1.73 (m, 3H), 1.10 (s, 3H). MS[M+H]⁺: 256.

### Example 7: Preparation of 2-amino-6-hydroxy-6-methyl-2-(3-(trifluoromethoxy)phenyl)cyclohexan-1-one (Compound 7)

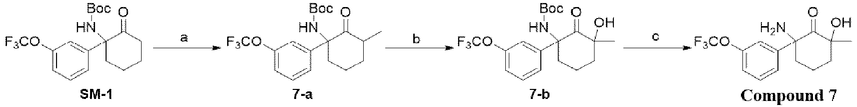

### Step a: Preparation of 7-a

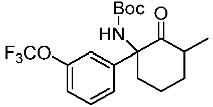

0.79 g of colorless oil was prepared according to the method described in step a of Example 1 with Tert-butyl (2-oxo-1-(3-(trifluoromethoxy)phenyl)cyclohexyl) carbamate (2 g, 5.36 mmol) as a raw material, yield: 38.1 %. ¹H NMR (400 MHz, CDCl₃) δ 7.39 (t, *J* = 8.1 Hz, 1H), 7.29 (m, 1H), 7.14 (m, 2H), 6.56 (s, 1H), 3.71 (s, 1H), 2.34 (s, 1H), 2.04 - 1.98 (m, 1H), 1.88 (m, 3H), 1.48 - 1.41 (m, 1H), 1.30 (s, 9H), 1.01 (d, *J =* 6.4 Hz, 3H). MS [M+H]⁺: 388.

### Step b: Preparation of 7-b

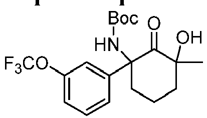

145 mg of white solid was prepared according to the method described in step b of Example 1 with compound 7-a (790 mg, 2.04 mmol) as a material, yield: 17.6%. ¹H NMR (400 MHz, CDCl₃) δ 7.46 - 7.33 (m, 2H), 7.19 (m, 2H), 6.35 (s, 1H), 3.57 (s, 1H), 2.09 (m, 2H), 1.86 (m, 3H), 1.31 (s, 9H), 0.74 (s, 3H). MS [M+Na]⁺: 426.

### Step c: Preparation of compound 7

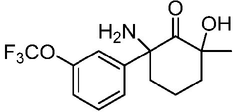

50 mg of white solid was prepared according to the method described in step c of Example 1 with compound 7-b (140 mg, 0.35 mmol) as a raw material, yield: 47.6%. ¹H NMR (400 MHz, CDCl₃) δ 7.42 (t, *J* = 8.0 Hz, 1H), 7.23 (d, *J* = 7.8 Hz, 1H), 7.18 (d, *J =* 8.2 Hz, 1H), 7.14 (s, 1H), 2.83 (d, *J =* 12.2 Hz, 1H), 2.07 (m, 1H), 1.94 - 1.74 (m, 4H), 0.82 (s, 3H). MS [M+H]⁺: 304.

### Example 8: Preparation of 2-amino-6-hydroxy-6-methyl-2-(4-(trifluoromethyl)phenyl)cyclohexan-1-one (Compound 8)

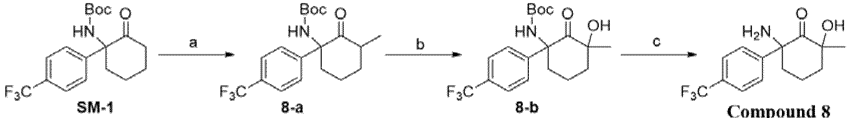

### Step a: Preparation of 8-a

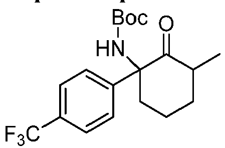

488 mg of colorless oil was obtained according to the method described in step a of Example 1 with Tert-butyl (2-oxo-1-(4-(trifluoromethyl)phenyl)cyclohexyl) carbamate (1.0 g, 2.80 mmol) as a raw material, yield: 46.9 %. ¹H NMR (400 MHz, CDCl₃) δ 7.62 (d, *J* = 8.2 Hz, 2H), 7.43 (d, *J* = 8.2 Hz, 2H), 6.60 (s, 1H), 3.77 (m, 1H), 2.32 (m, 1H), 2.01 (m, 1H), 1.86 (m, 3H), 1.46 (m, 1H), 1.30 (s, 9H), 1.01 (d, *J =* 6.3 Hz, 3H). MS (M+Na)⁺: 394.

### Step b: Preparation of 8-b

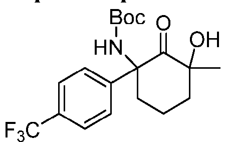

282 mg of white solid was prepared according to the method described in step b of Example 1 with compound 8-a (488 mg, 1.31 mmol) as a raw material, yield: 55.4 %. ¹H NMR (400 MHz, CDCl₃) δ 7.63 (d, *J* = 8.0 Hz, 2H), 7.50 (d, *J* = 8.0 Hz, 2H), 6.42 (s, 1H), 3.67 (m, 1H), 2.16 - 1.96 (m, 2H), 1.88 (m, 3H), 1.30 (s, 9H), 0.72 (s, 3H). MS (M+H)⁺: 388.

### Step c: Preparation of compound 8

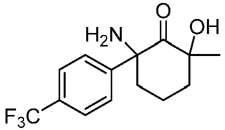

58 mg of white solid was prepared according to the method described in step c of Example 1 with compound 8-b (100 mg, 0.26 mmol) as a raw material, yield: 78.4%. ¹H NMR (400 MHz, CDCl₃) δ 7.65 (d, *J =* 8.0 Hz, 2H), 7.42 (d, *J =* 8.0 Hz, 2H), 2.87 (m, 1H), 2.11 - 2.03 (m, 1H), 1.97 - 1.75 (m, 4H), 0.83 (s, 3H). MS (M+H)⁺: 288.

### Example 9: Preparation of (2R, 6R)-2-amino-6-hydroxy-6-deutero-2-(4-(trifluoromethyl)phenyl)cyclohexan-1-one (Compound 9)

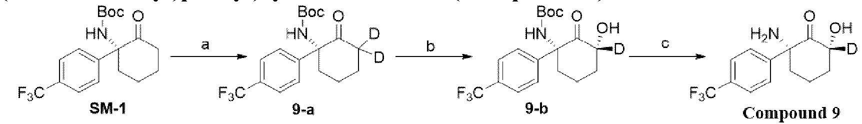

### Step a: Preparation of 9-a

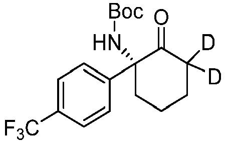

Tert-butyl (R)-(2-oxo-1-(4-(trifluoromethyl)phenyl)cyclohexyl)carbamate (0.6 g, 1.68 mmol) was dissolved in anhydrous THF (6 mL), NaOD (1.8 mL) and D2O (1.2 mL) were added, and the reaction was carried out under microwave conditions at 120 °C for 2 h. TLC (DCM/MeOH=15/1) detected the raw material was reacted completely,, the solvent was concentrated to dryness, and subjected to a column chromatography (PE/EA=5/1-1/1) to obtain 0.56 g of colorless oil in 92.9% yield. ¹H NMR (400 MHz, CDCl₃) δ 7.62 (d, *J =* 8.0 Hz, 2H), 7.47 (d, *J =* 8.0 Hz, 2H), 6.41 (s, 1H), 3.65 (d, *J =* 11.1 Hz, 1H), 2.01 (d, *J =* 10.8 Hz, 1H), 1.96 - 1.71 (m, 4H), 1.32 (s, 9H). MS [M+H]⁺: 360.

### Step b: Preparation of 9-b

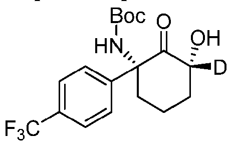

130 mg of white solid was prepared according to the method described in step b of Example 1 with compound 9-a (300 mg, 0.83 mmol) as a raw material, yield: 41.7%. MS [M+Na]⁺: 397.

### Step c: Preparation of compound 9

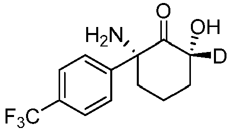

70 mg of white solid was prepared according to the method described in step c of Example 1 with compound 9-b (130 mg, 0.35 mmol) as a raw material, yield: 73.7%. ¹H NMR (400 MHz, CDCl₃) δ 7.66 (d, *J =* 8.0 Hz, 2H), 7.36 (d, *J =* 8.0 Hz, 2H), 2.90 (d, *J =* 13.9 Hz, 1H), 2.37 (d, *J =* 12.0 Hz, 1H), 1.79 (m, 2H), 1.71 - 1.61 (m, 1H), 1.62 - 1.50 (m, 1H). MS [M+Na]⁺: 297.

### Example 10: Preparation of (2R, 6R)-2-amino-6-hydroxy-6-methyl-2-(4-(trifluoromethyl)phenyl)cyclohexan-1-one (Compound 10)

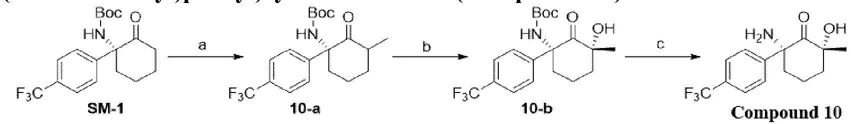

### Step a: Preparation of 10-a

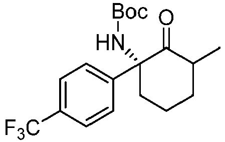

1.05 g of colorless oil was prepared according to the method described in Step a of Example 1 with Tert-butyl (R)-(2-oxo-1-(4-(trifluoromethyl)phenyl)cyclohexyl) carbamate (2.0 g, 5.60 mmol) as a raw material, yield: 50.5 %. ¹H NMR (400 MHz, CDCl₃) δ 7.62 (d, *J* = 8.3 Hz, 2H), 7.43 (d, *J* = 8.3 Hz, 2H), 6.60 (s, 1H), 3.77 (m, 1H), 2.32 (m, 1H), 2.01 (m, 1H), 1.86 (m, 3H), 1.45 (m, 1H), 1.30 (s, 9H), 1.01 (d, *J* = 6.4 Hz, 3H). MS (M+Na)⁺: 394.

### Step b: Preparation of 10-b

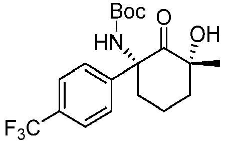

310 mg of white solid was prepared according to the method described in step b in Example 1 with compound 10-a (500 mg, 1.35 mmol) as raw material, yield: 59.4 %. ¹H NMR (400 MHz, CDCl₃) δ 7.63 (d, *J =* 7.9 Hz, 2H), 7.50 (d, *J =* 7.9 Hz, 2H), 6.42 (s, 1H), 3.67 (m, 1H), 2.16 - 1.97 (m, 2H), 1.88 (m, 3H), 1.30 (s, 9H), 0.72 (s, 3H). MS (M+Na)⁺: 410.

### Step c: Preparation of compound 10

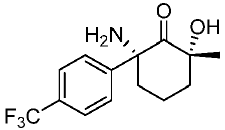

62 mg of white solid was prepared according to the method described in step c of Example 1 with compound 10-b (100 mg, 0.26 mmol) as raw material, yield: 83.8%. ¹H NMR (400 MHz, CDCl₃) δ 7.65 (d, *J =* 8.0 Hz, 2H), 7.42 (d, *J =* 8.0 Hz, 2H), 2.87 (d, *J =* 13.7 Hz, 1H), 2.11 - 2.02 (m, 1H), 1.97 - 1.74 (m, 4H), 0.83 (s, 3H). MS (M+H)⁺: 288.

### Example 11: Preparation of (2S, 6S)-2-amino-6-hydroxy-6-methyl-2-(4-(trifluoromethyl)phenyl)cyclohexan-1-one (Compound 11)

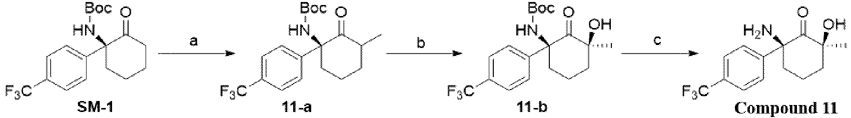

### Step a: Preparation of 11-a

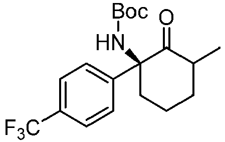

1.64 g of a colorless oil was obtained according to the method described in Step a of Example 1 with Tert-butyl (*S*)-(2-oxo-1-(4-(trifluoromethyl)phenyl)cyclohexyl) carbamate (3.0 g, 8.39 mmol) as a raw material, yield: 52.6 %. ¹H NMR (400 MHz, CDCl₃) δ 7.62 (d, *J* = 8.2 Hz, 2H), 7.43 (d, *J* = 8.1 Hz, 2H), 6.61 (s, 1H), 3.77 (m, 1H), 2.32 (s, 1H), 2.01 (d, *J =* 14.3 Hz, 1H), 1.94 - 1.79 (m, 3H), 1.47 (dd, *J =* 14.7, 10.1 Hz, 1H), 1.30 (s, 9H), 1.01 (d, *J =* 6.3 Hz, 3H). MS (M+Na)⁺ :394.

### Step b: Preparation of 11-b

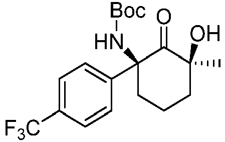

690 mg of white solid was prepared according to the method described in step b of Example 1 with compound 11-a (1.0 g, 2.69 mmol) as a raw material, yield: 66.1 %. ¹H NMR (400 MHz, CDCl₃) δ 7.63 (d, *J =* 8.2 Hz, 2H), 7.50 (d, *J =* 8.2 Hz, 2H), 6.42 (s, 1H), 3.68 (d, *J =* 14.6 Hz, 1H), 2.17 - 2.01 (m, 2H), 1.95 - 1.75 (m, 3H), 1.29 (s, 9H), 0.72 (s, 3H). MS (M+Na)+:410.

### Step c: Preparation of compound 11

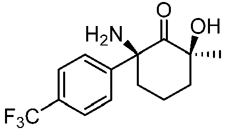

202 mg of white solid was prepared according to the method described in step c of Example 1 with compound 11-b (300 mg, 0.77 mmol) as a raw material, yield: 91.0 %. ¹H NMR (400 MHz, CDCl₃) δ 7.65 (d, *J =* 8.0 Hz, 2H), 7.42 (d, *J =* 8.0 Hz, 2H), 2.87 (d, *J =* 13.6 Hz, 1H), 2.11 - 2.03 (m, 1H), 1.97 - 1.73 (m, 4H), 0.83 (s, 3H). MS (M+H)⁺: 288.

### Example 12: Preparation of (2R, 6S)-2-amino-6-hydroxy-6-methyl-2-(4-(trifluoromethyl)phenyl)cyclohexan-1-one (Compound 12)

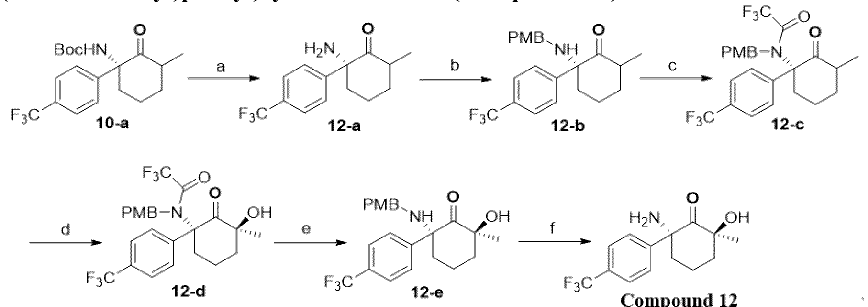

### Step a: Preparation of 12-a

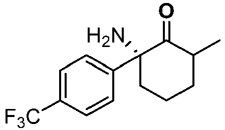

Tert-butyl ((1R)-3-methyl-2-oxo-1-(4-(trifluoromethyl) phenyl) cyclohexyl) carbamate (1.52 g, 4.09 mmol) was dissolved in dichloromethane (20 mL), cooled to 0 °C, TFA was added (5 mL), and the reaction was stirred at room temperature for 1 h. TLC (PE/EA=10/1) detected the raw material was reacted completely, then spun-dried the solvent, then dissolved in dichloromethane (50 mL), the pH was adjusted to about 8 with saturated sodium bicarbonate aqueous solution to , partitioned, the aqueous phase was extracted with dichloromethane (50 mL × 2), the combined organic phase was washed with saturated NaCl aqueous solution, dried over anhydrous sodium sulfate, filtrated, the filtrate was concentrated to dryness to obtain a light yellow oil 1.13 g, crude yield: 101.8%. MS[M+H]⁺: 272.

### Step b: Preparation of 12-b

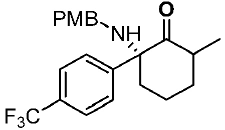

1.13 g of crude compound 12-a was dissolved in DCE (12 mL), ice acetic acid (51 mg, 0.84 mmol) and p-methoxybenzaldehyde (744 mL, 5.46 mmol) were added, and the reaction was carried out at room temperature for 16 h. The reaction was then cooled down to 0°C, and sodium triacetoxyborohydride (1.34 g, 6.32 mmol) was added in batches and reacted at room temperature for 1 h. TLC (PE/EA=3/1) detected the raw material was reacted completely, then adjusted the pH to about 8 with saturated aqueous sodium bicarbonate, diluted with water (100 mL), extracted with dichloromethane (30 mL×3), the combined organic phase was washed with saturated aqueous NaCl, dried over anhydrous sodium sulfate, filtered, and the filtrate was concentrated to dryness and , and subjected to a column chromatography (PE/EA = 5/1) to obtain 1.4 g of light yellow solid, the yield: 87.4% (total of two steps). ¹H NMR (400 MHz, CDCl₃) δ 7.66 (d, *J =* 8.1 Hz, 2H), 7.40 (d, *J =* 8.1 Hz, 2H), 7.12 (d, *J =* 8.2 Hz, 2H), 6.78 (d, *J =* 8.3 Hz, 2H), 3.75 (s, 3H), 3.31 (d, *J =* 12.0 Hz, 1H), 3.20 (d, *J =* 12.1 Hz, 1H), 3.01 (d, *J =* 12.1 Hz, 1H), 2.39 (dt, *J* = 12.6, 6.3 Hz, 1H), 2.04 - 1.91 (m, 2H), 1.80 (m, 2H), 1.48 (m, 1H), 1.05 (d, *J =* 6.4 Hz, 3H). MS[M+H]⁺: 392.

### Step c: Preparation of 12-c

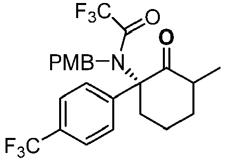

Compound 12-b (600 mg, 1.53 mmol) was dissolved in THF (10 mL), triethylamine (465 mg, 4.59 mmol) and DMAP (94 mg, 0.77 mmol) were added, and trifluoroacetic anhydride (644 mg, 3.06 mmol) was added dropwise in an ice bath, and the reaction was carried out at room temperature for 1 h. TLC (PE/EA=5/1) detected the raw material was reacted completely, the reaction was then quenched with saturated aqueous sodium bicarbonate, extracted with ethyl acetate (30 mL×3), the combined organic phase was washed with saturated aqueous NaCl, dried over anhydrous sodium sulfate, filtered, the filtrate was concentrated o dryness, and subjected to a column chromatography (PEÆA = 20/1) to obtain 740 mg of colorless mucilage , yield: 99.1%. ¹H NMR (400 MHz, CDCl₃) δ 7.46 (d, *J =* 8.3 Hz, 2H), 7.32 (d, *J =* 8.0 Hz, 2H), 6.65 (d, *J* = 8.5 Hz, 2H), 6.50 (d, *J* = 8.4 Hz, 2H), 4.59 (d, *J* = 16.9 Hz, 1H), 4.04 (d, *J =* 17.1 Hz, 1H), 3.77 (s, 3H), 2.80 (m, 1H), 2.43 (d, *J =* 14.2 Hz, 1H), 2.01 - 1.89 (m, 1H), 1.83 (m, 3H), 1.55 (m, 1H), 1.12 (d, *J* = 6.4 Hz, 3H). MS[M+H]⁺: 488.

### Step d: Preparation of 12-d

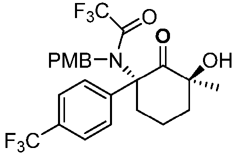

165 mg of white solid was prepared according to the method described in step b of Example 1 with compound 12-c (430 mg, 0.88 mmol) as a raw material, yield: 37.2%. ¹H NMR (400 MHz, DMSO-*d₆*) δ 7.53 (d, *J* = 8.1 Hz, 2H), 7.45 (d, *J* = 8.4 Hz, 2H), 6.69 (d, *J =* 8.3 Hz, 2H), 6.52 (d, *J =* 8.3 Hz, 2H), 4.89 (s, 1H), 4.47 (d, *J =* 17.7 Hz, 1H), 4.12 (d, *J =* 17.4 Hz, 1H), 3.69 (s, 3H), 2.40 (m, 2H), 1.84 - 1.65 (m, 3H), 1.54 (m, 1H), 1.20 (s, 3H). MS[M+H]⁺: 504.

### Step e: Preparation of 12-e

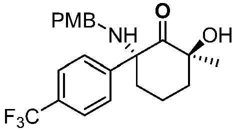

Compound 12-d (160 mg, 0.32 mmol) was dissolved in methanol (5 mL), 0.5 M aqueous sodium hydroxide solution (3 mL) was added dropwise under an ice bath, and the reaction was carried out at room temperature for 0.5 h. TLC (PE/EA=3/1) detected the raw material was reacted completely, then the reaction was diluted with water (20 mL), and extracted with ethyl acetate (10 mL×3), the combined organic phase was washed with saturated NaCl, dried over anhydrous sodium sulfate, and filtered, the filtrate was concentrated to dryness and subjected to a column chromatography (PE/EA=10/1-3/1) to obtain 110 mg colorless viscous oil in 85.3% yield. ¹H NMR (400 MHz, DMSO-*d₆*) δ 7.69 (d, *J =* 8.2 Hz, 2H), 7.53 (d, *J =* 8.2 Hz, 2H), 7.19 (d, *J =* 8.4 Hz, 2H), 6.86 (d, *J =* 8.4 Hz, 2H), 5.02 (s, 1H), 3.72 (s, 3H), 3.28 (d, *J =* 5.8 Hz, 1H), 3.00 (s, 1H), 2.98 (s, 1H), 2.27 (t, *J =* 10.3 Hz, 1H), 1.97 - 1.88 (m, 2H), 1.84 (d, *J* = 5.5 Hz, 2H), 1.71 (m, 1H), 1.39 (s, 3H). MS[M+H]⁺: 408.

### Step f: Preparation of compound 12

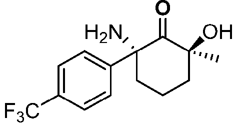

Compound 12-e (110 mg, 0.27 mmol) was dissolved in trifluoroacetic acid (2 mL), and stirred at 100°C for 16 h. TLC (PE/EA=3/1) detected the raw material was reacted completely, then rotatory evaporated to remove trifluoroacetic acid, saturated aqueous sodium carbonate (20 mL) was added, extracted with dichloromethane (10 mL×3), the organic phases were combined and washed with saturated aqueous NaCl, dried over anhydrous sodium sulfate, filtered, the filtrate was concentrated and subjected to a column chromatography (DCM/MeOH=150/1-80/1) to obtain 40 mg of yellowish oil in 89.7% yield. ¹H NMR (400 MHz, CD₃OD) δ 7.63 (d, *J* = 8.3 Hz, 2H), 7.54 (d, *J =* 8.2 Hz, 2H), 2.86 - 2.76 (m, 1H), 2.08 (qd, *J* = 13.5, 7.0 Hz, 1H), 2.00 - 1.91 (m, 1H), 1.84 (ddd, *J* = 14.3, 12.1, 3.6 Hz, 1H), 1.74 (ddt, *J* = 15.6, 8.0, 4.1 Hz, 2H), 1.33 (s, 3H). MS[M+H]⁺: 288.

### Example 13: Preparation of (2S, 6R)-2-amino-6-hydroxy-6-methyl-2-(4-(trifluoromethyl)phenyl)cyclohexan-1-one (Compound 13)

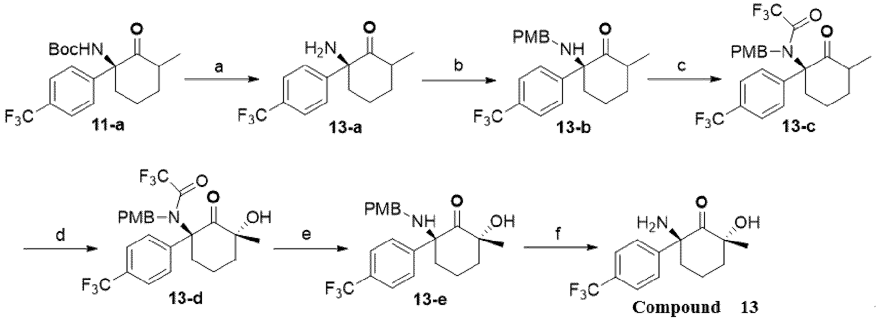

### Step a: Preparation of 13-a

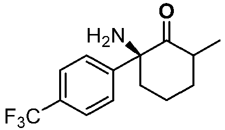

1.51 g of a pale yellow oily crude product was obtained according to the method described in step a of Example 12 with compound 11-a (2.1 g, 5.65 mmol) as a raw material, yield: 98.4%. MS[M+H]⁺: 272.

### Step b: Preparation of 13-b

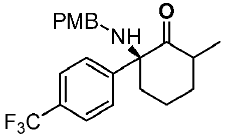

1.88 g of white solid was obtained according to the method described in step b of Example 12 with the crude product of the above step compound 13-a (1.51 g) as raw material, yield: 85.0% (total of two steps). ¹H NMR (400 MHz, CDCl₃) δ 7.66 (d, *J =* 8.1 Hz, 2H), 7.40 (d, *J* = 8.1 Hz, 2H), 7.12 (d, *J* = 8.2 Hz, 2H), 6.78 (d, *J* = 8.2 Hz, 2H), 3.75 (s, 3H), 3.31 (d, *J =* 12.0 Hz, 1H), 3.20 (d, *J =* 12.1 Hz, 1H), 3.01 (d, *J =* 12.1 Hz, 1H), 2.38 (m, 1H), 2.03 - 1.90 (m, 2H), 1.80 (m, 2H), 1.48 (m, 1H), 1.05 (d, *J =* 6.3 Hz, 3H). MS[M+H]⁺: 392.

### Step c: Preparation of 13-c

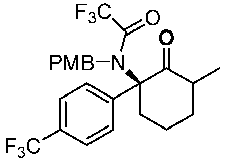

610 mg of colorless oil was prepared according to the method described in step c of Example 12 with the above compound 13-b (500 mg, 1.28 mmol) as raw material, yield: 97.9%. ¹H NMR (400 MHz, CDCl₃) δ 7.46 (d, *J* = 8.3 Hz, 2H), 7.32 (d, *J* = 8.0 Hz, 2H), 6.65 (d, *J =* 8.5 Hz, 2H), 6.50 (d, *J =* 8.4 Hz, 2H), 4.59 (d, *J =* 16.9 Hz, 1H), 4.04 (d, *J =* 17.1 Hz, 1H), 3.77 (s, 3H), 2.80 (m, 1H), 2.43 (d, *J =* 14.2 Hz, 1H), 2.01 - 1.89 (m, 1H), 1.83 (m, 3H), 1.55 (m, 1H), 1.12 (d, *J* = 6.3 Hz, 3H). MS[M+H]⁺: 488.

### Step d: Preparation of 13-d

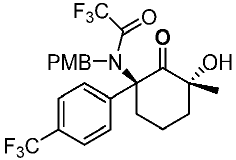

178 mg of white solid was prepared according to the method described in step b of Example 1 with compound 13-c (400 mg, 0.82 mmol) as a raw material, yield: 43.1%. ¹H NMR (400 MHz, DMSO-*d₆*) δ 7.53 (d, *J* = 8.1 Hz, 2H), 7.45 (d, *J* = 8.4 Hz, 2H), 6.69 (d, *J* = 8.3 Hz, 2H), 6.52 (d, *J* = 8.3 Hz, 2H), 4.90(s, 1H), 4.47 (d, *J* = 17.7 Hz, 1H), 4.12 (d, *J =* 17.4 Hz, 1H), 3.69 (s, 3H), 2.40 (m, 2H), 1.83 - 1.63 (m, 3H), 1.53 (m, 1H), 1.20 (s, 3H). MS[M+H]⁺: 504.

### Step e: Preparation of 13-e

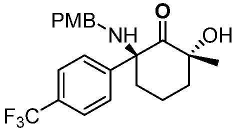

65 mg of colorless oil was prepared according to the method described in step e of Example 12 with compound 13-d (100 mg, 0.20 mmol) as a raw material, yield: 80.2%. ¹H NMR (400 MHz, DMSO-*d₆*) δ 7.69 (d, *J* = 8.2 Hz, 2H), 7.53 (d, *J* = 8.2 Hz, 2H), 7.19 (d, *J* = 8.4 Hz, 2H), 6.86 (d, *J* = 8.4 Hz, 2H), 5.02 (s, 1H), 3.72 (s, 3H), 3.28 (d, *J* = 5.8 Hz, 1H), 3.00 (s, 1H), 2.98 (s, 1H), 2.27 (t, *J =* 10.3 Hz, 1H), 1.98 - 1.87 (m, 2H), 1.84 (d, *J =* 5.5 Hz, 2H), 1.70 (m, 1H), 1.39 (s, 3H). MS[M+H]⁺: 408.

### Step f: Preparation of 13

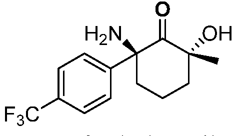

18 mg of colorless oil was prepared according to the method described in step f of Example 12 with Compound 13-e (50 mg, 0.12 mmol) as a raw material, yield: 51.4%. ¹H NMR (400 MHz, CD₃OD) δ 7.62 (d, *J =* 8.4 Hz, 2H), 7.54 (d, *J =* 8.2 Hz, 2H), 2.80 (dd, *J* = 14.5, 3.1 Hz, 1H), 2.14 - 2.01 (m, 1H), 1.99 - 1.90 (m, 1H), 1.85 (dd, *J* = 15.0, 3.0 Hz, 1H), 1.83 - 1.69 (m, 2H), 1.32 (s, 3H). MS[M+H]⁺: 288.

### Example 14: Preparation of 2-(2-fluoro-3-(trifluoromethoxy)phenyl)-6-hydroxy-6-methyl-2-(methylamino)cyclohexan-1-one (Compound 14)

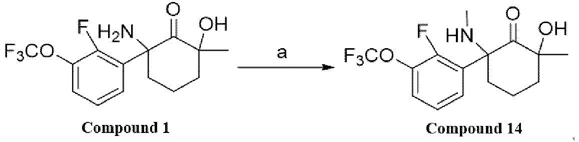

Compound 1 (230 mg, 0.72 mmol) and 37% formaldehyde aqueous solution (58 mg, 0.72 mmol) were dissolved in formic acid (3 mL), and the temperature was raised to 100 °C and stirred for 3 h. The reaction solution was cooled to room temperature, saturated sodium carbonate aqueous solution was added to adjust the pH to about 10, and extracted with ethyl acetate (15 mL×3), the organic phases were combined and washed with saturated NaCl aqueous solution, dried over anhydrous sodium sulfate, filtered, the filtrate was spun-dried and subjected to a column chromatography (PE/EA = 10/1) to obtain 110mg of light yellow oil in 45.8% yield. ¹H NMR (400 MHz, CDCl₃) δ 7.31 (t, *J* = 7.5 Hz, 1H), 7.25 - 7.16 (m, 2H), 2.46 - 2.36 (m, 1H), 2.16 (s, 3H), 2.15 - 2.05 (m, 2H), 1.98 (m, 1H), 1.94 - 1.86 (m, 1H), 1.72 (m, 1H), 1.16 (s, 3H). MS[M+H]⁺. 336.

### Example 15: Preparation of (2R,6S)-2-(2-fluoro-3-(trifluoromethoxy)phenyl)-6-hydroxy-6-methyl-2-(methylamino)cyclohexan-1-one (Compound 15)

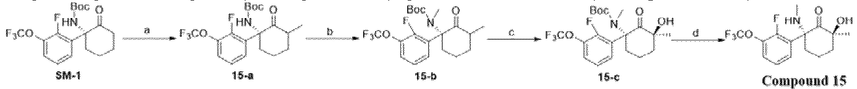

### Step a: Preparation of 15-a

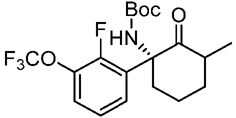

1.05 g of a colorless oil was prepared according to the method described in step a of Example 1 with Tert-butyl (R)(1-(2-fluoro-3-(trifluoromethoxy)phenyl)-2-oxocyclohexyl) carbamate (1.7 g, 4.34 mmol) as a raw material, yield: 59.6 %. ¹H NMR (400 MHz, CDCl₃) δ 7.71 (s, 1H), 7.25 - 7.17 (m, 2H), 6.62 (s, 1H), 3.79 (m, 1H), 2.46 (m, 1H), 2.01 (m, 1H), 1.85 - 1.62 (m, 3H), 1.42 (m, 1H), 1.31 (s, 9H), 1.06 (d, *J =* 6.2 Hz, 3H). MS[M+H]⁺: 406.

### Step b: Preparation of 15-b

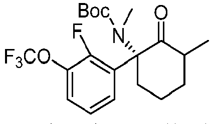

Tert-butyl ((1R)- 1-(2-fluoro-3-(trifluoromethoxy)phenyl)-3-methyl-2-oxocyclohexyl)carbamate (1.1 g, 2.71 mmol) was dissolved in anhydrous THF (15 mL), cooled to -78°C, protected by N2 gas, and LDA (2.62 mL, 5.24 mmol) was added slowly dropwise, then stirred at -78°C for 1 h. Iodomethane (0.25 mL, 3.93 mmol) was dissolved in THF (1 mL), and slowly dripped into the reaction solution at -78°C, then naturally raised to room temperature and reacted for 3 h. The reaction was detected by TLC (PE/EA=10/1), and a small amount of raw material was left, the reaction was then quenched with saturated aqueous NH4Cl (10 mL), diluted with water (40 mL), and extracted with ethyl acetate (15 mL×3), the organic phases were combined, washed with saturated aqueous NaCl, dried over anhydrous sodium sulfate, filtered, and the filtrate was concentrated o dryness, and subjected to a column chromatography (PE/EA=20/1) to obtain 850 mg of white solid in 74.7% yield. ¹H NMR (400 MHz, CDCl₃) δ 7.26 (s, 1H), 7.09 (t, *J* = 8.0 Hz, 1H), 6.76 (s, 1H), 3.05 (s, 3H), 2.79 (m, 1H), 2.58 (m, 1H), 2.31 (m, 1H), 2.08 (m, 1H), 1.75 (m, 3H), 1.49 (s, 9H), 1.11 (s, 3H). MS [M+Na]⁺: 442.27.

### Step c: Preparation of 15-c

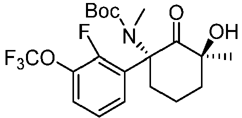

162 mg as white solid was prepared according to the method described in step b of Example 1 with compound 15-b (350 mg, 0.83 mmol) as a raw material, yield: 44.6%. MS[M+H]⁺: 436.

### Step d: Preparation of compound 15

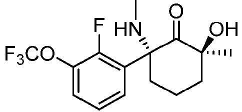

91 mg of colorless oil was prepared according to the method described in step c of Example 1 Compound 15-c (150 mg, 0.34 mmol) as a raw material, yield: 78.4%. ¹H NMR (400 MHz, CDCl₃) δ 7.32 (t, *J =* 7.0 Hz, 1H), 7.24 (m, 1H), 7.16 (t, *J =* 8.1 Hz, 1H), 2.25 - 2.18 (m, 4H), 2.17 - 2.08 (m, 1H), 2.05 - 1.94 (m, 4H), 1.60 (s, 3H). MS[M+H]⁺: 336.

### Example 16: Preparation of 2-hydroxy-2-methyl-6-methylamino-6-(2,4,6-trifluorotoluene)cyclohexan-1-one (Compound 16)

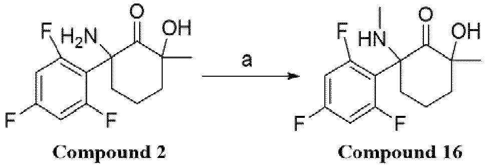

30 mg of a light yellow oil was prepared according to the method described in Example 14 with compound 2 (75 mg, 0.27 mmol) as a raw material, yield: 38.0%. ¹H NMR (400 MHz, CDCl₃) δ 6.67 (t, *J =* 9.6 Hz, 2H), 2.51 - 2.42 (m, 1H), 2.30 (m, 1H), 2.26 (s, 3H), 2.13 (m, 1H), 1.99 (m, 1H), 1.90 - 1.80 (m, 1H), 1.64 (m, 1H), 1.23 (s, 3H). MS [M+H]⁺:288.

### Example 17: Preparation of (2S,6R)-2-hydroxy-2-methyl-6-methylamino-6-(2,4,6-trifluorophenyl)cyclohexan-1-one (Compound 17)

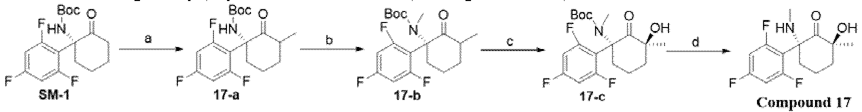

### Step a: Preparation of 17-a

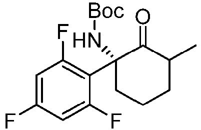

2.8 g of colorless oil was prepared according to the method described in Step a of Example 1 with Tert-butyl (R)(2-oxo-1-(2,4,6-trifluorophenyl)cyclohexyl)carbamate (3.1 g, 9.03 mmol) as a raw material, yield: 86.8 %. ¹H NMR (400 MHz, CDCl₃) δ 6.67 (t, *J =* 9.0 Hz, 2H), 6.55 (s, 1H), 3.82 (d, *J =* 8.0 Hz, 1H), 2.56 (m, 1H), 2.03 (m, 1H), 1.88 - 1.65 (m, 3H), 1.49 - 1.43 (m, 1H), 1.34 (s, 9H), 1.06 (d, *J* = 6.1 Hz, 3H). MS [M+H]⁺: 358.

### Step b: Preparation of 17-b

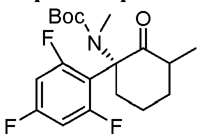

1.68 g of white solid was prepared according to the method described in step b of Example 15 with compound 17-a (2.0 g, 5.60 mmol) as a raw material, yield: 80.8 %. MS (M+H)⁺:372.

### Step c: Preparation of 17-c

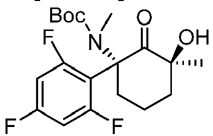

600 mg of white foam was prepared according to the method described in step b in Example 1 with compound 17-b (1.2 g, 3.23 mmol) as a raw material, yield: 47.9 %. MS (M+H)⁺:388.

### Step d: Preparation of compound 17

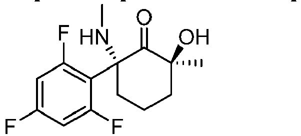

265 mg of colorless oil was obtained according to the method described in step c in Example 1 with compound 17-c (400 mg, 1.03 mmol) as a raw material, yield: 89.2%. ¹H NMR (400 MHz, CDCl₃) 6.68 (t, *J =* 9.5 Hz, 1H), 2.21 (m, 1H), 2.12 (s, 3H), 2.08 - 2.01 (m, 2H), 1.93 (m, 2H), 1.82 (m, 1H), 1.56 (s, 3H).MS (M+H)⁺:288.

### Example 18: Preparation of 2-(4-chloro-3-(trifluoromethoxy)phenyl)-6-hydroxy-6-methyl-2-methylamino-cyclohexan-1-one (Compound 18)

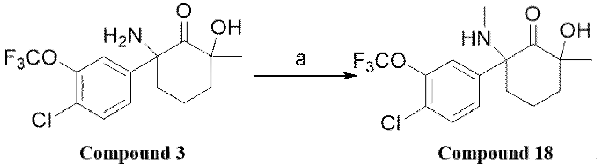

35 mg of white solid was prepared according to the method described in Example 14 with compound 3 (145 mg, 0.43 mmol) as a raw material, yield: 23.2%. ¹H NMR (400 MHz, CDCl₃) δ 7.35 (m, 3H), 2.37 (t, *J* = 11.1 Hz, 1H), 2.26 - 2.19 (m, 1H), 2.17 - 2.06 (m, 4H), 2.01 (m, 1H), 1.93 - 1.84 (m, 1H), 1.76 - 1.68 (m, 1H), 1.22 (s, 3H). MS (M+H)⁺: 352.

### Example 19: Preparation of (2R, 6S)-2-(4-chloro-3-(trifluoromethoxy) phenyl)-6-hydroxy-6-methyl-2-methylamino-cyclohexan-1-one (Compound 19)

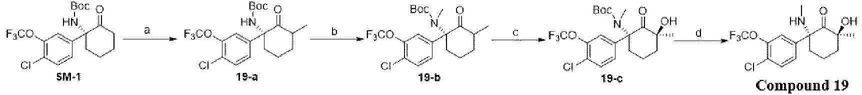

### Step a: Preparation of 19-a

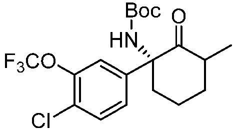

2.5 g of a colorless oil was prepared according to the method described in step a of Example 1 with Tert-butyl (R)-(1-(4-chloro-3-(trifluoromethoxy)phenyl)-2-oxocyclohexyl) carbamate (3.0 g, 7.36 mmol) as a raw material, yield: 80.6 %. ¹H NMR (400 MHz, CDCl₃) δ 7.82 (d, *J* = 7.4 Hz, 1H), 7.40 (t, *J* = 8.2 Hz, 1H), 7.32 (d, *J* = 8.2 Hz, 1H), 6.66 (s, 1H), 3.82 (d, *J =* 11.9 Hz, 1H), 2.46 - 2.36 (m, 1H), 2.02 (m, 1H), 1.83 - 1.58 (m, 3H), 1.45 (m, 1H), 1.31 (s, 9H), 1.06 (d, *J =* 6.3 Hz, 3H). MS (M+H)⁺: 422.

### Step b: Preparation of 19-b

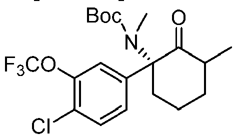

1.52 g of white solid was prepared according to the method described in step b of Example 15 with compound 19-a (2.0 g, 4.74 mmol) as a raw material, yield: 73.5 %. MS (M+H)⁺:436.

### Step c: Preparation of 19-c

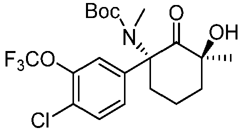

570 mg of colorless oil was prepared according to the method described in step b in Example 1 with compound 19-b (1.4 g, 3.21 mmol) as a raw material, yield: 39.3 %. MS (M+H)⁺:452.

### Step d: Preparation of compound 19

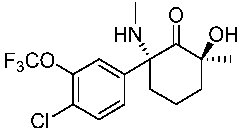

120 mg of colorless oil was prepared according to the method described in step c of Example 1 with compound 19-c (200 mg, 0.44 mmol) as a raw material, yield: 76.9%. ¹H NMR (400 MHz, CDCl₃) δ 7.43 (m, 1H), 7.20 - 6.98 (m, 2H), 2.20 (m, 4H), 2.13 (m, 1H), 2.01 (m, 1H), 1.95 (m, 2H), 1.85 - 1.78 (m, 1H), 1.54 (s, 3H). MS (M+H)⁺:352.

### Example 20: Preparation of 2-(3-fluoro-2-(trifluoromethyl)phenyl)-6-hydroxy-6-methyl-2-methylamino-cyclohexan-1-one (Compound 20)

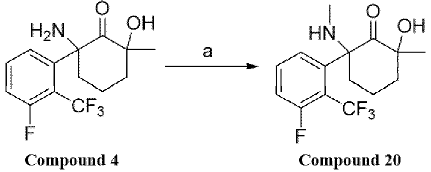

70 mg of white solid was prepared according to the method described in Example 14 with compound 4 (300 mg, 0.98 mmol) as a raw material yield: 22.3 %. ¹H NMR (400 MHz, CDCl₃) δ 7.52 (dd, *J* = 14.1, 7.1 Hz, 1H), 7.33 (m, 1H), 7.16 (t, *J* = 9.1 Hz, 1H), 2.26 - 2.00 (m, 6H), 1.81 (m, 1H), 1.67 (m, 2H), 1.36 (s, 3H). MS (M+H)⁺:320.

### Example 21: Preparation of 3-(3-hydroxy-3-methyl-1-methylamino-2-oxocyclohexyl)benzonitrile (Compound 21)

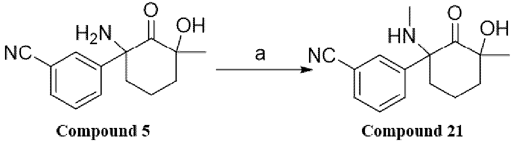

50 mg of white solid was prepared according to the method described in Example 14 with compound 5 (200 mg, 0.82 mmol) as a raw material, yield: 23.7 %. ¹H NMR (400 MHz, CDCl₃) δ 7.65 - 7.55 (m, 2H), 7.54 - 7.45 (m, 2H), 2.67 (d, *J =* 14.2 Hz, 1H), 2.09 - 1.92 (m, 7H), 1.79 - 1.66 (m, 1H), 0.90 (s, 3H). MS (M+H)⁺: 259.

### Example 22: Preparation of 3-((1R, 3S)-3-hydroxy-3-methyl-1-methylamino-2-oxocyclohexyl)benzonitrile (Compound 22)

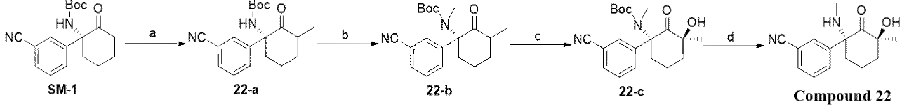

### Step a: Preparation of 22-a

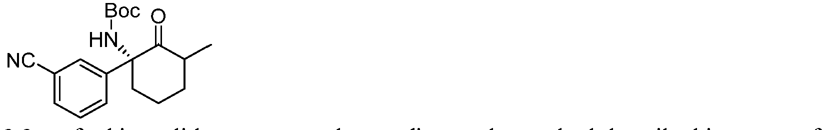

2.2 g of white solid was prepared according to the method described in step a of Example 1 with Tert-butyl (R) (1-(3-cyanophenyl)-2-oxocyclohexyl)carbamate (3.0 g, 9.54 mmol) as a raw material, yield: 70.2 %. ¹H NMR (400 MHz, CDCl₃) δ 7.61 (m, 3H), 7.47 (t, *J* = 7.4 Hz, 1H), 6.59 (s, 1H), 3.73 (m, 1H), 2.30 (m, 1H), 2.03 (m, 1H), 1.85 (m, 3H), 1.50 - 1.42 (m, 1H), 1.30 (s, 9H), 1.02 (d, *J* = 6.2 Hz, 3H). MS [M+H]⁺: 329.

### Step b: Preparation of 22-b

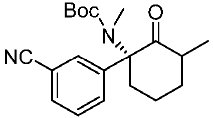

1.22 g of white solid was prepared according to the method described in step b of Example 15 with compound 22-a (2.0 g, 6.09 mmol) as a raw material, yield: 58.5 %. ¹H NMR (400 MHz, CDCl₃) δ 7.57 (d, *J* = 6.7 Hz, 1H), 7.51 - 7.41 (m, 3H), 3.05 (s, 1H), 2.89 (s, 3H), 2.41 (m, 1H), 2.10 - 2.01 (m, 2H), 1.80 (m, 3H), 1.50 (s, 9H), 1.09 (d, *J* = 6.4 Hz, 3H). MS (M+H)⁺:343.

### Step c: Preparation of 22-c

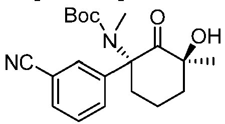

390 mg of colorless oil was prepared according to the method described in step b of Example 1 with compound 22-b (800 mg, 2.34 mmol) as a raw material yield: 46.6 %. MS (M+H)⁺:359.

### Step d: Preparation of compound 22

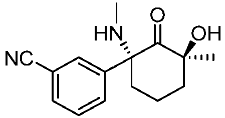

90 mg of a colorless oil was obtained according to the method described in step c of Example 1 with compound 22-c (200 mg, 0.56 mmol) as a raw material, yield: 62.5 %. ¹H NMR (400 MHz, CDCl₃) δ 7.62 (s, 1H), 7.56 (d, *J* = 7.1 Hz, 1H), 7.46 (dq, *J* = 15.6, 7.9 Hz, 2H), 2.18 - 2.08 (m, 5H), 2.03 (m, 1H), 1.92 (m, 2H), 1.85 - 1.78 (m, 1H), 1.56 (s, 3H). MS (M+H)⁺:259.

### Example 23: Preparation of 2-(2,3-difluorophenyl)-6-hydroxy-6-methyl-2-methylaminocyclohexan-1-one (Compound 23)

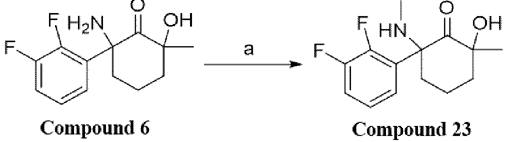

73 mg of white solid was prepared according to the method described in Example 14 with compound 6 (300 mg, 1.18 mmol) as a raw material, yield: 23.1 %. ¹H NMR (400 MHz, CDCl₃) δ 7.20 - 7.07 (m, 2H), 7.03 (t, *J* = 6.8 Hz, 1H), 2.43 - 2.34 (m, 1H), 2.16 (m, 4H), 2.13 - 2.04 (m, 1H), 2.00 (m, 1H), 1.86 (m, 1H), 1.77 - 1.66 (m, 1H), 1.17 (s, 3H). MS (M+H)⁺: 270.

### Example 24: Preparation of (2R, 6S)-2-(2,3-difluorophenyl)-6-hydroxy-6-methyl-2-methylaminocyclohexan-1-one (Compound 24)

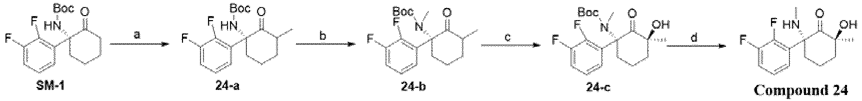

### Step a: Preparation of 24-a

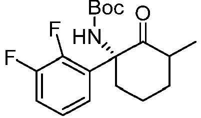

1.2 g of white solid was prepared according to the method described in Step a of Example 1 with Tert-butyl ((1R)- 1-(2,3-difluorophenyl)-3-methyl-2-oxocyclohexyl) carbamate (1.3 g, 4.0 mmol) as a raw material, yield: 88.5%. ¹H NMR (400 MHz, CDCl₃) δ 7.50 (s, 1H), 7.14 (m, 2H), 6.61 (s, 1H), 3.81 (m, 1H), 2.49 (m, 1H), 2.02 (m, 1H), 1.80 - 1.71 (m, 2H), 1.65 (m, 1H), 1.48 - 1.37 (m, 1H), 1.32 (s, 9H), 1.06 (d, *J =* 6.2 Hz, 3H). MS[M+H]⁺: 340.

### Step b: Preparation of 24-b

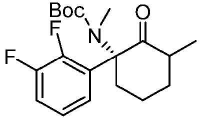

350 mg of a colorless oil was prepared according to the method described in step b of Example 15 with compound 24-a (1.2 g, 3.54 mmol) as a raw material was prepared in 28 % yield. MS [M+H]⁺: 354.36.

### Step c: Preparation of 24-c

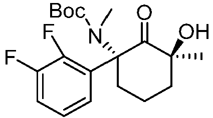

150 mg of white solid was obtained according to the method described in step b in Example 1 with compound 24-b (350 mg, 0.99 mmol) as a raw material, yield: 41%. MS [M+H]⁺:370.37.

### Step d: Preparation of compound 24

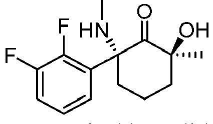

85 mg of white solid was prepared according to the method described in step c of Example 1 with compound 24-c (150 mg, 0.41 mmol) as a raw material, yield: 79.0%. ¹H NMR (400 MHz, CDCl₃) δ 7.10 (m, 3H), 2.21 (m, 4H), 2.13 (m, 1H), 1.99 (m, 3H), 1.77 (m, 1H), 1.60 (s, 3H). MS [M+H]⁺: 270.

### Example 25: Preparation of 2-hydroxy-2-methyl-6-methylamino-6-(3-(trifluoromethoxy)phenyl)cyclohexan-1-one (Compound 25)

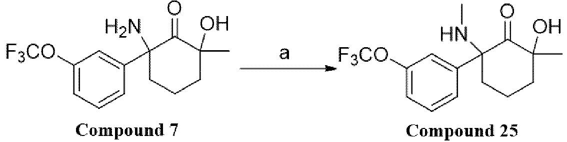

75 mg of white solid was prepared according to the method described in Example 14 with compound 7 (200 mg, 0.66 mmol) as a raw material, yield: 35.9 %. ¹H NMR (400 MHz, CDCl₃) δ 7.41 (t, *J* = 8.0 Hz, 1H), 7.18 (t, *J* = 7.3 Hz, 2H), 7.10 (s, 1H), 2.77 (d, *J* = 15.8 Hz, 1H), 2.02 (s, 3H), 2.00 - 1.84 (m, 4H), 1.80 - 1.69 (m, 1H), 0.84 (s, 3H). MS [M+H]⁺: 318.

### Example 26: Preparation of (2S,6R)-2-hydroxy-2-methyl-6-methylamino-6-(3-(trifluoromethoxy)phenyl)cyclohexan-1-one (Compound 26)

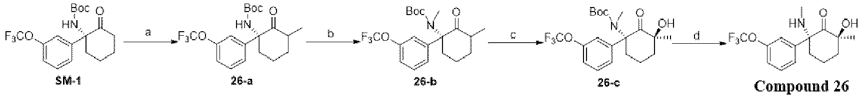

### Step a: Preparation of 26-a

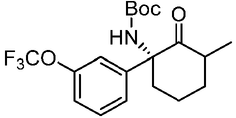

1.4 g of white solid was prepared according to the method described in step a of Example 1 with Tert-butyl (R)-(2-oxo-1-(3-(trifluoromethoxy)phenyl)cyclohexyl) carbamate (2.5 g, 6.70 mmol) as a raw material, yield: 53.9 %. ¹H NMR (400 MHz, CDCl₃) δ 7.39 (t, *J =* 8.1 Hz, 1H), 7.29 (m, 1H), 7.14 (m, 2H), 6.56 (s, 1H), 3.71 (s, 1H), 2.34 (s, 1H), 2.03 - 1.97 (m, 1H), 1.88 (m, 3H), 1.47 - 1.40 (m, 1H), 1.30 (s, 9H), 1.01 (d, *J =* 6.3 Hz, 3H). MS [M+H]⁺:388.

### Step b: Preparation of 26-b

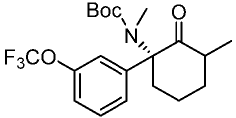

685 mg of a colorless oil was prepared according to the method described in step b of Example 15 with compound 26-a (1.47 g, 3.79 mmol) as a raw material, as a mixture of diastereoisomers in a ratio of about 1:1.8, yield: 44.9 %.¹H NMR (400 MHz, CDCl₃) δ 7.35 (t, *J* = 7.6 Hz, 3H), 7.19 - 7.02 (m, 7.2H), 6.95 (s, 1H), 3.04 (s, 4H), 2.88 (s, 7.2H), 2.38 (m, 2.8H), 2.03 (m, 5.6H), 1.91 - 1.70 (m, 5.6H), 1.49 (s, 16.2H), 1.41 (m, 1.8H), 1.30 (s, 9H), 1.25 (s, 1H), 1.10 (m, 8.4H). MS [M+Na]⁺: 424.

### Step c: Preparation of 26-c

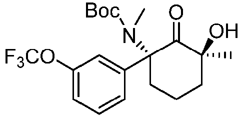

145 mg of colorless oil was prepared according to the method described in step b of Example 1 with compound 26-b (685 mg, 1.71 mmol) as a raw material, yield: 20.4 %. ¹H NMR (400 MHz, CDCl₃) δ 7.38 (t, *J =* 8.4 Hz, 1H), 7.16 (d, *J =* 8.3 Hz, 2H), 7.00 (s, 1H), 4.30 (s, 1H), 2.94 (s, 3H), 2.41 (d, *J =* 14.8 Hz, 1H), 2.23 (m, 1H), 2.11 (m, 1H), 1.76 (s, 2H), 1.49 (s, 9H), 1.26 (s, 3H). MS [M+Na]⁺: 440.

### Step d: Preparation of compound 26

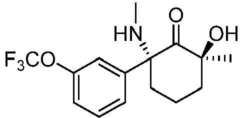

80 mg of white solid was obtained according to the method described in step c of Example 1 with compound 26-c (140 mg, 0.34 mmol) as a raw material yield: 75.5 %. ¹H NMR (400 MHz, CDCl₃) δ 7.37 (t, *J* = 8.2 Hz, 1H), 7.18 - 7.09 (m, 3H), 2.22 (m, 1H), 2.12 (s, 3H), 2.03 (m, 2H), 1.92 (m, 2H), 1.81 (m, 1H), 1.55 (s, 3H). MS [M+H]⁺. 318.

### Example 27: Preparation of (2R,6S)-2-(2-chlorophenyl)-6-hydroxy-6-methyl-2-methylaminocyclohexan-1-one (Compound 27) and (2R,6R)-2-(2-chlorophenyl)-6-hydroxy-6-methyl-2-methylaminocyclohexan-1-one (Compound 28)

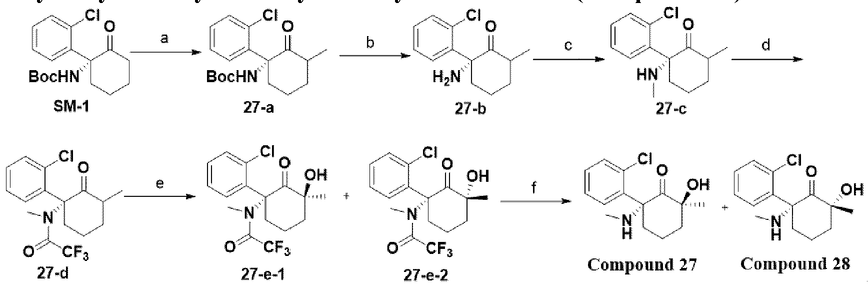

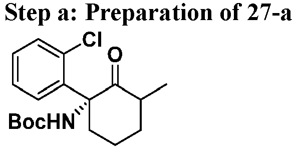

410 mg of white solid was obtained according to the method described in step a of Example 1 with tert-butyl (R)-(1-(2-chlorophenyl)-2-oxocyclohexyl)carbamate (2.1 g, 6.48 mmol) as a raw material, yield: 18.7%.¹H NMR (400 MHz, CDCl₃) δ 7.85 (s, 1H), 7.33 (m, 2H), 7.24 (m, 1H), 6.66 (s, 1H), 3.84 (m, 1H), 2.48 (m, 1H), 1.99 (m, 1H), 1.71 (m, 3H), 1.43 (dd, *J =* 12.4, 4.3 Hz, 1H), 1.06 (d, *J =* 6.3 Hz, 3H). MS [M+H]⁺: 338.

### Step b: Preparation of 27-b

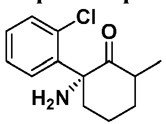

Compound 27-a (410 mg, 1.21 mmol) was dissolved in dichloromethane (5 mL), cooled to 0 °C, and TFA (2.5 mL) was added, then the reaction was raised to room temperature for 1 h. TLC (PE/EA=10/1) detected the raw material was reacted completely, spun-dried to remove the solvent, and dissolved in dichloromethane (20 mL), adjusted the pH to about 8 with saturated aqueous sodium bicarbonate, and partitioned. The aqueous phase was extracted with dichloromethane (10 mL×2), the organic phases were combined, washed with saturated aqueous NaCl, dried over anhydrous sodium sulfate, filtered, and the filtrate was concentrated to dryness to obtain 288 mg of colorless oil in 99.8% yield. MS[M+H]⁺: 238.

### Step c: Preparation of 27-c

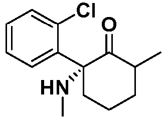

Compound 27-b 288 mg, 1.21 mmol) was dissolved in methanol 4 mL), ice acetic acid (15 mg, 0.24 mmol) and 38% aqueous formaldehyde (95.6 mg, 1.21 mmol) were added, and the reaction was carried out at room temperature for 2 h. Then it was cooled down to 0 °C, and sodium cyanoborohydride (114 mg, 1.82 mmol) was added in batches and the reaction was carried out for 1 h at room temperature. TLC (PE/EA=1/1) detected the raw material was reacted completely, then adjusted the pH to about 8 with saturated aqueous sodium bicarbonate solution, diluted with water (30 mL), and extracted with dichloromethane (15 mL×3), the organic phases were combined, washed with saturated aqueous NaCl, dried over anhydrous sodium sulfate, filtered, the filtrate was concentrated to dryness, and subjected to a column chromatography (EA/PE=0-3/2) to obtain 260 mg of colorless oil in 85.0% yield (total of two steps). MS[M+H]⁺. 252.

### Step d: Preparation of compound 27-d

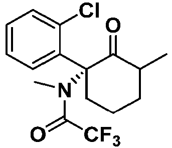

Compound 27-c (260 mg, 1.03 mmol) was dissolved in THF (4 mL), triethylamine (313 mg, 3.09 mmol) and DMAP (63 mg, 0.52 mmol) were added, trifluoroacetic anhydride (433 mg, 2.06 mmol) was added dropwise in an ice bath, and the reaction was carried out at room temperature for 2 h. TLC (PE/EA=10/1) detected the raw material was reacted completely, the reaction was then quenched with saturated ammonium chloride aqueous solution, and extracted with ethyl acetate (15 mL×3), the organic phases were combined, washed with saturated NaCl aqueous solution, dried over anhydrous sodium sulfate, filtered, the filtrate was concentrated to dryness, and subjected to a column chromatography (PE/EA=10/1) to obtain a white solid of 243 mg in 67.7% yield. ¹H NMR (400 MHz, CDCl₃) δ 7.47 - 7.43 (m, 1H), 7.39 - 7.34 (m, 1H), 7.31 (dd, *J =* 5.9, 3.5 Hz, 2H), 3.25 (dd, *J* = 14.1, 2.9 Hz, 1H), 2.95 (s, 3H), 2.77 - 2.65 (m, 2H), 2.02 - 1.92 (m, 1H), 1.83 - 1.74 (m, 1H), 1.66 (dt, *J* = 25.0, 7.9 Hz, 1H), 1.51 - 1.41 (m, 1H), 1.19 (d, *J =* 6.5 Hz, 3H). MS[M+H]⁺: 348.

### Step e: Preparation of compound 27-e-1 and 27-e-2

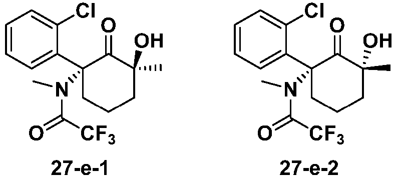

A white solid compound 27-e-1 (75 mg, yield: 21.7 %) was prepared according to the method described in step b in Example 1 with Compound 27-d (330 mg, 0.95 mmol) as a raw material.¹H NMR (400 MHz, CDCl₃) δ 7.49 (d, *J* = 7.2 Hz, 1H), 7.31 (dd, *J* = 13.8, 7.2 Hz, 2H), 7.21 (d, *J* = 7.2 Hz, 1H), 3.10 (dt, *J* = 14.3, 7.0 Hz, 1H), 2.99 (s, 3H), 2.80 - 2.72 (m, 1H), 2.07 - 1.98 (m, 1H), 1.95 - 1.85 (m, 1H), 1.82 - 1.73 (m, 2H), 1.54 (s, 3H). MS[M+H]⁺: 364.

The white solid compound 27-e-2 (50 mg, yield: 14.5%) was obtained. MS[M+H]⁺. 364.

### Step f: Preparation of compound 27 and 28

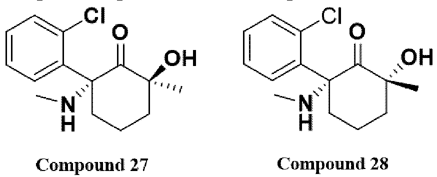

Compound 27-e-1 (25 mg, 0.069 mmol) was dissolved in methanol (1.5 mL), aqueous sodium hydroxide solution (0.5 mL) was added dropwise under an ice bath with 1 M, and the reaction was carried out at room temperature for 0.5 h. TLC (PE/EA=3/1) detected the raw material was reacted completely, then the reaction was diluted by adding water (20 mL), and extracted with ethyl acetate (10 mL×3), and the organic phases were combined, washed with saturated aqueous NaCl, dried over anhydrous sodium sulfate, and filtered, the filtrate was spun-dried to obtain 11 mg of colorless viscous oil in 61.1% yield. ¹H NMR (400 MHz, CDCl₃) δ 7.51 (d, *J =* 7.7 Hz, 1H), 7.36 (d, *J =* 7.7 Hz, 1H), 7.30 (t, *J =* 7.5 Hz, 1H), 7.22 (t, *J =* 7.5 Hz, 1H), 2.55 - 2.44 (m, 1H), 2.24 - 2.14 (m, 4H), 2.08 (m, 1H), 1.93 (d, *J =* 13.0 Hz, 1H), 1.88 - 1.80 (m, 2H), 1.56 (s, 3H). MS[M+H]⁺: 268.

12 mg of white solid (compound 28) was prepared according to the above method with Compound 27-e-2 (25 mg, 0.069 mmol) as a raw material, yield: 66.7 %. ¹H NMR (400 MHz, CDCl₃) δ 7.38 (t, *J* = 8.2 Hz, 2H), 7.29 (dd, *J* = 16.9, 8.4 Hz, 2H), 2.46 - 2.35 (m, 1H), 2.22 (m, 1H), 2.09 (m, 4H), 2.02 - 1.94 (m, 1H), 1.87 (m, 1H), 1.75 (m, 1H), 1.20 (s, 3H). MS[M+H]⁺: 268.

### Example 28: Preparation of (2R,6R)-2-hydroxy-2-methyl-6-methylamino-6-(4-(trifluoromethyl)phenyl)cyclohexan-1-one (Compound 29)

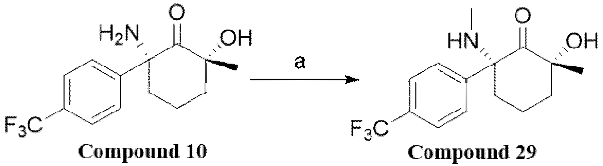

35 mg of white solid was prepared according to the method described in Example 14 with Compound 10 (200 mg, 0.70 mmol) as a raw material, yield: 16.7 %.¹H NMR (400 MHz, CDCl₃) δ 7.64 (d, *J* = 8.1 Hz, 2H), 7.38 (d, *J* = 8.1 Hz, 2H), 2.78 (d, *J =* 14.2 Hz, 1H), 2.05 - 1.96 (m, 6H), 1.89 (dd, *J* = 18.8, 9.6 Hz, 1H), 1.75 (dd, *J* = 26.7, 12.5 Hz, 1H), 0.86 (s, 3H). MS[M+H]⁺: 302.

### Example 29: Preparation of (2R,6R)-2-dimethylamino-6-hydroxy-6-methyl-2-(4-(trifluoromethyl)phenyl)cyclohexan-1-one (Compound 30)

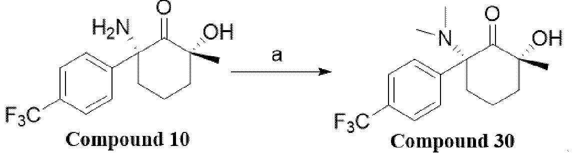

70 mg of white solid was prepared according to the method described in Example 14 with compound 10 (100 mg, 0.35 mmol), 38% aqueous solution of formaldehyde (5 ml) as raw materials, yield: 63.6 %.¹H NMR (400 MHz, CDCl₃) δ 7.63 (d, *J* = 8.2 Hz, 2H), 7.48 (d, *J* = 8.2 Hz, 2H), 2.72 - 2.64 (m, 1H), 2.22 - 2.07 (m, 8H), 2.05 - 1.96 (m, 1H), 1.86 - 1.71 (m, 2H), 1.08 (s, 3H). MS[M+H]⁺: 316.

### Example 30: Preparation of (2S,6R)-2-hydroxy-2-methyl-6-methylamino-6-(4-(trifluoromethyl)phenyl)cyclohexan-1-one (Compound 31)

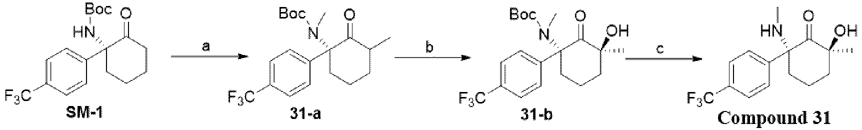

### Step a: Preparation of 31-a

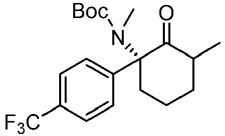

Tert-butyl (R)-(2-oxo-1-(4-(trifluoromethyl)phenyl)cyclohexyl)carbamate (5.5 g, 15.39 mmol) was dissolved in dry THF (60 mL), protected by Ar gas, cooled by ice-water bath, CH3I (5.5 g, 38.75 mmol) was added dropwise, and NaH (1.85 g, 46.2 mmol) was added in batches. The reaction was raised to room temperature for 1 h. TLC detected the raw material was reacted completely, then the reaction was diluted with water (100 mL), extracted with ethyl acetate (40 mL×3), the organic phases were combined, washed with saturated aqueous NaCl, dried over anhydrous sodium sulfate, filtered, rotatory evaporated to remove the low-boiling solvent, and then subjected to a column chromatography (PE/EA=15/1) to obtain 1.32 g of white solid, which was a diastereoisomeric mixture of about 1:1.7. Yield: 22.2 %. ¹H NMR (400 MHz, CDCl₃) δ 7.59 (d, *J =* 6.5 Hz, 5.4H), 7.33 (d, *J =* 7.7 Hz, 3.4H), 7.22 (d, *J =* 7.2 Hz, 2H), 3.06 (s, 4H), 2.89 (s, 6.8H), 2.42 (d, *J =* 14.4 Hz, 1.7H), 2.33 (d, *J =* 15.3 Hz, 1H), 2.17 - 2.03 (m, 5.4H), 1.83 (m, 5.4H), 1.50 (s, 15.3H), 1.46 - 1.39 (m, 2.7H), 1.30 (s, 9H), 1.10 (m, 8.1H). MS (M+Na)⁺: 408.

### Step b: Preparation of 31-b

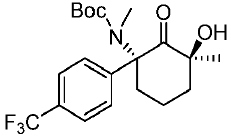

500 mg of yellow oil was prepared according to the method described in step b of Example 1 with compound 31-a (1.32 g, 3.42 mmol) as raw material, yield: 36.4%. MS (M+Na)⁺: 424.

### Step c: Preparation of compound 31

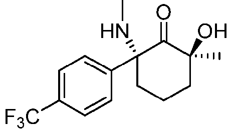

158 mg of white solid was prepared according to the method described in step c of Example 1 with compound 31-b (580 mg, 1.44 mmol) as a raw material, yield: 36.3%.¹H NMR (400 MHz, CDCl₃) δ 7.60 (d, *J* = 7.8 Hz, 2H), 7.38 (d, *J =* 8.0 Hz, 2H), 2.21 (m, 1H), 2.12 (s, 3H), 2.07 (m, 1H), 2.01 (m, 1H), 1.97 - 1.90 (m, 2H), 1.82 (m, 1H), 1.56 (s, 3H). MS (M+H)⁺: 302.

### Example 31: Preparation of (2S,6S)-2-hydroxy-2-methyl-6-methylamino-6-(4-(trifluoromethyl)phenyl)cyclohexan-1-one (Compound 32)

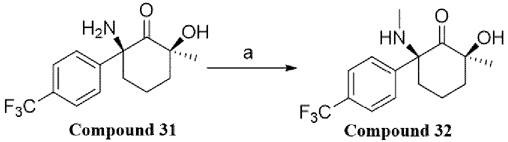

130 mg of white solid was obtained according to the method described in Example 14 with compound 11 (200 mg, 0.70 mmol) as a raw material, yield: 61.9%.¹H NMR (400 MHz, CDCl₃) δ 7.64 (d, *J* = 8.0 Hz, 2H), 7.38 (d, *J* = 8.1 Hz, 2H), 2.78 (d, *J =* 11.7 Hz, 1H), 2.07 - 1.95 (m, 6H), 1.89 (m, 1H), 1.75 (m, 1H), 0.86 (s, 3H). MS (M+H)⁺: 302.

### Example 32: Preparation of (2R,6S)-2-hydroxy-2-methyl-6-methylamino-6-(4-(trifluoromethyl)phenyl)cyclohexan-1-one (Compound 33)

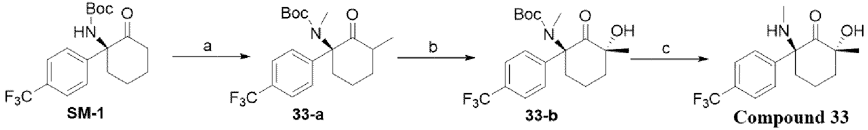

### Step a: Preparation of 33-a

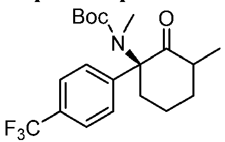

923 mg of white solid was obtained according to the method described in step a of Example 30 with tert-butyl (S)-(2-oxo-1-(4-(trifluoromethyl)phenyl)cyclohexyl)carbamate (2.0 g, 5.60 mmol) as a raw material, as a diastereoisomer mixture of about 1:1.7, yield: 42.8 %.¹H NMR (400 MHz, CDCl₃) δ 7.60 (t, *J* = 7.3 Hz, 5.4H), 7.32 (d, *J* = 8.0 Hz, 3.4H), 7.21 (d, *J* = 8.2 Hz, 2H), 3.06 (s, 4H), 2.89 (s, 6.8H), 2.40 (t, *J =* 11.1 Hz, 1.7H), 2.33 (d, *J =* 16.2 Hz, 1H), 2.18 - 2.03 (m, 5.4H), 1.90 - 1.75 (m, 5.4H), 1.50 (s, 15.3H), 1.48 - 1.39 (m, 2.7H), 1.30 (s, 9H), 1.10 (m, 8.1H). MS (M+H)⁺: 386.

### Step b: Preparation of 33-b

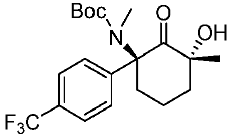

253mg of yellow oil was obtained according to the method described in step b in Example 1 with compound 33-a (915 mg, 2.37 mmol) as a raw material, yield: 26.5%. MS (M+Na)⁺: 424.

### Step c: Preparation of compound 33

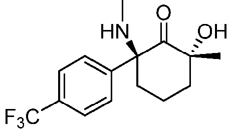

100 mg of white solid was obtained according to the method described in step c of Example 1 with compound 33-b (250 mg, 0.62 mmol) as a raw material, yield: 53.2 %.¹H NMR (400 MHz, CDCl₃) δ 7.60 (d, *J =* 8.0 Hz, 2H), 7.38 (d, *J =* 8.0 Hz, 2H), 2.26 - 2.18 (m, 1H), 2.12 (s, 3H), 2.07 (m, 1H), 2.01 (m, 1H), 1.93 (dd, *J =* 21.1, 10.8 Hz, 2H), 1.84 - 1.79 (m, 1H), 1.56 (s, 3H). MS (M+H)⁺: 302.

### Example 33: Preparation of (2S,6R)-2-hydroxy-2-methyl-6-trideutero-methylamino-6-(4-(trifluoromethyl)phenyl)cyclohexan-1-one (compound 34)

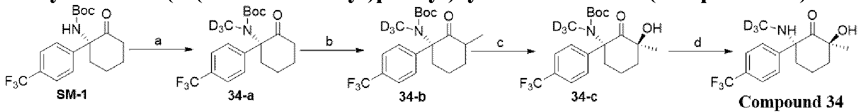

### Step a: Preparation of 34-a

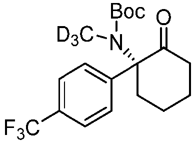

Tert-butyl (R)-(2-oxo-1-(4-(trifluoromethyl)phenyl)cyclohexyl)carbamate (2.5 g, 7.00 mmol) was dissolved in dry THF (40 mL), protected by Ar gas, cooled in ice-water bath, CD₃I (1.49 g, 10.50 mmol) was added dropwise, and NaH (420 mg, 10.50 mmol) was added in batches, the reaction was raised to room temperature for 30 min. TLC detected the raw material was reacted completely, the reaction was then quenched with water (100 mL), and extracted with ethyl acetate (50 mL×3). The organic phases were combined, washed with saturated aqueous NaCl, dried over anhydrous sodium sulfate, filtered, rotatory evaporated to remove the low-boiling solvent, and then subjected to a column chromatography (PE/EA=10/1-5/1) to obtain 1.55 g of white solid in 59.2% yield. ¹H NMR (400 MHz, CDCl₃) δ 7.61 (d, *J =* 8.2 Hz, 2H), 7.32 (d, *J =* 8.2 Hz, 2H), 2.80 (m, 1H), 2.47 (m, 2H), 2.14 (m, 2H), 1.80 (m, 3H), 1.43 (s, 9H). MS (M+Na)⁺: 397.

### Step b: Preparation of 34-b

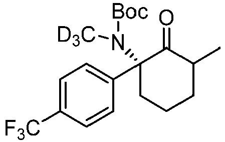

Compound 34-a (1.52 g, 4.06 mmol) was dissolved in dry THF (25 mL), protected by Ar gas, cooled to -78 °C, LDA (4.06 mL, 8.12 mmol) was added dropwise with stirring for 1 h, and CH₃I (0.86 g, 6.06 mmol) was added dropwise, then the reaction was slowly raised to room temperature overnight. TLC detected the raw material was reacted completely, then the reaction was quenched with saturated aqueous NH4Cl (100 mL), and extracted with ethyl acetate (50 mL x 3). The organic phases were combined, washed with saturated aqueous NaCl, dried over anhydrous sodium sulfate, filtered, rotatory evaporated to remove the low-boiling solvent, and subjected to a column chromatography (PE/EA=30/1-20/1) to obtain 770 mg of white solids, as a diastereoisomers mixture of about 1:1.4 in 48.8 % yield. ¹H NMR (400 MHz, CDCl₃) δ 7.60 (t, *J* = 7.5 Hz, 4.8H), 7.32 (d, *J* = 8.1 Hz, 2.8H), 7.21 (d, *J* = 8.3 Hz, 2H), 3.07 (m, 1H), 2.93 - 2.83 (m, 1.4H), 2.41 (m, 1.4H), 2.31 (m, 1H), 2.18 - 2.02 (m, 4.8H), 1.92 - 1.74 (m, 4.8H), 1.50 (s, 12.6H), 1.47 - 1.43 (m, 2.4H), 1.29 (s, 9H), 1.10 (m, 7.2H). MS (M+Na)⁺: 411.

### Step c: Preparation of 34-c

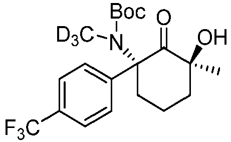

193 mg of white solids were obtained according to the method described in step b in Example 1 with compound 34-b (760 mg, 1.96 mmol) as a raw material, yield: 24.4 %. MS (M+Na)⁺:427.

### Step d: Preparation of compound 34

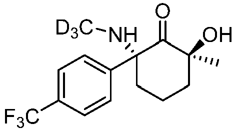

87 mg of white solids were obtained according to the method described in step c in Example 1 with compound 34-c (190 mg, 0.47 mmol) as a raw material, yield: 60.8 %.¹H NMR (400 MHz, CDCl₃) δ 7.60 (d, *J =* 8.0 Hz, 2H), 7.39 (d, *J =* 8.0 Hz, 2H), 2.21 (m, 1H), 2.08 (m, 1H), 2.00 (m, 1H), 1.96 - 1.87 (m, 2H), 1.84 - 1.80 (m, 1H), 1.56 (s, 3H). MS (M+H)⁺: 305.

### Example 34: Preparation of (2R,6S)-2-ethylamino-6-hydroxy-6-methyl-2-(4-(trifluoromethyl)phenyl)cyclohexan-1-one (compound 35)

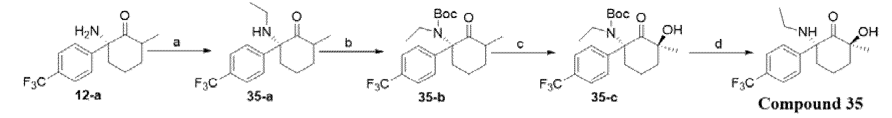

### Step a: Preparation of 35-a

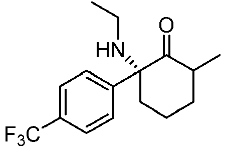

(2R)-2-Amino-6-methyl-2-(4-(trifluoromethyl)phenyl)cyclohexanone (1.13 g, 4.16 mmol) was dissolved in DCE (20 mL), ice acetic acid (50 mg, 0.83 mmol) and a solution of 5M acetaldehyde (0.91 mL, 4.58 mmol) in tetrahydrofuran were added, and the reaction was carried out at room temperature for 1 h, then cooled down to 0 °C, and sodium triacetoxyborohydride (1.32 g, 6.23 mmol) was added in batches and reacted at 0 °C for 1 h. TLC (PE/EA=1/1) detected the raw material was reacted completely, pH was adjusted to about 8 with saturated NaHCO3 aqueous solution, diluted with water (100 mL), and extracted with dichloromethane (30 mL×3). The organic phases were combined, washed with saturated NaCl aqueous solution, dried over anhydrous sodium sulfate, and filtered. The filtrate was concentrated to dryness, and subjected to a column chromatography (MeOH/DCM=1/50) to obtain 0.99 g of light yellow solids, yield: 79.4%.¹H NMR (400 MHz, CDCl₃) δ 7.62 (d, *J =* 8.2 Hz, 2H), 7.33 (d, *J =* 8.2 Hz, 2H), 2.97 (dd, *J =* 13.9, 2.8 Hz, 1H), 2.38 - 2.22 (m, 2H), 2.04 - 1.95 (m, 2H), 1.91 - 1.79 (m, 2H), 1.78 - 1.71 (m, 1H), 1.46 (ddd, *J* = 25.1, 12.7, 3.9 Hz, 1H), 1.03 (d, *J* = 6.4 Hz, 3H), 0.99 (t, *J* = 7.1 Hz, 3H). MS[M+H]⁺: 300.

### Step b: Preparation of 35-b

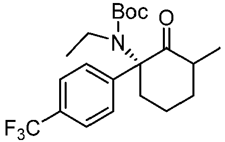

Compound 35-a (990 mg,3.31 mmol) was dissolved in toluene (10 mL), potassium carbonate (1.37 g, 9.90 mmol) and (Boc)2O (1.08 g, 4.95 mmol) were added, and the reaction was stirred at 85 °C for 16 h. TLC (PE/EA=2/1) detected the raw material was reacted completely, filtered, the filtrate was concentrated to dryness, and subjected to a column chromatography (PE/EA=50/1-20/1). to obtain colorless viscous oil 0.66 g, yield: 50.0%. MS[M+Na]⁺: 422.

### Step c: Preparation of 35-c

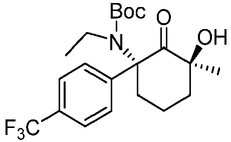

72 mg of colorless oil was obtained according to the method described in step b in Example 1 with compound 35-b (320 mg, 0.80 mmol) as a raw material, yield: 21.6%. MS (M+Na)⁺:438.

### Step d: Preparation of compound 35

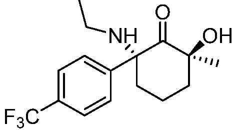

27 mg of white solid was obtained according to the method described in step c in Example 1 with compound 35-c (70 mg, 0.17 mmol) as a raw material, yield: 50.9 %.¹H NMR (400 MHz, CDCl₃) δ 7.59 (d, *J* = 8.1 Hz, 2H), 7.38 (d, *J* = 8.0 Hz, 2H), 2.42 (dq, *J* = 14.4, 7.3 Hz, 1H), 2.25 - 2.15 (m, 1H), 2.06 (m, 3H), 1.95 (m, 2H), 1.86 - 1.77 (m, 1H), 1.56 (s, 3H), 1.09 (t, *J* = 7.1 Hz, 3H). MS[M+H]⁺: 316.

### Example 35: Preparation of (2S,6R)-2-hydroxy-6-isopropylamino-2-methyl-6-(4-(trifluoromethyl)phenyl)cyclohexan-1-one (Compound 36)

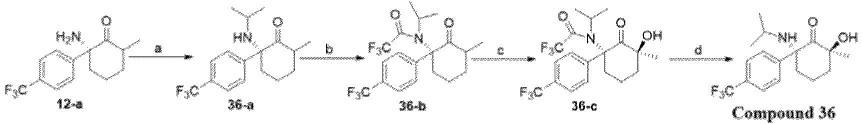

### Step a: Preparation of 36-a

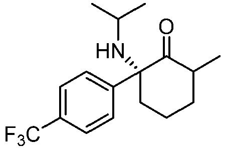

(2R)-2-Amino-6-methyl-2-(4-(trifluoromethyl)phenyl)cyclohexan-1-one (950 mg, 3.50 mmol) was dissolved in acetone (10 mL), and acetic acid (0.042 mL, 0.70 mmol) was added, and the tube was closed and stirred overnight at 56°C, then cooled to room temperature, and sodium borohydride acetate (1.11 g, 5.24 mmol) was added under an ice bath., and continue the reaction at room temperature for 1 h. TLC (PE/EA=3/1) detected the raw material was reacted completely, then the reaction was quenched with saturated aqueous NaHCO₃ (50 mL), and extracted with ethyl acetate (20 mL×3), the organic phases were combined, washed with saturated aqueous NaCl, dried over anhydrous sodium sulfate, filtered, and the filtrate was concentrated to dryness, and subjected to a column chromatography (PE/EA= 10/1) to obtain 800 mg of a colorless oil, yield: 72.9%.¹H NMR (400 MHz, CDCl₃) δ 7.63 (d, *J* = 8.2 Hz, 2H), 7.35 (d, *J* = 8.2 Hz, 2H), 3.01 (dd, *J* = 14.3, 2.5 Hz, 1H), 2.42 (dt, *J* = 12.6, 6.3 Hz, 1H), 2.30 (dq, *J* = 19.1, 6.3 Hz, 1H), 1.94 (m, 2H), 1.80 (m, 2H), 1.45 (ddd, *J* = 25.4, 12.8, 4.9 Hz, 1H), 1.01 (d, *J* = 6.4 Hz, 3H), 0.90 (d, *J* = 6.2 Hz, 3H), 0.75 (d, *J* = 6.4 Hz, 3H). MS [M+H]⁺: 314.25.

### Step b: Preparation of 36-b

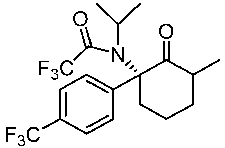

Compound 36-a (740 mg, 2.36 mmol) was dissolved in anhydrous THF (10 mL), TEA (1 mL), DMAP (148 mg, 1.21 mmol) were added, then trifluoroacetic anhydride (990 mg, 4.72 mmol) was added under stirring condition at 0°C, and the reaction was gradually raised to room temperature and stirred for 1 h. TLC (PE/EA=3/1) detected the raw material was reacted completely, then the reaction was quenched with saturated NH₄Cl aqueous solution (60 mL), and extracted with ethyl acetate (30 mL×3), the organic phases were combined, washed with saturated NaCl aqueous solution, dried over anhydrous sodium sulfate, filtered, and the filtrate was concentrated to dryness, and then subjected to a column chromatography (PE/EA= 10/1) to obtain 250 mg of light yellow solid, yield: 25.8%.¹H NMR (400 MHz, CDCl₃) δ 7.83 (d, *J* = 8.1 Hz, 2H), 7.66 (d, *J =* 8.1 Hz, 2H), 3.82 - 3.66 (m, 1H), 2.96 (d, *J =* 13.6 Hz, 1H), 2.67 (td, *J =* 13.9, 4.3 Hz, 1H), 2.00 - 1.74 (m, 5H), 1.11 (d, *J =* 6.6 Hz, 6H), 1.02 (d, *J =* 7.0 Hz, 3H). MS [M+H]⁺: 410.20.

### Step c: Preparation of 36-c

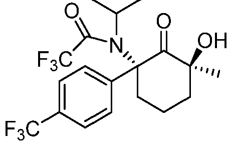

134 mg of light yellow oil was obtained according to the method described in step b of Example 1 with compound 36-b (500 mg, 1.22 mmol) as a raw material, yield: 25.8%.¹H NMR (400 MHz, CDCl₃) δ 7.93 (d, *J =* 8.3 Hz, 2H), 7.65 (d, *J =* 8.3 Hz, 2H), 3.77 - 3.61 (m, 1H), 2.93 (d, *J =* 14.5 Hz, 1H), 2.49 (td, *J* = 13.8, 4.0 Hz, 1H), 2.04 (dd, *J* = 24.1, 8.4 Hz, 2H), 1.88 - 1.77 (m, 2H), 1.45 (s, 3H), 1.15 (d, *J* = 7.0 Hz, 3H), 1.11 (d, *J* = 7.0 Hz, 3H). MS [M+H]⁺: 426.23.

### Step d: Preparation of compound 36

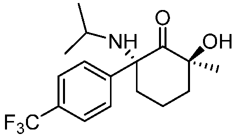

22 mg of white solid was obtained according to the method described in step f of Example 27 with compound 36-c (134 mg, 0.32 mmol) as a raw material, yield: 21.2 %.¹H NMR (400 MHz, CDCl₃) δ 7.59 (d, *J* = 8.1 Hz, 2H), 7.46 (d, *J* = 8.1 Hz, 2H), 2.56 (d, *J* = 15.5 Hz, 1H), 2.47 (dt, *J* = 12.5, 6.1 Hz, 1H), 2.12 - 1.86 (m, 5H), 1.45 (s, 3H), 0.95 (d, *J* = 6.2 Hz, 3H), 0.82 (d, *J* = 6.3 Hz, 3H). MS [M+H]⁺: 330.29.

### Example 36: Preparation of N-((1R,3S)-3-hydroxy-3-methyl-2-oxo-1-(4-(trifluoromethyl)phenyl)cyclohexyl)acetamide (Compound 37)

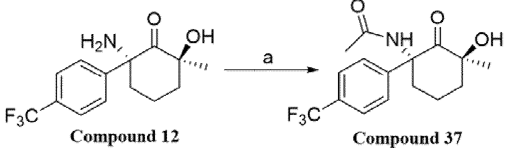

Compound 12 (300 mg, 1.04 mmol) was dissolved in THF (5 mL), triethylamine (210 mg, 2.08 mmol), DMAP (26 mg, 0.21 mmol), and acetic anhydride (155 mg, 1.52 mmol) were added sequentially, and reacted at room temperature for 1 hour. TLC detected the raw material was reacted completely, then the reaction was quenched with saturated aqueous NH₄Cl, and extracted with ethyl acetate (20 mL × 3), the organic phases were combined, washed with saturated aqueous NaCl, dried over anhydrous sodium sulfate, filtered, rotatory evaporated to remove the low-boiling solvent, and then subjected to a column chromatographed (DCM/MeOH=150/1-50/1) to obtain 142 mg of white solid. Yield: 41.3%.¹H NMR (400 MHz, DMSO-*d₆*) δ 8.49 (s, 1H), 7.68 (d, *J* = 8.4 Hz, 2H), 7.53 (d, *J* = 8.3 Hz, 2H), 4.95 (s, 1H), 2.42 (m, 1H), 2.28 - 2.18 (m, 1H), 1.90 (s, 3H), 1.88 - 1.74 (m, 4H), 1.28 (s, 3H). MS[M+H]⁺: 330.

### Example 37: Preparation of N-((1R,3S)-3-hydroxy-3-methyl-2-oxo-1-(4-(trifluoromethyl)phenyl)cyclohexyl)propanamide(Compound 38)

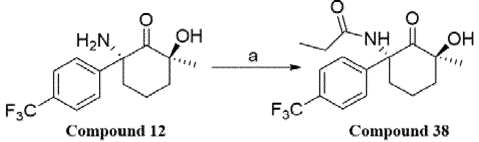

95 mg of white solid was obtained according to the method described in Example 36 with compound 12 (130 mg, 0.45 mmol), propionic anhydride (88 mg, 0.68 mmol as raw materials, yield: 61.3%.1H NMR (400 MHz, DMSO-*d₆*) δ 8.40 (s, 1H), 7.69 (d, J = 8.3 Hz, 2H), 7.54 (d, J = 8.2 Hz, 2H), 4.94 (s, 1H), 2.43 (m, 1H), 2.29 - 2.16 (m, 3H), 1.91 - 1.73 (m, 4H), 1.28 (s, 3H), 0.98 (t, J = 7.6 Hz, 3H). MS (M+H)⁺: 344.

### Example 38: Preparation of (2R,6R)-2-methoxy-2-methyl-6-methylamino-6-(4-(trifluoromethyl)phenyl)cyclohexan-1-one (compound 39)

### Step a: Preparation of 39-a

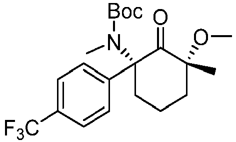

Compound 10-b (150 mg, 0.39 mmol) was dissolved in anhydrous THF (5 mL), CH₃I (220 mg, 1.55 mmol) was added in an ice bath, under the protection of N₂, stirred for 10 min, then NaH (62 mg, 1.55 mmol) was added in an ice bath, and the reaction was carried out at room temperature for about 2 h. TLC (PE/EA=10/1) detected the raw material was reacted completely, then the reaction was quenched with saturated aqueous NH₄Cl (20 mL), extracted with ethyl acetate (10 mL×3), the organic phases were combined, washed with saturated aqueous NaCl, dried over anhydrous sodium sulfate, filtered, and the filtrate was concentrated to dryness, and then subjected to a column chromatography (PE/EA=100/1) to obtain 84 mg of colorless oil, the yield: 52.2%. MS [M+H]⁺: 416.

### Step b: Preparation of compound 39

60 mg of white solid was obtained according to the method described in step c in Example 1 with compound 39-a (84 mg, 0.20 mmol) as a raw material, yield: 93.8 %.¹H NMR (400 MHz, CDCl₃) δ 7.60 (d, *J =* 8.0 Hz, 2H), 7.43 (d, *J =* 8.0 Hz, 2H), 3.31 (s, 3H), 2.51 - 2.38 (m, 1H), 2.14 (m, 2H), 2.02 (s, 3H), 1.97 (m, 1H), 1.76 - 1.65 (m, 2H), 1.04 (s, 3H). MS [M+H]⁺: 316.29.

### Example 39: Preparation of (2S,6R)-2-methoxy-2-methyl-6-methylamino-6-(4-(trifluoromethyl)phenyl)cyclohexan-1-one (compound 40)

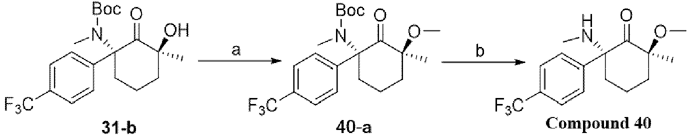

### Step a: Preparation of 40-a

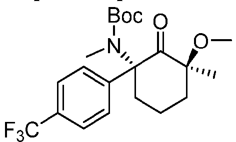

Compound 31-b (100 mg, 0.25 mmol) was dissolved in anhydrous THF (5 mL), NaH (40 mg, 1.0 mmol) was added under the condition of ice-bath cooling under the protection of N₂ and the reaction was continued at 0 °C for 20 min, then CH₃I (227 mg, 1.60 mmol) was slowly dripped into the reaction solution, and the reaction was naturally raised to room temperature for 2 h. TLC (PE/EA=3/1) detected the raw material was reacted completely, then the reaction was quenched with saturated aqueous NH₄Cl (50 mL), extracted with ethyl acetate (20 mL × 3), the organic phases were combined, washed with saturated aqueous NaCl, dried over anhydrous sodium sulfate, filtered, the filtrate was concentrated to dryness, and the subjected to a column chromatography (PE/EA = 10/1) to obtain 45 mg of yellow oil, yield: 43.7 %.¹H NMR (400 MHz, CDCl₃) δ 7.66 (d, *J* = 8.2 Hz, 2H), 7.59 (d, *J* = 8.2 Hz, 2H), 2.68 (d, *J =* 13.8 Hz, 1H), 2.47 (s, 3H), 2.45 (s, 3H), 2.38 (m, 1H), 2.13 (m, 2H), 1.98 (m, 1H), 1.73 (m, 1H), 1.46 (s, 9H), 1.29 (s, 3H). MS [M+H]⁺: 416.

### Step b: Preparation of compound 40

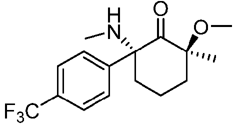

26 mg of white solid was obtained according to the method described in step c of Example 1 with compound 40-a (45 mg, 0.11 mmol) as a raw material, yield: 76.5%.¹H NMR (400 MHz, CDCl₃) δ 7.60 (d, *J* = 8.0 Hz, 2H), 7.45 (d, *J* = 8.0 Hz, 2H), 2.88 (d, *J =* 14.3 Hz, 1H), 2.41 (s, 3H), 2.14 (m, 2H), 1.96 (s, 3H), 1.89 (m, 1H), 1.69 (m, 2H), 1.22 (s, 3H). MS [M+H]⁺: 316.17.

### Example 40: Preparation of (1S,3R)-1-methyl-3-methylamino-2-oxo-3-(4-(trifluoromethyl)phenyl)cyclohexanol acetate (Compound 41)

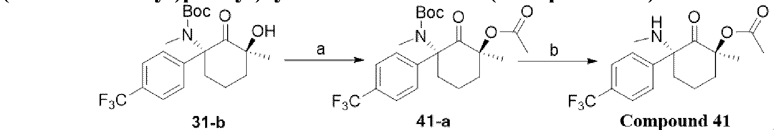

### Step a: Preparation of 41-a

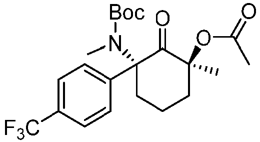

Compound 31-b (50 mg, 0.12 mmol) was dissolved in anhydrous THF (5 mL) and slowly added acetic anhydride (14 mg, 0.14 mmol), DMAP (29 mg, 0.24 mmol) under N₂ protection, and stirred at 40 °C overnight. TLC (PE/EA=5/1) detected the raw material was reacted completely, then the reaction was quenched with saturated NaHCO₃ aqueous solution (20 mL), extracted with ethyl acetate (10 mL×3), the organic phases were combined, washed with saturated NaCl aqueous solution, dried over anhydrous sodium sulfate, filtered, and the filtrate was concentrated to dryness, and the subjected to a column chromatography (PE/EA= 10/1) to obtain 40 mg of a gray solid, yield: 72.7%. MS [M+Na]⁺: 466.27.

### Step b: Preparation of compound 41

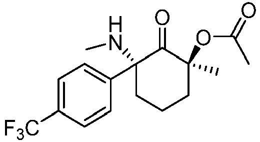

18 mg of white solid was obtained according to the method described in step c of Example 1 with compound 41-a (40 mg, 0.09 mmol) as a raw material, yield: 58.1 %.¹H NMR (400 MHz, CDCl₃) δ 7.59 (d, *J =* 7.9 Hz, 2H), 7.49 (d, *J =* 8.0 Hz, 2H), 2.55 (t, *J* = 11.2 Hz, 1H), 2.40 (t, *J =* 10.9 Hz, 1H), 2.10 (s, 3H), 1.99 - 1.82 (m, 7H), 1.62 (s, 3H). MS [M+H]⁺: 344.24.

### Example 41: Preparation of (1S,3R)-1-methyl-3-methylamino-2-oxo-3-(4-(trifluoromethyl)phenyl)cyclohexanol propionate (compound 42)

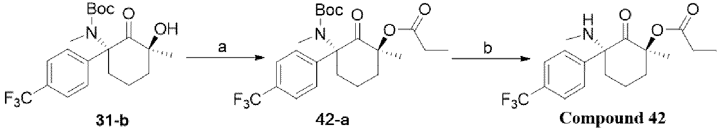

### Step a: Preparation of 42-a

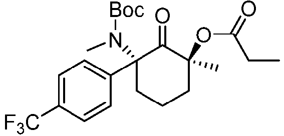

40 mg of off-white solid was obtained according to the method described in step a of Example 40 with compound 31-b (50 mg, 0.12 mmol), propionic anhydride (18 mg, 0.14 mmol) as raw material, yield: 70.2 %. MS [M+Na]⁺: 480.26.

### Step b: Preparation of compound 42

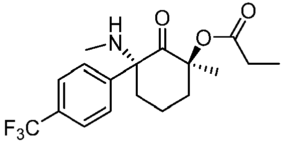

14 mg of white solid was obtained according to the method described in step c of Example 1 with compound 42-a (40 mg, 0.09 mmol) as a raw material, yield: 45.2 %. ¹H NMR (400 MHz, CDCl₃) δ 7.59 (d, *J* = 8.0 Hz, 2H), 7.49 (d, *J* = 8.0Hz, 2H), 2.54 (t, *J =* 11.4 Hz, 1H), 2.43 (t, *J =* 11.1 Hz, 1H), 2.25 - 2.12 (m, 1H), 2.09 (s, 3H), 2.07 - 1.98 (m, 1H), 1.88 (m, 4H), 1.62 (s, 3H), 1.03 (t, *J* = 7.4 Hz, 3H). MS [M+H]⁺: 358.25.

### Example 42: Preparation of 2-amino-2-(2-fluoro-3-(trifluoromethoxy)phenyl)-6-hydroxy-6-methylcyclohexan-1-one hydrochloride (compound 43)

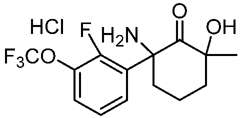

Compound 1 (90 mg, 0.28 mmol) was dissolved in DCM (2 mL), under ice bath, added HCl/dioxane (0.5 mL, 4 M), and stirred at room temperature for 30 min, a solid was precipitated, filtered, and the filter was washed with a mixed solvents of ethyl acetate/petroleum ether = 1 / 1, and dried to obtain a white solid 91 mg, yield: 91.0%. ¹H NMR (400 MHz, CD₃OD) δ 7.84 (t, *J =* 7.4 Hz, 1H), 7.71 (t, *J =* 7.6 Hz, 1H), 7.55 (t, *J* = 8.2 Hz, 1H), 3.17 (d, *J =* 14.2 Hz, 1H), 2.11 - 1.96 (m, 3H), 1.92 - 1.78 (m, 2H), 0.97 (s, 3H). MS[M+H]⁺: 322.

### Example 43: Preparation of 2-amino-6-hydroxy-6-methyl-2-(2,4,6-trifluorotoluene)cyclohexan-1-one hydrochloride (compound 44)

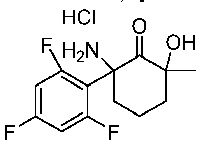

423 mg of white solid was obtained according to the method described in Example 42 with compound 2 (400 mg, 1.46 mmol) as a raw material, yield: 93.4 %. ¹H NMR (400 MHz, CD₃OD) δ 7.20 - 7.11 (m, 2H), 3.31 (m, 1H), 2.08 (m, 1H), 2.03 (m, 1H), 1.96 - 1.80 (m, 3H), 1.14 (s, 3H). MS [M+H]⁺: 274.

### Example 44: Preparation of 2-amino-2-(4-chloro-3-(trifluoromethoxy)phenyl)-6-hydroxy-6-methylcyclohexan-1-one hydrochloride (Compound 45)

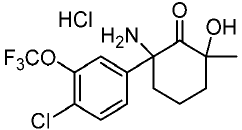

145 mg of white solid was obtained according to the method described in Example 42 with compound 3 (150 mg, 0.44 mmol) as a raw material, yield: 87.3 %. ¹H NMR (400 MHz, CD₃OD) δ 7.96 (t, *J* = 4.7 Hz, 1H), 7.73 (d, *J* = 5.0 Hz, 2H), 3.21 (d, *J =* 15.1 Hz, 1H), 2.04 (m, 3H), 1.95 - 1.79 (m, 2H), 0.98 (s, 3H). MS (M+H)⁺: 338.

### Example 45: Preparation of 2-amino-2-(3-fluoro-2-(trifluoromethyl)phenyl)-6-hydroxy-6-methylcyclohexan-1-one hydrochloride (compound 46)

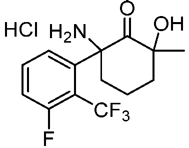

82 mg of white solid was obtained according to the method described in Example 42 with compound 4 (80 mg, 0.26 mmol) as a raw material, yield: 91.1 %. ¹H NMR (400 MHz, CD₃OD) δ 7.81 (dd, *J =* 14.3, 7.8 Hz, 1H), 7.51 (dd, *J* = 23.7, 9.3 Hz, 2H), 2.80 (m, 1H), 2.45 - 2.25 (m, 2H), 2.20 (m, 1H), 1.90 (m, 2H), 1.37 (s, 3H). MS (M+H)⁺: 306.

### Example 46: Preparation of 3-(1-amino-3-hydroxy-3-methyl-2-oxocyclohexyl)benzonitrile hydrochloride (compound 47)

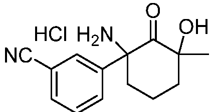

145 mg of white solid was obtained according to the method described in Example 42 with compound 5 (150 mg, 0.61 mmol) as a raw material, yield: 84.3%. ¹H NMR (400 MHz, CD₃OD) δ 7.97 - 7.89 (m, 2H), 7.86 - 7.72 (m, 2H), 3.16 (d, *J* = 14.3 Hz, 1H), 2.14 (t, *J* = 13.1 Hz, 1H), 2.04 (m, 2H), 1.91 (q, *J* = 12.1 Hz, 2H), 0.82 (s, 3H). MS [M+H]⁺: 245.

### Example 47: Preparation of 2-amino-2-(2,3-difluorophenyl)-6-hydroxy-6-methylcyclohexan-1-one hydrochloride (compound 48)

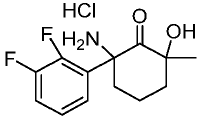

132 mg of white solid was obtained according to the method described in Example 42 with compound 6 (150 mg, 0.59 mmol) as a raw material, yield: 77.2%. ¹H NMR (400 MHz, CD₃OD) δ 7.62 - 7.50 (m, 2H), 7.45 (dd, *J =* 13.6, 8.1 Hz, 1H), 3.16 (d, *J =* 13.6 Hz, 1H), 2.03 (m, 3H), 1.87 (m, 2H), 1.00 (s, 3H). MS[M+H]⁺: 256.

### Example 48: Preparation of 2-amino-6-hydroxy-6-methyl-2-(3-(trifluoromethoxy)phenyl)cyclohexan-1-one hydrochloride (compound 49)

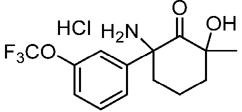

28 mg of white solid was obtained according to the method described in Example 42 with compound 7 (30 mg, 0.099 mmol) as a raw material, yield: 82.4%. ¹H NMR (400 MHz, CD₃OD) δ 7.71 (t, *J* = 8.1 Hz, 1H), 7.57 (d, *J =* 8.0 Hz, 1H), 7.51 (d, *J* = 8.2 Hz, 1H), 7.40 (s, 1H), 3.12 (t, *J* = 13.6 Hz, 1H), 2.19 - 2.00 (m, 3H), 1.95 - 1.86 (m, 2H), 0.81 (s, 3H). MS [M+H]⁺: 304.

### Example 49: Preparation of 2-amino-6-hydroxy-6-methyl-2-(4-(trifluoromethyl)phenyl)cyclohexan-1-one hydrochloride (compound 50)

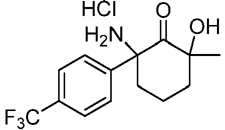

30 mg of white solid was obtained according to the method described in Example 42 with compound 8 (30 mg, 0.10 mmol) as a raw material, yield: 88.2%. ¹H NMR (400 MHz, CD₃OD) δ 7.89 (d, *J* = 8.2 Hz, 2H), 7.74 (d, *J* = 8.2 Hz, 2H), 3.18 (d, *J* = 12.2 Hz, 1H), 2.14 (dd, *J* = 17.8, 7.8 Hz, 1H), 2.04 (m, 2H), 1.92 (m, 2H), 0.81 (s, 3H). MS (M+H)⁺: 288.

### Example 50: Preparation of (2R, 6R)-2-amino-6-hydroxy-6-deutero-2-(4-(trifluoromethyl)phenyl)cyclohexan-1-one hydrochloride (compound 51)

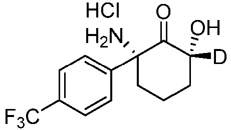

39 mg of white solid was obtained according to the method described in Example 42 with compound 9 (40 mg, 0.14 mmol) as a raw material, yield: 86.7%. ¹H NMR (400 MHz, CD₃OD) δ 7.90 (d, *J* = 8.1 Hz, 2H), 7.67 (d, *J* = 8.0 Hz, 2H), 3.17 (d, *J* = 13.5 Hz, 1H), 2.32 (d, *J* = 10.6 Hz, 1H), 2.09 (dd, *J* = 18.9, 8.5 Hz, 1H), 1.99 (d, *J* = 14.8 Hz, 1H), 1.88 - 1.65 (m, 2H). MS [M+H]⁺: 275.

### Example 51: Preparation of (2R, 6R)-2-amino-6-hydroxy-6-methyl-2-(4-(trifluoromethyl)phenyl)cyclohexan-1-one hydrochloride (compound 52)

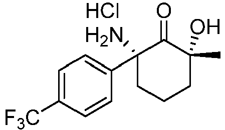

35 mg of white solid was obtained according to the method described in Example 42 with compound 10 (40 mg, 0.14 mmol) as a raw material, yield: 77.8%. ¹H NMR (400 MHz, CD₃OD) δ 7.89 (d, *J =* 8.2 Hz, 2H), 7.74 (d, *J =* 8.2 Hz, 2H), 3.18 (d, *J =* 12.2 Hz, 1H), 2.14 (dd, *J =* 17.8, 7.8 Hz, 1H), 2.04 (d, *J* = 8.4 Hz, 2H), 1.92 (t, *J =* 8.7 Hz, 2H), 0.81 (s, 3H). MS (M+H)⁺: 288.

### Example 52: Preparation of (2S, 6S)-2-amino-6-hydroxy-6-methyl-2-(4-(trifluoromethyl)phenyl)cyclohexan-1-one hydrochloride (compound 53)

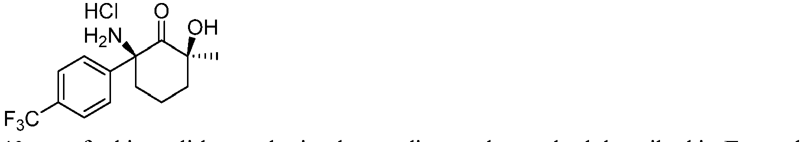

40 mg of white solid was obtained according to the method described in Example 42 with compound 11 (40 mg, 0.14 mmol) as a raw material, yield: 88.9%. ¹H NMR (400 MHz, CD₃OD) δ 7.89 (d, *J =* 8.3 Hz, 2H), 7.74 (d, *J =* 8.3 Hz, 2H), 3.17 (d, *J =* 13.8 Hz, 1H), 2.14 (td, *J =* 13.2, 4.1 Hz, 1H), 2.04 (dd, *J =* 7.0, 4.2 Hz, 2H), 1.93 (m, 2H), 0.81 (s, 3H). MS (M+H)⁺: 288.

### Example 53: Preparation of (2R, 6S)-2-amino-6-hydroxy-6-methyl-2-(4-(trifluoromethyl)phenyl)cyclohexan-1-one hydrochloride (compound 54)

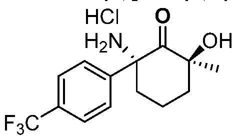

18 mg of white solid was obtained according to the method described in Example 42 with compound 12 (20 mg, 0.70 mmol) as a raw material, yield: 81.8%. ¹H NMR (400 MHz, DMSO-*d₆*) δ 8.73 (s, 3H), 7.82 (d, *J =* 8.2 Hz, 2H), 7.63 (d, *J =* 8.2 Hz, 2H), 5.15 (s, 1H), 3.01 (d, *J =* 12.1 Hz, 1H), 2.07 (dd, *J* = 23.6, 12.2 Hz, 1H), 1.98 (d, *J =* 13.2 Hz, 1H), 1.90 (d, *J* = 14.8 Hz, 1H), 1.72 - 1.53 (m, 2H), 1.21 (s, 3H). MS[M+H]⁺:288.

### Example 54: Preparation of (2S, 6R)-2-amino-6-hydroxy-6-methyl-2-(4-(trifluoromethyl)phenyl)cyclohexan-1-one hydrochloride (compound 55)

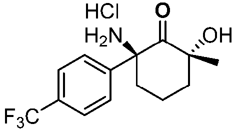

15 mg of white solid was obtained according to the method described in Example 42 with compound 13 (20 mg, 0.70 mmol) as a raw material, yield: 68.2%. ¹H NMR (400 MHz, DMSO-d₆) δ 8.72 (s, 3H), 7.82 (d, *J =* 8.2 Hz, 2H), 7.63 (d, *J =* 8.2 Hz, 2H), 5.15 (s, 1H), 3.01 (d, *J =* 12.0 Hz, 1H), 2.07 (dd, *J* = 23.6, 12.2 Hz, 1H), 1.98 (d,*J* = 13.1 Hz, 1H), 1.90 (d, *J =* 14.6 Hz, 1H), 1.73 - 1.52 (m, 2H), 1.21 (s, 3H). MS[M+H]⁺. 288.

### Example 55: Preparation of 2-(2-fluoro-3-(trifluoromethoxy)phenyl)-6-hydroxy-6-methyl-2-(methylamino)cyclohexan-1-one hydrochloride (compound 56)

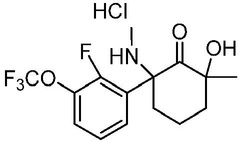

92 mg of white solid was obtained according to the method described in Example 42 with compound 14 (100 mg, 0.30 mmol) as a raw material, yield: 82.9%.¹H NMR (400 MHz, CD₃OD) δ 7.85 (s, 1H), 7.77 (s, 1H), 7.59 (s, 1H), 3.29 - 3.22 (m, 1H), 2.37 (s, 3H), 2.04 (m, 2H), 1.98 - 1.77 (m, 3H), 0.96 (s, 3H). MS [M+H]⁺: 336.

### Example 56: Preparation of (2R,6S)-2-(2-fluoro-3-(trifluoromethoxy)phenyl)-6-hydroxy-6-methyl-2-(methylamino)cyclohexan-1-one hydrochloride (compound 57)

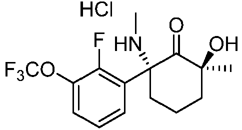

37 mg of white solid was obtained according to the method described in Example 42 with compound 15 (40 mg, 0.12 mmol) as a raw material, yield: 84.1%. ¹H NMR (400 MHz, CD₃OD) δ 7.75 (t, *J =* 7.3 Hz, 1H), 7.60 (t, *J =* 7.6 Hz, 1H), 7.45 (t, *J =* 8.1 Hz, 1H), 3.29 (m, 1H), 2.35 (s, 3H), 2.11 - 1.98 (m, 2H), 1.90 - 1.64 (m, 3H), 1.35 (s, 3H). MS [M+H]⁺: 336.34.

### Example 57: Preparation of 2-hydroxy-2-methyl-6-methylamino-6-(2,4,6-trifluorotoluene)cyclohexan-1-one hydrochloride (Compound 58)

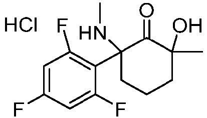

17 mg of white solid was obtained according to the method described in Example 42 with compound 16 (20 mg, 0.070 mmol) as a raw material, yield: 77.3%. ¹H NMR (400 MHz, CD₃OD) δ 7.26 - 7.17 (m, 2H), 3.46 (d, *J =* 11.2 Hz, 1H), 2.53 (s, 3H), 2.07 (m, 2H), 1.79 (m, 3H), 1.12 (s, 3H). MS [M+H]⁺:288.

### Example 58: Preparation of (2S,6R)-2-hydroxy-2-methyl-6-methylamino-6-(2,4,6-trifluorophenyl)cyclohexan-1-one hydrochloride (compound 59)

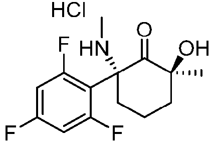

15 mg of white solid was obtained according to the method described in Example 42 with compound 17 (20 mg, 0.070 mmol) as a raw material, yield: 68.2%. ¹H NMR (400 MHz, CD₃OD) δ 7.14 - 7.05 (m, 2H), 3.29 (m, 1H), 2.33 (s, 3H), 2.09 - 1.97 (m, 2H), 1.88 - 1.61 (m, 3H), 1.32 (s, 3H). MS [M+H]⁺: 288. MS [M+H]⁺:288.

### Example 59: Preparation of 2-(4-chloro-3-(trifluoromethoxy)phenyl)-6-hydroxy-6-methyl-2-methylamino-cyclohexan-1-one hydrochloride (compound 60)

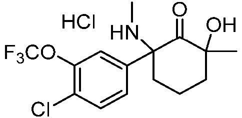

17 mg of white solid was obtained according to the method described in Example 42 with compound 18 (20 mg, 0.057 mmol) as a raw material, yield: 77.3%. ¹H NMR (400 MHz, CD₃OD) δ 7.97 (m, 1H), 7.77 (m, 2H), 3.41 (d, *J =* 12.7 Hz, 1H), 2.33 (s, 3H), 2.09 (d, *J =* 10.4 Hz, 1H), 2.00 (m, 1H), 1.89 (dt, *J* = 21.0, 11.1 Hz, 3H), 0.95 (s, 3H). MS (M+H)⁺: 352.

### Example 60: Preparation of (2R, 6S)-2-(4-chloro-3-(trifluoromethoxy)phenyl) -6-hydroxy-6-methyl-2-methylamino-cyclohexan-1-one hydrochloride (Compound 61)

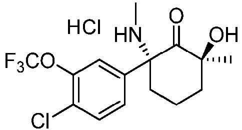

15 mg of white solid was obtained according to the method described in Example 42 with compound 19 (20 mg, 0.057 mmol) as a raw material, yield: 68.2%. ¹H NMR (400 MHz, CD₃OD) δ 7.65 (m, 1H), 7.42 - 7.18 (m, 2H), 3.22 (d, *J =* 13.5 Hz, 1H), 2.25 (s, 3H), 2.14 (m, 1H), 2.05 (m, 1H), 1.93 (m, 1H), 1.82 (m, 1H), 1.70 (m, 1H), 1.35 (s, 3H). MS [M+H]⁺:352.

### Example 61: Preparation of 2-(3-fluoro-2-(trifluoromethyl)phenyl)-6-hydroxy-6-methyl-2-methylamino-cyclohexan-1-one hydrochloride (Compound 62)

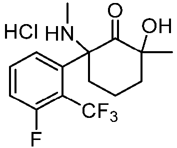

32 mg of white solid was obtained according to the method described in Example 42 with compound 20 (40 mg, 0.12 mmol) as raw material, yield: 72.7%. ¹H NMR (400 MHz, CD₃OD) δ 7.87 (dd, *J =* 14.4, 7.4 Hz, 1H), 7.58 (t, *J =* 8.5 Hz, 2H), 2.53 (s, 4H), 2.27 (d, *J =* 10.7 Hz, 1H), 2.11 (s, 1H), 1.90 (d, *J =* 10.9 Hz, 2H), 1.31 (s, 4H). MS (M+H)⁺: 320.

### Example 62: Preparation of 3-(3-hydroxy-3-methyl-1-methylamino-2-oxocyclohexyl)benzonitrile hydrochloride (Compound 63)

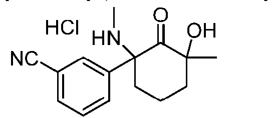

18 mg of white solid was obtained according to the method described in Example 42 with compound 21 (25 mg, 0.097 mmol) as a raw material, yield: 64.3 %. ¹H NMR (400 MHz, CD₃OD) δ 7.98 (m, 1H), 7.91 (m, 1H), 7.80 (m, 2H), 3.26 (d, *J =* 13.9 Hz, 1H), 2.31 (s, 3H), 2.04 (m, 3H), 1.89 (q, *J =* 14.0 Hz, 2H), 0.80 (s, 3H). MS [M+H]⁺. 259.

### Example 63: Preparation of 3-((1R, 3S)-3-hydroxy-3-methyl-1-methylamino-2-oxocyclohexyl)benzonitrile hydrochloride (compound 64)

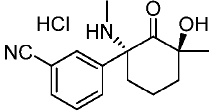

22 mg of white solid was obtained according to the method described in Example 42 with compound 22 (25 mg, 0.097 mmol) as a raw material, yield: 78.6 %. ¹H NMR (400 MHz, CD₃OD) δ 7.87 (d, *J* = 7.6 Hz, 1H), 7.78 (m, 2H), 7.70 (t, *J =* 7.9 Hz, 1H), 3.23 (d, *J =* 13.7 Hz, 1H), 2.27 (s, 3H), 2.13 (dd, *J* = 26.8, 13.6 Hz, 1H), 2.03 (d, *J* = 13.9 Hz, 1H), 1.99 - 1.89 (m, 1H), 1.84 (d, *J =* 14.3 Hz, 1H), 1.78 - 1.68 (m, 1H), 1.33 (s, 3H). MS [M+H]⁺:259.

### Example 64: Preparation of 2-(2,3-difluorophenyl)-6-hydroxy-6-methyl-2-methylaminocyclohexan-1-one hydrochloride (Compound 65)

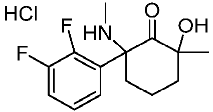

25 mg of white solid was obtained according to the method described in Example 42 with compound 23 (30 mg, 0.11 mmol) as a raw material, yield: 73.5 %. ¹H NMR (400 MHz, CD₃OD) δ 7.59 (t, *J =* 7.8 Hz, 2H), 7.49 (dd, *J =* 13.6, 8.0 Hz, 1H), 3.29 (s, 1H), 2.37 (s, 3H), 2.05 (m, 2H), 1.96 - 1.79 (m, 3H), 0.98 (s, 3H). MS [M+H]⁺: 270.

### Example 65: Preparation of (2R, 6S)-2-(2,3-difluorophenyl)-6-hydroxy-6-methyl-2-methylaminocyclohexan-1-one hydrochloride (Compound 66)

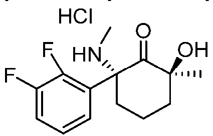

32 mg of white solid was obtained according to the method described in Example 42 with compound 24 (40 mg, 0.15 mmol) as a raw material, yield: 71.1 %. ¹H NMR (400 MHz, CD₃OD) δ 7.53 (s, 1H), 7.45 (d, *J =* 7.4 Hz, 1H), 7.37 (s, 1H), 3.25 (m, 1H), 2.34 (s, 3H), 2.14 - 1.97 (m, 2H), 1.76 (m, 3H), 1.35 (s, 3H). MS [M+H]⁺: 270.36.

### Example 66: Preparation of 2-hydroxy-2-methyl-6-methylamino-6-(3-(trifluoromethoxy)phenyl)cyclohexan-1-one hydrochloride (compound 67)

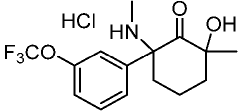

30 mg of white solid was obtained according to the method described in Example 42 with compound 25 (40 mg, 0.13 mmol) as a raw material, yield: 68.2 %. ¹H NMR (400 MHz, CD₃OD) δ 7.74 (t, *J* = 8.1 Hz, 1H), 7.57 (t, *J* = 7.8 Hz, 2H), 7.39 (s, 1H), 3.25 (d, *J =* 11.6 Hz, 1H), 2.31 (s, 3H), 2.04 (dd, *J* = 23.5, 12.6 Hz, 3H), 1.94 - 1.81 (m, 2H), 0.79 (s, 3H). MS[M+H]⁺: 318.

### Example 67: Preparation of (2S,6R)-2-hydroxy-2-methyl-6-methylamino-6-(3-(trifluoromethoxy)phenyl)cyclohexan-1-one hydrochloride (Compound 68)

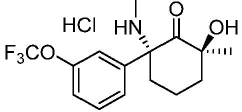

34 mg of white solid was obtained according to the method described in Example 42 with compound 26 (40 mg, 0.13 mmol) as a raw material, yield: 77.3%. ¹H NMR (400 MHz, CD₃OD) δ 7.62 (t, *J =* 8.1 Hz, 1H), 7.43 (d, *J =* 8.2 Hz, 2H), 7.39 (s, 1H), 3.20 (d, *J =* 13.8 Hz, 1H), 2.27 (s, 3H), 2.23 - 2.10 (m, 1H), 2.03 (d, *J* = 13.5 Hz, 1H), 1.93 (td, *J =* 13.6, 3.5 Hz, 1H), 1.83 (d, *J =* 13.9 Hz, 1H), 1.73 (td, *J =* 13.7, 3.7 Hz, 1H), 1.32 (s, 3H). MS [M+H]⁺: 318.34.

### Example 68: (2R,6S)-2-(2-chlorophenyl)-6-hydroxy-6-methyl-2-methylaminocyclohexan-1-one hydrochloride (Compound 69)

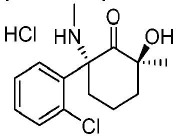

19 mg of white solid was obtained according to the method described in Example 42 with compound 27 (25 mg, 0.093 mmol) as a raw material, yield: 67.8 %. ¹H NMR (400 MHz, CD₃OD) δ 7.81 (d, *J =* 7.1 Hz, 1H), 7.55 - 7.47 (m, 3H), 3.36 (m, 1H), 2.27 (s, 3H), 2.21 - 2.06 (m, 1H), 2.03 (d, *J* = 13.0 Hz, 1H), 1.83 - 1.63 (m, 3H), 1.37 (s, 3H). MS[M+H]⁺: 268.

### Example 69: (2R,6R)-2-(2-chlorophenyl)-6-hydroxy-6-methyl-2-methylaminocyclohexan-1-one hydrochloride (Compound 70)

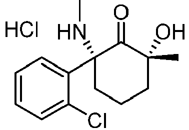

22 mg of white solid was obtained according to the method described in Example 42 with compound 28 (25 mg, 0.093 mmol) as a raw material, yield: 78.6 %. ¹H NMR (400 MHz, CD₃OD) δ 7.95 - 7.88 (m, 1H), 7.67 - 7.58 (m, 3H), 3.40 (d, *J =* 11.0 Hz, 1H), 2.30 (s, 3H), 2.13 - 2.05 (m, 1H), 2.03 - 1.93 (m, 1H), 1.83 (m, 3H), 0.97 (s, 3H). MS[M+H]⁺: 268.

### Example 70: Preparation of (2R,6R)-2-hydroxy-2-methyl-6-methylamino-6-(4-(trifluoromethyl)phenyl)cyclohexan-1-one hydrochloride (Compound 71)

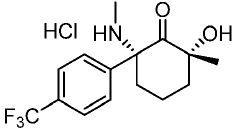

18 mg of white solid was obtained according to the method described in Example 42 with compound 29 (20 mg, 0.066 mmol) as a raw material, yield: 81.8 %. ¹H NMR (400 MHz, CD₃OD) δ 7.93 (d, *J =* 8.1 Hz, 2H), 7.73 (d, *J =* 8.1 Hz, 2H), 3.26 (m, 1H), 2.30 (s, 3H), 2.12 - 1.98 (m, 3H), 1.92 (m, 2H), 0.79 (s, 3H). MS (M+H)⁺: 302.

### Example 71: Preparation of (2R,6R)-2-dimethylamino-6-hydroxy-6-methyl-2-(4-(trifluoromethyl)phenyl)cyclohexan-1-one hydrochloride (Compound 72)

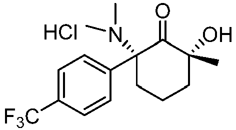

38 mg of white solid was obtained according to the method described in Example 42 with compound 30 (40 mg, 0.13 mmol) as a raw material, yield: 84.4 %. ¹H NMR (400 MHz, CD₃OD) δ 7.95 (d, *J* = 8.4 Hz, 2H), 7.79 (d, *J* = 8.4 Hz, 2H), 3.11 (d, *J =* 14.1 Hz, 1H), 2.93 (s, 3H), 2.44 (m, 1H), 2.36 (s, 3H), 2.15 (d, *J =* 13.5 Hz, 1H), 1.94 (m, 3H), 0.76 (s, 3H). MS (M+H)⁺: 316.

### Example 72: Preparation of (2S,6R)-2-hydroxy-2-methyl-6-methylamino-6-(4-(trifluoromethyl)phenyl)cyclohexan-1-one hydrochloride (Compound 73)

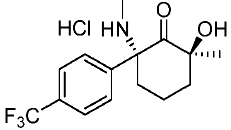

45 mg of white solid was obtained according to the method described in Example 42 with compound 31 (50 mg, 0.16 mmol) as a raw material, yield: 80.4 %. ¹H NMR (400 MHz, CD₃OD) δ 7.82 (d, *J* = 8.2 Hz, 2H), 7.66 (d, *J* = 8.2 Hz, 2H), 3.24 (dd, *J =* 13.8, 2.4 Hz, 1H), 2.26 (s, 3H), 2.24 - 2.11 (m, 1H), 2.04 (dd, *J =* 14.0, 2.2 Hz, 1H), 1.95 (td, *J =* 13.6, 3.7 Hz, 1H), 1.84 (d, *J* = 13.8 Hz, 1H), 1.74 (td, *J* = 13.8, 3.8 Hz, 1H), 1.32 (s, 3H). MS (M+H)⁺: 302.

### Example 73: Preparation of (2S,6S)-2-dimethylamino-6-hydroxy-6-methyl-2-(4-(trifluoromethyl)phenyl)cyclohexan-1-one hydrochloride (compound 74)

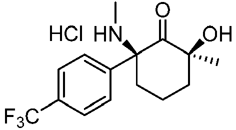

50 mg of white solid was obtained according to the method described in Example 42 with compound 32 (50 mg, 0.16 mmol) as a raw material, yield: 89.3 %. ¹H NMR (400 MHz, CD₃OD) δ 7.93 (d, *J =* 8.2 Hz, 2H), 7.73 (d, *J =* 8.2 Hz, 2H), 3.26 (s, 1H), 2.30 (s, 3H), 2.12 - 1.98 (m, 3H), 1.92 (m, 2H), 0.78 (s, 3H). MS (M+H)⁺: 302.

### Example 74: Preparation of (2R,6S)-2-hydroxy-2-methyl-6-methylamino-6-(4-(trifluoromethyl)phenyl)cyclohexan-1-one hydrochloride (compound 75)

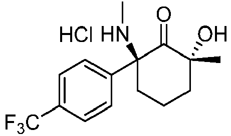

47 mg of white solid was obtained according to the method described in Example 42 with compound 33 (50 mg, 0.16 mmol) as a raw material, yield: 83.9 %. ¹H NMR (400 MHz, CD₃OD) δ 7.82 (d, *J =* 8.3 Hz, 2H), 7.66 (d, *J =* 8.3 Hz, 2H), 3.24 (dd, *J =* 13.7, 2.8 Hz, 1H), 2.26 (s, 3H), 2.24 - 2.12 (m, 1H), 2.04 (dd, *J =* 14.7, 2.8 Hz, 1H), 1.94 (td, *J =* 13.6, 3.9 Hz, 1H), 1.88 - 1.80 (m, 1H), 1.74 (td, *J* = 13.7, 4.0 Hz, 1H), 1.32 (s, 3H). MS (M+H)+: 302.

### Example 75: Preparation of (2S,6R)-2-hydroxy-2-methyl-6-trideutero-methylamino-6-(4-(trifluoromethyl)phenyl)cyclohexan-1-one hydrochloride (Compound 76)

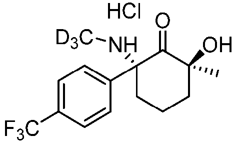

50 mg of white solid was obtained according to the method described in Example 42 with compound 34 (50 mg, 0.16 mmol) as a raw material, yield: 89.3 %. ¹H NMR (400 MHz, CD₃OD) δ 7.82 (d, *J* = 8.3 Hz, 2H), 7.66 (d, *J* = 8.2 Hz, 2H), 3.24 (dd, *J =* 13.8, 2.9 Hz, 1H), 2.25 - 2.11 (m, 1H), 2.08 - 2.00 (m, 1H), 1.94 (td, *J =* 13.6, 3.9 Hz, 1H), 1.88 - 1.80 (m, 1H), 1.74 (td, *J =* 13.7, 4.0 Hz, 1H), 1.32 (s, 3H). MS (M+H)⁺: 305.

### Example 76: Preparation of (2R,6S)-2-ethylamino-6-hydroxy-6-methyl-2-(4-(trifluoromethyl)phenyl)cyclohexan-1-one hydrochloride (compound 77)

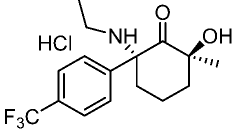

15 mg of white solid was obtained according to the method described in Example 42 with compound 35 (20 mg, 0.063 mmol) as a raw material, yield: 68.2 %. ¹H NMR (400 MHz, CD₃OD) δ 7.81 (d, *J =* 8.2 Hz, 2H), 7.68 (d, *J =* 8.2 Hz, 2H), 3.27 (dd, *J =* 13.9, 2.5 Hz, 1H), 2.88 (dq, *J =* 14.4, 7.3 Hz, 1H), 2.29 (dq, *J =* 14.5, 7.2 Hz, 1H), 2.16 (qt, *J =* 12.6, 2.8 Hz, 1H), 2.00 (qd, *J =* 13.8, 3.3 Hz, 2H), 1.87 - 1.80 (m, 1H), 1.73 (td, *J =* 13.6, 3.9 Hz, 1H), 1.33 (s, 3H), 1.21 (t, *J =* 7.2 Hz, 3H). MS[M+H]⁺: 316.

### Example 77: Preparation of (2S,6R)-2-hydroxy-6-isopropylamino-2-methyl-6-(4-(trifluoromethyl)phenyl)cyclohexan-1-one hydrochloride (Compound 78)

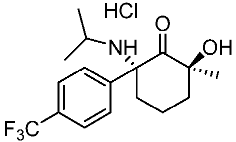

18 mg of white solid was obtained according to the method described in Example 42 with compound 36 (20 mg, 0.061 mmol) as a raw material, yield: 81.8 %. ¹H NMR (400 MHz, CD₃OD) δ 7.81 (d, *J =* 8.3 Hz, 2H), 7.75 (d, *J =* 8.3 Hz, 2H), 3.40 (dd, *J =* 13.0, 6.5 Hz, 1H), 2.27 - 1.96 (m, 4H), 1.84 (d, *J =* 13.3 Hz, 1H), 1.75 (td, *J =* 13.5, 3.6 Hz, 1H), 1.32 (s, 3H), 1.22 (d, *J =* 6.5 Hz, 3H), 0.76 (d, *J =* 6.4 Hz, 3H). MS [M+H]⁺: 330.29.

### Example 78: Preparation of (2R,6R)-2-methoxy-2-methyl-6-methylamino-6-(4-(trifluoromethyl)phenyl)cyclohexan-1-one hydrochloride (compound 79)

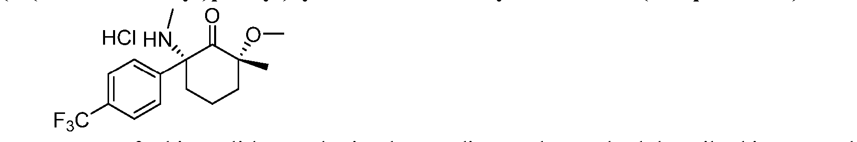

26 mg of white solid was obtained according to the method described in Example 42 with compound 39 (30 mg, 0.095 mmol) as a raw material, yield: 78.8 %. ¹H NMR (400 MHz, CD₃OD) δ 7.93 (d, *J =* 8.2 Hz, 2H), 7.74 (d, *J =* 8.2 Hz, 2H), 3.38 (s, 3H), 3.24 (dd, *J =* 13.7, 2.7 Hz, 1H), 2.30 (s, 3H), 2.17 - 2.01 (m, 3H), 1.96 - 1.83 (m, 2H), 0.85 (s, 3H). MS [M+H]⁺: 316.29.

### Example 79: Preparation of (2S,6R)-2-methoxy-2-methyl-6-methylamino-6-(4-(trifluoromethyl)phenyl)cyclohexan-1-one hydrochloride (Compound 80)

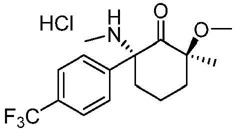

25 mg of white solid was obtained according to the method described in Example 42 with compound 40 (30 mg, 0.095 mmol) as a raw material, yield: 75.8 %. ¹H NMR (400 MHz, CD₃OD) δ 7.87 (d, *J =* 8.6 Hz, 2H), 7.70 (d, *J =* 8.3 Hz, 2H), 3.22 (dd, *J =* 14.0, 2.8 Hz, 1H), 2.49 (s, 3H), 2.27 (s, 3H), 2.26 - 2.15 (m, 2H), 2.05 - 1.94 (m, 1H), 1.86 (dt, *J =* 7.0, 3.0 Hz, 1H), 1.74 (td, *J =* 13.9, 3.8 Hz, 1H), 1.29 (s, 3H). MS [M+H]⁺. 316.17.

### Example 80: Preparation of (1S,3R)-1-methyl-3-methylamino-2-oxo-3-(4-(trifluoromethyl)phenyl)cyclohexanol acetate (Compound 81)

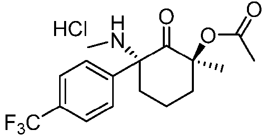

14 mg of white solid was obtained according to the method described in Example 42 with compound 41 (20 mg, 0.058 mmol) as a raw material, yield: 63.6 %. ¹H NMR (400 MHz, CD₃OD) δ 7.89 (d, *J =* 8.3 Hz, 2H), 7.69 (d, *J =* 8.3 Hz, 2H), 3.26 (m, 1H), 2.47 (dd, *J* = 14.3, 2.4 Hz, 1H), 2.31 (d, *J* = 13.1 Hz, 1H), 2.23 (s, 3H), 2.01 (m, 2H), 1.83 (m, 1H), 1.56 (s, 3H), 1.13 (s, 3H). MS [M+H]⁺: 344.24.

### Example 81: Preparation of (1S,3R)-1-methyl-3-methylamino-2-oxo-3-(4-(trifluoromethyl)phenyl)cyclohexanol propionate (Compound 82)

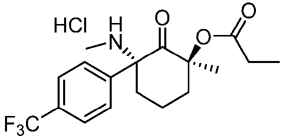

16 mg of white solid was obtained according to the method described in Example 42 with compound 42 (20 mg, 0.056 mmol) as a raw material, yield: 72.7 %. ¹H NMR (400 MHz, CD₃OD) δ 7.87 (d, *J =* 8.3 Hz, 2H), 7.68 (d, *J =* 8.3 Hz, 2H), 3.28 (m, 1H), 2.45 (d, *J =* 14.6 Hz, 1H), 2.30 (m, 1H), 2.21 (s, 3H), 2.07 - 1.94 (m, 2H), 1.85 (m, 1H), 1.72 - 1.61 (m, 1H), 1.56 (s, 3H), 1.10 - 0.99 (m, 1H), 0.61 (t, *J =* 7.4 Hz, 3H). MS [M+H]⁺: 358.25.

### Example 82: Preparation of (2S,6R)-2-hydroxy-2-methyl-6-methylamino-6-(4-(trifluoromethyl)phenyl)cyclohexan-1-one hydrobromide (Compound 83)

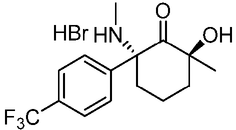

55 mg of white solid was obtained according to the method described in Example 42 with compound 31 (50 mg, 0.16 mmol) as raw material, yield: 87.3 %. ¹H NMR (400 MHz, CD₃OD) δ 7.82 (d, *J =* 8.2 Hz, 2H), 7.66 (d, *J =* 8.3 Hz, 2H), 3.25 (dd, *J =* 13.7, 2.4 Hz, 1H), 2.26 (s, 3H), 2.17 (t, *J =* 13.5 Hz, 1H), 2.03 (d, *J =* 14.2 Hz, 1H), 1.95 (dt, *J =* 17.3, 8.5 Hz, 1H), 1.84 (d, *J =* 14.1 Hz, 1H), 1.74 (td, *J =* 13.6, 3.8 Hz, 1H), 1.32 (s, 3H). MS (M+H)⁺: 302.

### Example 83: Preparation of (2S,6R)-2-hydroxy-2-methyl-6-methylamino-6-(4-(trifluoromethyl)phenyl)cyclohexan-1-one sulfate (Compound 84)

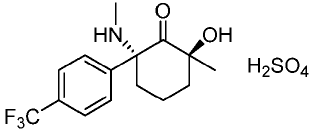

50 mg of white solid was obtained according to the method described in Example 42 with compound 31 (50 mg, 0.16 mmol) as raw material, yield: 75.8 %. ¹H NMR (400 MHz, CD₃OD) δ 7.82 (d, *J* = 8.2 Hz, 2H), 7.66 (d, *J* = 8.3 Hz, 2H), 3.24 (dd, *J =* 13.8, 2.5 Hz, 1H), 2.26 (s, 3H), 2.18 (q, *J =* 13.2 Hz, 1H), 2.03 (dd, *J* = 14.2, 2.5 Hz, 1H), 1.93 (td, *J* = 13.5, 3.8 Hz, 1H), 1.84 (d, *J* = 14.0 Hz, 1H), 1.73 (td, *J* = 13.8, 4.0 Hz, 1H), 1.32 (s, 3H). MS (M+H)⁺: 302.

### Example 84: Preparation of (2S,6R)-2-hydroxy-2-methyl-6-methylamino-6-(4-(trifluoromethyl)phenyl)cyclohexan-1-one phosphate (Compound 85)

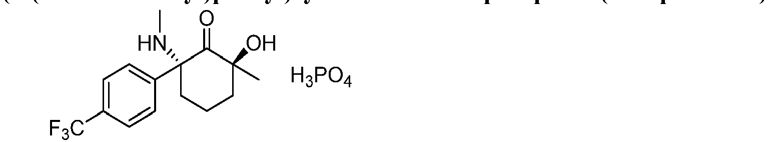

53 mg of white solid was obtained according to the method described in Example 42 with compound 31 (50 mg, 0.16 mmol) as a raw material, yield: 80.3 %. ¹H NMR (400 MHz, CD₃OD) δ 7.82 (d, *J =* 8.2 Hz, 2H), 7.66 (d, *J =* 8.2 Hz, 2H), 3.23 - 3.17 (m, 1H), 2.24 (s, 3H), 2.16 (t, *J* = 13.3 Hz, 1H), 2.09 - 1.93 (m, 2H), 1.90 - 1.72 (m, 2H), 1.34 (s, 3H). MS (M+H)⁺: 302.

### Example 85: Preparation of (2S,6R)-2-hydroxy-2-methyl-6-methylamino-6-(4-(trifluoromethyl)phenyl)cyclohexan-1-one acetate (Compound 86)

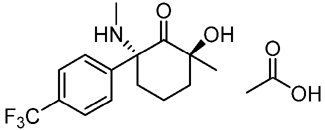

53 mg of white solid was obtained according to the method described in Example 42 with compound 31 (50 mg, 0.16 mmol) as a raw material, yield: 80.3 %. ¹H NMR (400 MHz, CD₃OD) δ 7.71 (d, *J* = 8.2 Hz, 2H), 7.56 (d, *J* = 8.2 Hz, 2H), 2.92 (d, *J =* 14.6 Hz, 1H), 2.08 (s, 4H), 1.97 (d, *J =* 16.9 Hz, 4H), 1.92 - 1.72 (m, 3H), 1.34 (s, 3H). MS (M+H)⁺: 302.

### Example 86: Preparation of (2S,6R)-2-hydroxy-2-methyl-6-methylamino-6-(4-(trifluoromethyl)phenyl)cyclohexan-1-one fumarate (Compound 87)

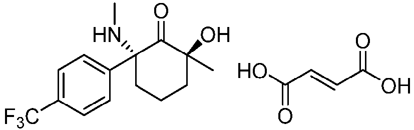

53 mg of white solid was obtained according to the method described in Example 42 with compound 31 (50 mg, 0.16 mmol) as a raw material, yield: 80.3 %. ¹H NMR (400 MHz, CD₃OD) δ 7.79 (d, *J =* 8.3 Hz, 2H), 7.63 (d, *J =* 8.3 Hz, 2H), 6.69 (s, 2H), 3.16 (dd, *J =* 13.9, 2.6 Hz, 1H), 2.21 (s, 3H), 2.20 - 2.09 (m, 1H), 2.02 (dd, *J =* 14.1, 2.7 Hz, 1H), 1.93 (td, *J =* 13.4, 3.7 Hz, 1H), 1.87 - 1.79 (m, 1H), 1.75 (td, *J =* 13.6, 3.9 Hz, 1H), 1.33 (s, 3H). MS (M+H)⁺: 302.

### Example 87: Preparation of (2S,6R)-2-hydroxy-2-methyl-6-methylamino-6-(4-(trifluoromethyl)phenyl)cyclohexan-1-one citrate (Compound 88)

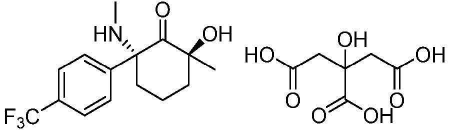

53 mg of white solid was obtained according to the method described in Example 42 with compound 31 (50 mg, 0.16 mmol) as a raw material, yield: 80.3 %. ¹H NMR (400 MHz, CD₃OD) δ 7.80 (d, *J =* 8.1 Hz, 2H), 7.64 (d, *J =* 8.2 Hz, 2H), 3.17 (d, *J =* 12.5 Hz, 1H), 2.78 (q, *J =* 15.4 Hz, 4H), 2.21 (s, 3H), 2.15 (dd, *J =* 25.6, 12.1 Hz, 1H), 2.06 - 1.96 (m, 2H), 1.97 - 1.69 (m, 2H), 1.33 (s, 3H). MS (M+H)⁺: 302.

### Example 88: Preparation of (2S,6R)-2-hydroxy-2-methyl-6-methylamino-6-(4-(trifluoromethyl)phenyl)cyclohexan-1-one maleate (Compound 89)

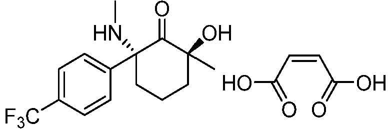

53 mg of white solid was obtained according to the method described in Example 42 with compound 31 (50 mg, 0.16 mmol) as a raw material, yield: 80.3 %. ¹H NMR (400 MHz, CD₃OD) δ 7.82 (d, *J =* 8.2 Hz, 2H), 7.65 (d, *J =* 8.1 Hz, 2H), 6.26 (s, 2H), 3.23 (dd, *J =* 14.0, 2.5 Hz, 1H), 2.26 (s, 3H), 2.16 (m, 1H), 2.03 (dd, *J =* 14.5, 2.5 Hz, 1H), 1.93 (td, *J =* 13.5, 3.8 Hz, 1H), 1.87 - 1.80 (m, 1H), 1.73 (td, *J =* 13.7, 3.8 Hz, 1H), 1.32 (s, 3H). MS (M+H)⁺: 302.

### Example 89: Preparation of (2S,6R)-2-hydroxy-2-methyl-6-methylamino-6-(4-(trifluoromethyl)phenyl)cyclohexan-1-one methanesulfonate (Compound 90)

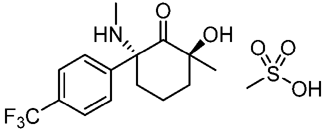

53 mg of white solid was obtained according to the method described in Example 42 with compound 31 (50 mg, 0.16 mmol) as a raw material, yield: 80.3 %. ¹H NMR (400 MHz, CD₃OD) δ 7.82 (d, *J* = 8.2 Hz, 2H), 7.66 (d, *J* = 8.3 Hz, 2H), 3.24 (dd, *J =* 13.6, 2.8 Hz, 1H), 2.70 (s, 3H), 2.26 (s, 3H), 2.23 - 2.11 (m, 1H), 2.03 (dd, *J =* 14.2, 2.4 Hz, 1H), 1.93 (td, *J =* 13.8, 3.5 Hz, 1H), 1.88 - 1.80 (m, 1H), 1.73 (td, *J =* 13.7, 3.9 Hz, 1H), 1.32 (s, 3H). MS (M+H)⁺: 302.

### Example 90: Preparation of (2S,6R)-2-hydroxy-2-methyl-6-methylamino-6-(4-(trifluoromethyl)phenyl)cyclohexan-1-one L-(+)-tartrate (Compound 91)

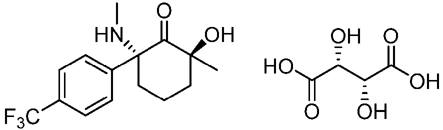

53 mg of white solid was obtained according to the method described in Example 42 with compound 31 (50 mg, 0.16 mmol) as a raw material, yield: 80.3 %. ¹H NMR (400 MHz, CD₃OD) δ 7.80 (d, *J =* 8.2 Hz, 2H), 7.64 (d, *J =* 8.2 Hz, 2H), 4.40 (s, 2H), 3.17 (dd, *J =* 14.0, 2.4 Hz, 1H), 2.23 (s, 3H), 2.15 (dd, *J =* 26.7, 13.4 Hz, 1H), 1.98 (m, 2H), 1.77 (m, 2H), 1.33 (s, 3H). MS (M+H)⁺: 302.

### Example 91: Preparation of (2S,6R)-2-hydroxy-2-methyl-6-methylamino-6-(4-(trifluoromethyl)phenyl)cyclohexan-1-one benzoate (Compound 92)

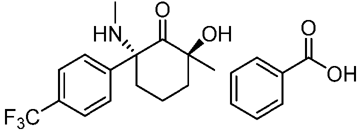

53 mg of white solid was obtained according to the method described in Example 42 with compound 31 (50 mg, 0.16 mmol) as a raw material, yield: 80.3 %. ¹H NMR (400 MHz, CD₃OD) δ 7.98 (d, *J* = 8.1 Hz, 2H), 7.72 (d, *J* = 8.2 Hz, 2H), 7.58 (d, *J* = 8.3 Hz, 2H), 7.49 (t, *J =* 7.3 Hz, 1H), 7.40 (t, *J =* 7.6 Hz, 2H), 2.97 (d, *J =* 13.4 Hz, 1H), 2.14 - 2.03 (m, 4H), 1.99 (m, 1H), 1.91 (dd, *J =* 14.2, 2.2 Hz, 1H), 1.87 - 1.71 (m, 2H), 1.34 (s, 3H). MS (M+H)⁺: 302.

### Example 92: Preparation of (2S,6R)-2-hydroxy-2-methyl-6-methylamino-6-(4-(trifluoromethyl)phenyl)cyclohexan-1-one L-lactate (Compound 93)

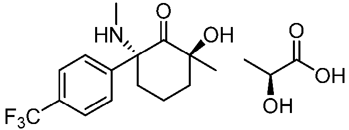

53 mg of white solid was obtained according to the method described in Example 42 with compound 31 (50 mg, 0.16 mmol) as a raw material, yield: 80.3 %. ¹H NMR (400 MHz, CD₃OD) δ 7.76 (d, *J* = 8.2 Hz, 2H), 7.61 (d, *J* = 8.2 Hz, 2H), 4.06 (q, *J* = 6.8 Hz, 1H), 3.06 (d, *J =* 13.9 Hz, 1H), 2.16 (s, 3H), 2.14 - 2.06 (m, 1H), 2.00 (dd, *J =* 14.0, 2.3 Hz, 1H), 1.93 (dd, *J =* 13.4, 3.2 Hz, 1H), 1.89 - 1.80 (m, 1H), 1.80 - 1.71 (m, 1H), 1.34 (d, *J =* 5.2 Hz, 3H), 1.33 (s, 3H). MS (M+H)⁺: 302.

### Example 93: Preparation of (2S,6R)-2-hydroxy-2-methyl-6-methylamino-6-(4-(trifluoromethyl)phenyl)cyclohexan-1-one benzenesulfonate (Compound 94)

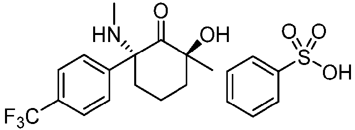

53 mg of white solid was obtained according to the method described in Example 42 with compound 31 (50 mg, 0.16 mmol) as a raw material, yield: 80.3 %. ¹H NMR (400 MHz, CD₃OD) δ 7.82 (t, *J =* 6.7 Hz, 4H), 7.65 (d, *J =* 8.2 Hz, 2H), 7.43 (d, *J =* 5.4 Hz, 3H), 3.24 (dd, *J =* 13.8, 2.4 Hz, 1H), 2.26 (s, 3H), 2.17 (q, *J =* 13.5 Hz, 1H), 2.03 (dd, *J =* 14.4, 2.6 Hz, 1H), 1.93 (td, *J =* 13.6, 3.4 Hz, 1H), 1.88 - 1.80 (m, 1H), 1.73 (td, *J =* 13.6, 3.8 Hz, 1H), 1.32 (s, 3H). MS (M+H)⁺: 302.

### Example 94: Preparation of (2S,6R)-2-hydroxy-2-methyl-6-methylamino-6-(4-(trifluoromethyl)phenyl)cyclohexan-1-one oxalate (Compound 95)

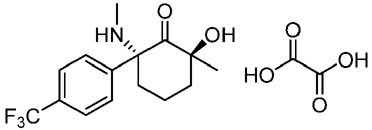

53 mg of white solid was obtained according to the method described in Example 42 with compound 31 (50 mg, 0.16 mmol) as a raw material, yield: 80.3 %. ¹H NMR (400 MHz, CD₃OD) δ 7.81 (d, *J =* 8.2 Hz, 2H), 7.65 (d, *J =* 8.3 Hz, 2H), 3.22 (dd, *J =* 13.8, 2.4 Hz, 1H), 2.25 (s, 3H), 2.18 (dd, *J =* 29.8, 16.4 Hz, 1H), 2.07 - 2.00 (m, 1H), 2.00 - 1.90 (m, 1H), 1.84 (d, *J =* 13.4 Hz, 1H), 1.74 (td, *J =* 13.9, 4.1 Hz, 1H), 1.32 (s, 3H). MS (M+H)⁺: 302.

### Evaluation of biological and chemical properties

The following further description explains the effects of the present invention in conjunction with test examples, but these examples are not meant to limit the scope of the invention.

### Test Example 1. Evaluation of antidepressant activity in a forced swim test of oral administration of the compound of the present invention in mice

### 2.1 Purpose of the experiment

To investigate the effects of different compounds on depression-like behavior in C57 mice by single oral administration of 20 mg/kg for 1h and 24h followed by forced swimming experiment, respectively.

### 2.2. Experimental protocol

### 1) Animals

The experimental animals were 6-week-old C57 mice, male, C57 breed mice purchased from Shanghai Slaughter Laboratory Animal Co. Ltd, weighing 20.45 ± 0.19 g. The animals arrived at the Animal Breeding Center of the Shanghai Institute of Pharmaceutical Sciences, Chinese Academy of Sciences (Animal Production License: SCXK9[Shanghai]2004-0002, Use License: SYXK [Shanghai]2003-0029) before the experiments and were acclimated to the animal facilities for more than 3 days, 6 animals/cage were feed. The feeding environment was room temperature 23 ± 0.2 °C and 12 /12 h day/night alternating light and dark. Prior to behavioral testing, animals were moved to the behavioral testing room 2 hours in advance to acclimatize them to the environment and reduce their nervousness.

### 2) Animal grouping

Animal grouping information and related drug administration information are shown in Table 1.

**Table 1.Animal grouping and drug administration information**

| Groups | Number (animal) | Mode of administration | Dose(mg/kg) | Delivery volume(ml/kg) |
|---|---|---|---|---|
| Blank control | 10 | Gavage | 0.9%NaCl | 10 |
| Fluoxetine | 10 | Gavage | 20 | 10 |
| S-ketamine | 10 | Abdominal injection | 10 | 10 |
| (2R,6R)-HNK | 10 | Gavage | 20 | 10 |
| Comparative compounds 1, 3-6 | 10 | Gavage | 20 | 10 |
| compound of the invention | 10 | Gavage | 20 | 10 |

### 3) Experimental steps

Preparation of the test material: the test compounds were weighed and dissolved in 0.9% NaCl solution, mixed well, and prepared into 1mg/ml and 2mg/ml solution for use, respectively.
24 h prior to drug administration, mice were placed in a cylindrical tank to acclimatize to the water environment for 10 min. On the day of the behavioral test, the animals were administered the drug once, by gavage/intraperitoneal injection at 1h and 24h prior to the behavioral test, respectively (as shown in Table 1). The mice were placed individually into a cylindrical glass tank of 30 cm height and 20 cm diameter, with a water depth of 15 cm, so that the animals could hardly escape from the tank and their feet and tails did not touch the bottom of the tank, and the temperature of the water was 23°C-25°C. A 6-minute video was taken of the mice after they entered the water, and since most of the animals were very active in the first two minutes, the immobility time was counted for the next 4 minutes (criteria for immobility: the mice stopped struggling in the water, did not move, and made small limb movements to maintain balance or float).

### 4) Test results

The antidepressant effect of the compounds was evaluated by their ability to cause a reduction in the immobility time of the animals in the forced swim test compared to the blank group. Inhibition rate 1h (%) = [immobility time (blank, 1h) - immobility time (administration, 1h)]/ immobility time (blank, 1h) × 100% Inhibition rate 24h (%) = [immobility time (blank, 24h) - immobility time (administration, 24h)]/ immobility time (blank, 24h) × 100%

**Table 2. results of antidepressant activity of oral administration of the compounds of the present invention in mice**

| Compound | 1h inhibition (%) | 24h inhibition (%) |
|---|---|---|
| 52 | 29.5±1.1 | 25.6±2.1 |
| 54 | 33.0±2.5 | 38.7±3.3 |
| 57 | 42.2±1.8 | 36.5±3.0 |
| 65 | 26.4±1.1 | 37.9±2.3 |
| 68 | 26.2±4.1 | 37.4±0.7 |
| 71 | 42.4±0.4 | 40.2±1.6 |
| 73 | 68.7±0.6 | 39.4±4.4 |
| 76 | 65.2±0.8 | 47.1±3.2 |
| 77 | 69.8±1.6 | 44.3±3.5 |
| Fluoxetine | 2.4±1.1 | 2.3±3.1 |
| S-ketamine | 20.7±3.7 | 23.6±1.0 |
| (2R,6R)-HNK | 21.1±1.4 | 24.8±6.9 |
| 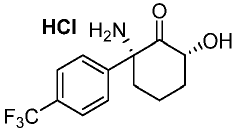 Comparative compound 1 | 30.6±3.1 | 35.0±0.3 |
| 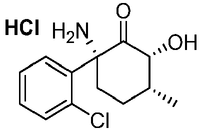 Comparative compound 3 | 22.6±2.7 | 23.7±3.2 |
| 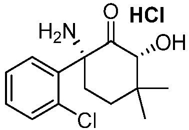 Comparative compound 4 | 2.9±1.0 | -17.2±2.0 |
| 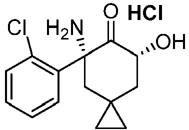 Comparative compound 5 | 22.8±2.5 | 19.1±2.1 |
| 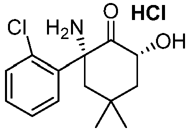 Comparative compound 6 | 18.2±1.6 | 11.26±2.0 |

Ketamine has a poor oral absorption and poor oral efficacy, so it is administered by injection when clinically used as an anesthetic. And at present, when clinically used for rapid antidepressant treatment, the nasal route of drug administration is adopted, which brings inconvenience to clinical use.

As can be seen from Table 2, the traditional antidepressant fluoxetine oral administrated in the forced swim test, 1 hour and 24 hours did not show antidepressant effect, indicating that fluoxetine does not have a rapid onset of antidepressant characteristics, which is consistent with the clinical results. In contrast, the oral administration of the compounds of the present invention significantly reduced the immobility time of forced swimming in mice for both 1h and 24h, showing rapid, long-lasting and potent antidepressant effects.

The compounds of the present invention have a significant advantage of substantially increased antidepressant activity relative to the positive control drugs S-Ketamine, (2R,6R)-HNK and each of the comparative compounds.

The compounds of the present invention are orally effective and can be made into oral dosage forms in the future which is another significant advantage of the compounds in the present invention over ketamine.

### Test Example 2. Brain tissue distribution study of compounds of the present

### invention in rats

Healthy male SD rats, randomly grouped with 3 rats in each group, were given the test compounds by gavage. The specific scheme is shown in Table 3 below:

**Table 3. Administration scheme for brain tissue distribution experiment in SD rats:**

| Compound | Mode of administration | Dose(mg/kg) | Delivery volume(ml/kg) |
|---|---|---|---|
| 2R,6R-HNK | Gavage | 10 | 10 |
| 73 | Gavage | 10 | 10 |

Each test compound was dissolved in saline to form a certain concentration solution for administration.

Fasting but not abstaining from water for 12-14 hours on the day before administration, and food was given 4h after administration.

Brain tissues were collected after the animals were bled, cleaned, and homogenized with 50% ice methanol 1:4 (m/v). The collection time points were 5 min, 10 min, 30 min, and 2 h after the administration of subjects; 30.0 µL of tissue samples were taken (the samples were taken from the refrigerator at -80 °C and vortexed for 30 s after natural dissolution at room temperature) into 1.5 mL centrifugal tubes, and 300.0 µL of the internal standard solution (30 ng/mL buspirone in acetonitrile) was added; the samples were vortexed for 60 s, and centrifuged for 3 min (the centrifugal force was 12,000 rpm). 75 µL of the supernatant was transferred to a 96-well plate containing an equal volume of water, shaken to mix well and then sampled for LC-MS/MS analysis, the sample volume is 10 µL.

**Table 4. Results of brain tissue distribution studies in rats**

| **Compound** | **Dose (mg/kg)** | **tissue** | **Cmax(plasma: ng/mL; brain: ng/g)** | **AUC₀₋ₜ(plasma: h•ng•mL ⁻¹; brain: h•ng•g⁻¹)** | **brain / plasma ratio** |
|---|---|---|---|---|---|
| 2R,6R-HNK | 10(gavage) | brain | 1495 | 2480 | 0.95 |
| | | plasma | 1480 | 2610 | |
| 73 | 10(gavage) | brain | 5567 | 6172 | 3.40 |
| | | plasma | 1600 | 1817 | |

As a drug for the treatment of depression, a disease of the central nervous system, drug distribution into the CNS system such as brain tissue is necessary, and the amount of drug entering the brain tissue directly determines its therapeutic effect. As shown in Table 4, the maximum drug concentration (Cmax) in the brain of the compounds of the present invention was more than three-fold higher than that of 2R,6R-HNK; and the brain drug exposure (AUC0-t) increased more than two-fold.

Meanwhile, although the compounds of the present invention have high brain drug exposure, they have lower plasma drug exposure compared to 2R,6R-HNK, i.e., the compounds of the present invention have a significantly higher brain/plasma ratio compared to 2R,6R-HNK. While, the significantly higher brain/plasma ratio also predicts a potentially lower risk of peripheral side effects and a better safety.

### Test example 3. in vivo pharmacokinetic experiments of the compound of the present invention in beagle dogs

Healthy male beagle dogs, randomly grouped with 3 dogs in each group, were given the test compound by gavage or intravenous injection. The specific arrangement is shown in Table 5 below:

**Table 5. Administration scheme for in vivo pharmacokinetic experiment in beagle dog:**

| Compound | Mode of administration | Dose(mg/kg) | Delivery volume(ml/kg) |
|---|---|---|---|
| 73 | gavage | 5 | 5 |
| 73 | intravenous | 1 | 2.5 |

Each test compound was dissolved in saline to form a certain concentration solution for administration.

Fasting but not abstaining from water for 12-14 hours on the day before administration, and food was given 4h after administration; no fasting was done for the intravenous experiments.

Each animal was sampled with 0.6 mL of blood each time through the jugular vein and anticoagulated with EDTAK₂. The collection time points were 5 min, 10 min, 15 min, 30 min, 1 h, 2 h, 4 h, 6 h, 8h, and 24h after administration of the subject in group PO; group IV: 2 min, 5 min, 15 min, 30 min, 1 h, 2 h, 4 h, 6 h, 24h after administration of the subject. Blood samples were placed on ice after collection and centrifuged to separate plasma within 30 min (centrifugation conditions: 5000 rpm, 10 min, 4°C). The samples were stored at -80°C before analysis.

**Table 6, Pharmacokinetic data in beagle dogs:**

| **Compou nd** | **Dose (mg/k g)** | **T_{1/2} (h)** | **Tₘₐₓ (h)** | **Cₘₐₓ (ng/mL g)** | **AUC₀₋ₜ (ngh/m L)** | **AUC_{0-∞} (ngh/m L)** | **MRT (h)** | **CL (ml/min/ Kg)** | **Vss (L/Kg)** | **F (% g)** |
|---|---|---|---|---|---|---|---|---|---|---|
| **73** | 1 (i.v.) | 1.61±0. 33 | / | / | 941±18 6 | 970±20 4 | 1.84±0. 2 | 17.66±3.5 2 | 1.93±0 .2 | / |
| | 5 (P.O.) | 2.33±1. 5 | 0.42±0. 14 | 1016±2 62 | 2179±3 84 | 2224±3 73 | 2.57±0. 82 | / | / | 46. 3 |

As can be seen from the dog pharmacokinetic data in Table 6, the compounds of the present invention are rapidly absorbed orally with good exposure and bioavailability and exhibit good metabolic properties.

### Test Example 4. Evaluation of solid-state chemical stability of compounds of the present invention

Like the properties of in vitro and in vivo activity and metabolism, etc., chemical stability is also crucial in determining whether a drug candidate can be successfully developed (Di L, Kerns EH. Stability challenges in drug discovery. Chem Biodivers. 2009, Nov; 6(11): 1875-1886.). The stability of chemical drugs is the basis for quality control and is closely related to the safety and therapeutic effectiveness of drugs. Chemically stable compound not only ensure the safety of patients and provide good therapeutic effects, but also simplify the subsequent formulation development and storage conditions, extend the shelf-life of the drug after its release, as well as facilitate the daily use of the drug by patients. Therefore, it is particularly important to enhance and improve the stability of candidate compounds. Because of the importance of chemical stability in drug discovery and development, the International Coordination Committee for the Registration of Pharmaceuticals for Human Use (ICH) has formulated technical guidelines to regulate the corresponding studies.

Each sample was sealed and stored in a low-density polyethylene bag as the inner package and an aluminum foil bag as the outer package, and then analyzed by liquid chromatography after being stored for a certain period of time under different temperature/humidity conditions (40 °C, 75% relative humidity; 25 °C, 60% relative humidity), comparing the solid-state chemical stability of the compounds of the present invention and the comparative compounds, which reflected the superior solid-state chemical stability of the compounds of the present invention.

The HPLC conditions were as follows:

### Method 1:

Configuration of test solution: appropriate amount of sample was dissolved and diluted with a diluent (0.03 mol/L phosphate buffer-acetonitrile (80:20)) to make 1mg/mL solution.

Chromatographic conditions: according to the high performance liquid chromatography (Chinese Pharmacopoeia 2020 version of the four general 0512) test, using octadecyl-bonded silica gel (Agilent Eclipse Plus C18, 4.6 mm * 250 mm, 5.0 µm, P / N for 959990-902 or a chromatographic column with equivalent performance) as a filler; 0.03 mol / L phosphate buffer (4.08g of potassium dihydrogen phosphate was dissolved in 1000ml of water and shaken well) was used as mobile phase A and acetonitrile as mobile phase B at a flow rate of 1 ml/min, the column temperature was 30 °C, the detection wavelength was 220 nm, the injection volume was 10 ul, and the sample plate was controlled to 8 °C and running for 65 min. Gradient elution was performed according to the following table:

| Time (min) | Mobile phase A (%) | Mobile phase B (%) |
|---|---|---|
| 0 | 88 | 12 |
| 2 | 88 | 12 |
| 27 | 75 | 25 |
| 47 | 25 | 75 |
| 57 | 25 | 75 |
| 58 | 75 | 12 |
| 65 | 75 | 12 |

### Method 2:

Configuration of test solution: appropriate amount of the product was dissolved and diluted with a diluent (0.1% phosphoric acid-acetonitrile (80:20)) to make 1mg/mL solution.

Chromatographic conditions: according to the high performance liquid chromatography (Chinese Pharmacopoeia 2020 version of the four general 0512) test, using octadecyl bonded silica gel (Agilent Eclipse Plus C18, 4.6mm * 250mm, 5.0µm, PIN 959990-902 or a chromatographic column with equivalent performance) as a filler; 0.1% phosphate buffer (1 ml of phosphoric acid in 1000 ml of water and shake well) as mobile phase A, acetonitrile as mobile phase B, flow rate of 1 ml/min, column temperature of 30 °C, detection wavelength of 220 nm, injection volume of 10 ul, running for 48 minutes. Gradient elution was performed according to the following table:

| Time (min) | Mobile phase A (%) | Mobile phase B (%) |
|---|---|---|
| 0 | 95 | 5 |
| 3 | 95 | 5 |
| 10 | 75 | 25 |
| 25 | 55 | 45 |
| 35 | 10 | 90 |
| 40 | 10 | 90 |
| 40.1 | 95 | 5 |
| 48 | 95 | 5 |

**Table 7. Stability results of solid state compounds under different storage conditions of the compounds of the present invention**

| Compound | Storage condition | 0 Days (%) | 5 Days (%) | 10 Days (%) | 30 Days (%) | 60 Days (%) | 180 Days (%) |
|---|---|---|---|---|---|---|---|
| 2R,6R-HNK | 40°C, 75%RH | 99.18 | 98.54 | 97.85 | | | |
| 69 | 40°C, 75%RH | 98.36 | | 98.43 | 98.34 | | |
| 70 | 40°C, 75%RH | 97.84 | | 97.84 | 97.82 | | |
| 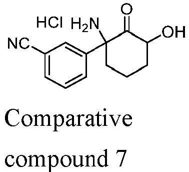 | 40°C, 75%RH | 98.1 | 87.5 | | | | |
| 47 | 40°C, 75%RH | 98.89 | 98.86 | - | 98.86 | - | |
| 63 | 40°C, 75%RH | 99.67 | 99.66 | 99.66 | 99.66 | - | |
| 64 | 40°C, 75%RH | 99.88 | 99.88 | 99.89 | 99.85 | | |
| 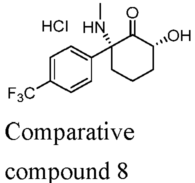 | 40°C, 75%RH | 98.4 | 73.0 | | | | |
| 71 | 40°C, 75%RH | 99.67 | 99.46 | 99.46 | 99.45 | | |
| 72 | 40°C, 75%RH | 99.69 | 99.66 | 99.62 | 99.61 | | |
| 73 | 40°C, 75%RH | 99.94 | 99.90 | 99.88 | 99.89 | | |
| 74 | 40°C, 75%RH | 99.26 | | 99.29 | 99.12 | | |
| 75 | 40°C, 75%RH | 99.85 | | 99.78 | 99.77 | | |
| 76 | 40°C, 75%RH | 99.88 | | 99.65 | 99.84 | | |
| 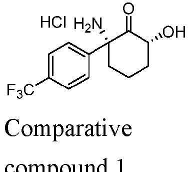 | 40°C, 75%RH | 99.98 | 98.94 | 98.74 | 95.88 | 91.57 | |
| 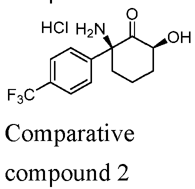 | 40°C, 75%RH | 99.48 | | 97.11 | 94.26 | 89.97 | |
| 52 | 40°C, 75%RH | 99.89 | | 99.89 | 99.90 | 99.88 | 99.82 |
| 54 | 40°C, 75%RH | 99.27 | | 99.23 | | 99.20 | |
| 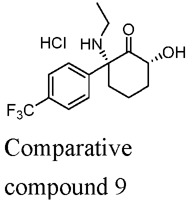 | 25°C, 60%RH | 98.2 | | 96.8 | 61% | | |
| 77 | 25°C, 60%RH | 99.70 | | 99.69 | 99.70 | | |
| 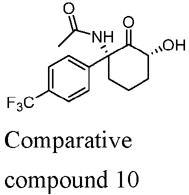 | 25°C, 60%RH | 99.77 | | 99.69 | 97.51 | | |
| 37 | 25°C, 60%RH | 99.69 | | 99.65 | 99.69 | | |
| 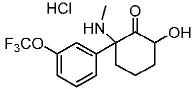 | 40°C, 75%RH | 98.15 | 87.8 | 75.44 | | | |
| 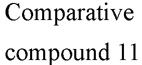 | | | | | | | |
| 67 | 40°C, 75%RH | 99.82 | | 99.73 | 99.57 | | |
| 68 | 40°C, 75%RH | 99.12 | 99.11 | 99.12 | | | |
| 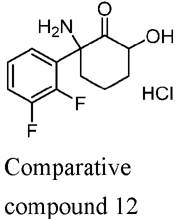 | 40°C, 75%RH | 97.16 | | | 94.59 | | |
| 48 | 40°C, 75%RH | 99.25 | 99.28 | 99.28 | 99.17 | | |
| 65 | 40°C, 75%RH | 99.54 | 99.37 | 99.32 | 99.22 | | |
| 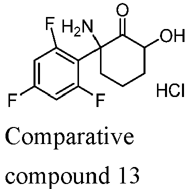 | 40°C, 75%RH | 98.31 | | 97.05 | 96.04 | | |
| 44 | 40°C, 75%RH | 98.45 | 98.53 | | 98.35 | | |
| 58 | 40°C, 75%RH | 99.34 | | 99.12 | 99.14 | | |
| 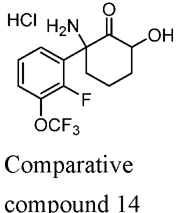 | 40°C, 75%RH | 98.58 | | | 93.96 | | |
| 43 | 40°C, 75%RH | 98.73 | | | 98.75 | | |
| 56 | 40°C, 75%RH | 99.61 | | | 99.50 | | |

As shown in Table 7, compared to the comparative compounds (only hydroxyl at the carbonyl α-position), the compounds of the present invention (additional introduction of a methyl at the carbonyl α-position) had a significant improved solid state chemical stability and exhibit obvious advantages, demonstrating better developability and druggability.

All literatures mentioned in the present invention are incorporated by reference herein, as though individually incorporated by reference. Additionally, it should be understood that after reading the above teaching, many variations and modifications may be made by those skilled in the art, and these equivalents also fall within the scope as defined by the appended claims.

## Claims

1. A compound of formula (I), or a tautomer, an enantiomer, a diastereoisomer, a racemate, or a mixture thereof, or a pharmaceutically acceptable salt thereof, 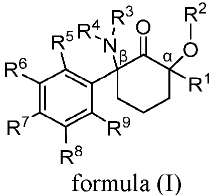 wherein,
R¹ is selected from the group consisting of deuterium and C₁-C₆ alkyl;
R², R³ and R⁴ are each independently selected from the group consisting of hydrogen, C₁-C₆ alkyl, deuterated C₁-C₆ alkyl and -(C=O)-C₁-C₆ alkyl;
R⁵, R⁶, R⁷, R⁸ and R⁹ are each independently selected from the group consisting of hydrogen, C₁-C₆ alkyl, halogenated C₁-C₆ alkyl, C₁-C₆ alkoxy, halogenated C₁-C₆ alkoxy, halogen, nitro and cyano; and at least 2 of R⁵, R⁶, R⁷, R⁸ and R⁹ are hydrogen;
the stereo configuration of the α-position or β-position carbon atom is each independently selected from the group consisting of: R, S and (R, S).

2. The compound according to claim 1, or the tautomer, the enantiomer, the diastereoisomer, the racemate, or the mixture thereof, or the pharmaceutically acceptable salt thereof, wherein the compound is a compound according to formula (I-A), (I-B), (I-C), (I-D), (I-E), (I-F) or (I-G): 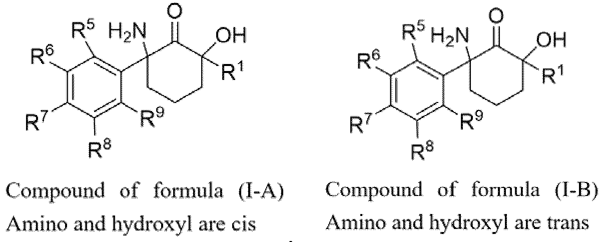
in formula (I-A), (I-B), R¹, R⁵, R⁶, R⁷, R⁸ and R⁹ are as defined in claim 1; 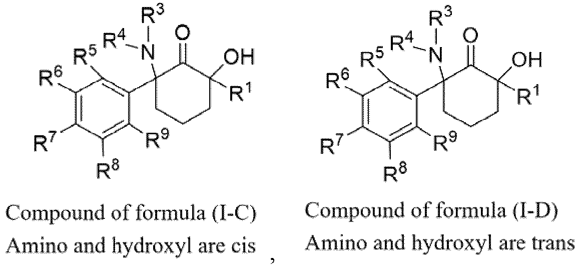
in formula (I-C), (I-D), R¹, R⁵, R⁶, R⁷, R⁸ and R⁹ are as defined in claim 1, R³ and R⁴ are independently hydrogen, C₁-C₆ alkyl, or deuterated C₁-C₆ alkyl, and R³ and R⁴ are not both hydrogen at the same time; 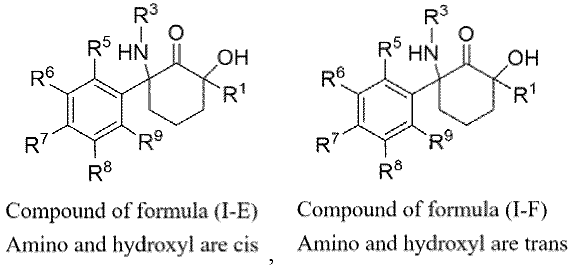
in formula (I-E), (I-F), R¹, R⁵, R⁶, R⁷, R⁸ and R⁹ are defined in claim 1 and R³ is - (C=O)-C₁-C₆ alkyl; 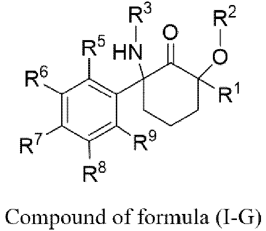
in formula (I-G), R¹, R⁵, R⁶, R⁷, R⁸ and R⁹ are as defined in claim 1; R² is selected from the group consisting of C₁-C₆ alkyl, deuterated C₁-C₆ alkyl and -(C=O)-Ci-Ce alkyl; R³ is selected from the group consisting of hydrogen, C₁-C₆ alkyl and deuterated C₁-C₆ alkyl.

3. The compound according to claim 1, or the tautomer, the enantiomer, the diastereoisomer, the racemate, or the mixture thereof, or the pharmaceutically acceptable salt thereof, wherein, R¹ is deuterium or methyl.

4. The compound according to claim 1, or the tautomer, the enantiomer, the diastereoisomer, the racemate, or the mixture thereof, or the pharmaceutically acceptable salt thereof, wherein R², R³ and R⁴ are independently hydrogen, methyl, deuteromethyl, ethyl, isopropyl, acetyl, or propionyl.

5. The compound according to claim 1, or the tautomer, the enantiomer, the diastereoisomer, the racemate, or the mixture thereof, or the pharmaceutically acceptable salt thereof, wherein R² is hydrogen; R³ is hydrogen;
R⁴ is methyl, deuteromethyl, ethyl, isopropyl, acetyl or propionyl.

6. The compound according to claim 1, or the tautomer, the enantiomer, the diastereoisomer, the racemate, or the mixture thereof, or the pharmaceutically acceptable salt thereof, wherein the compound is selected from the group consisting of:

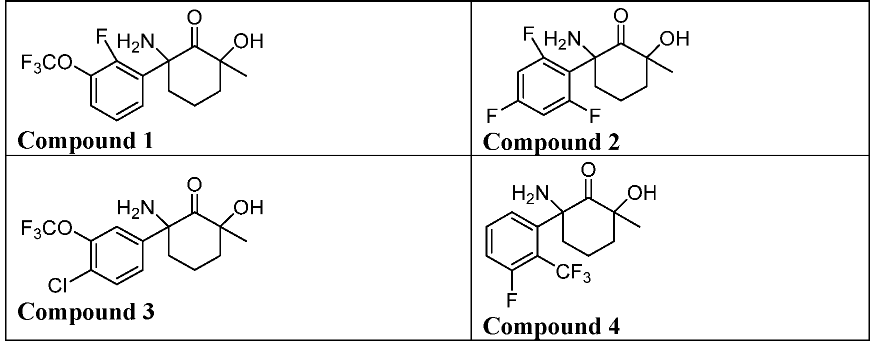

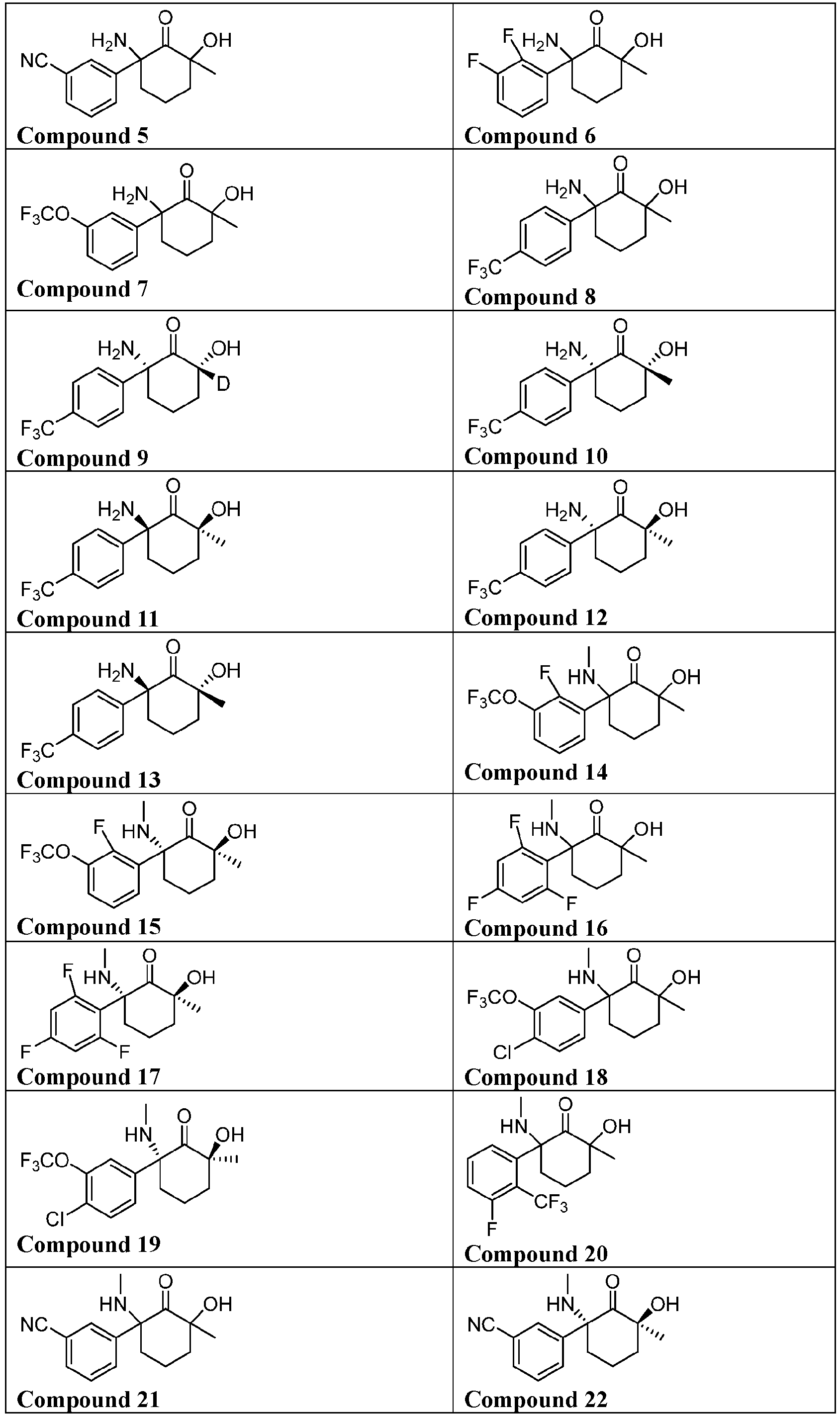

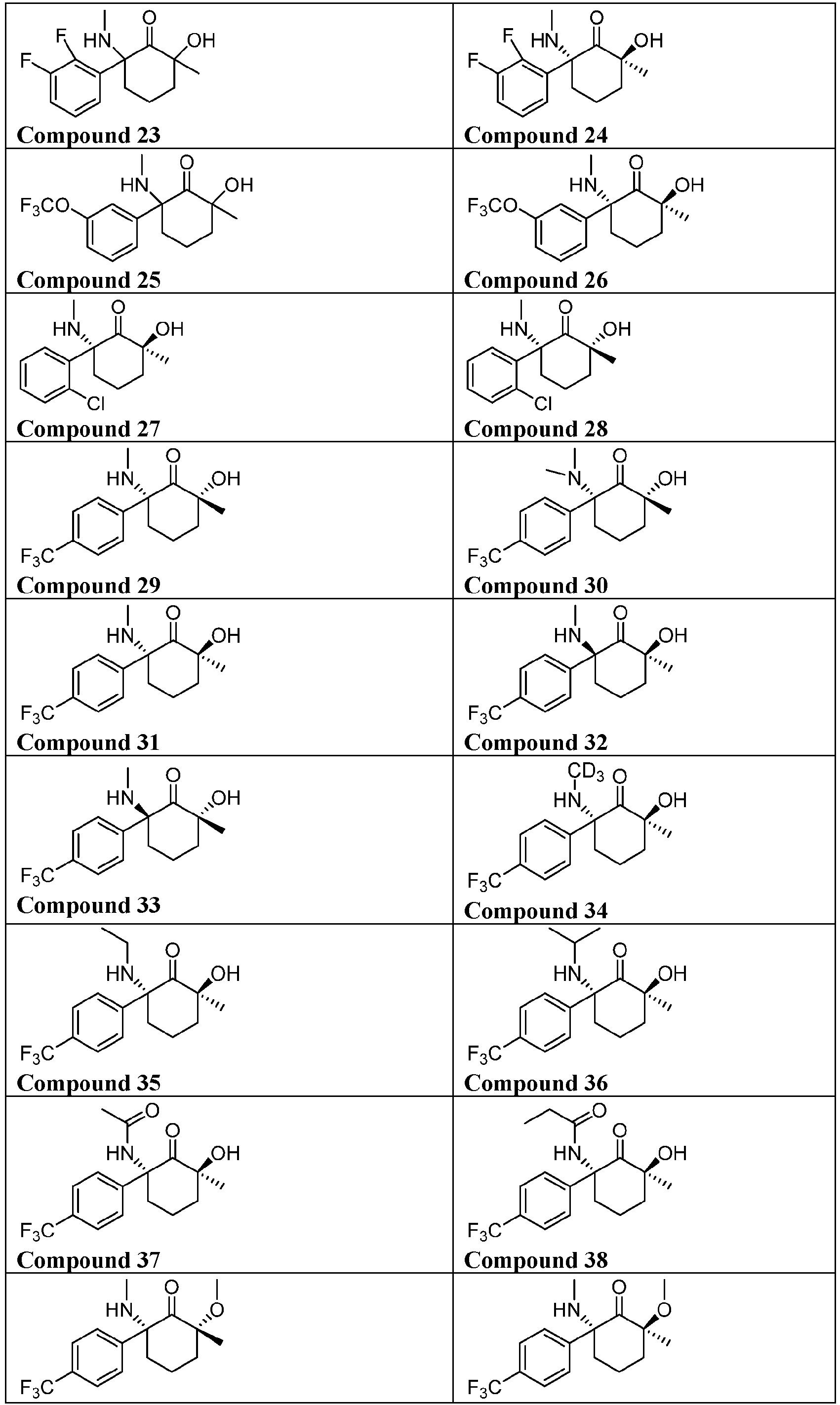

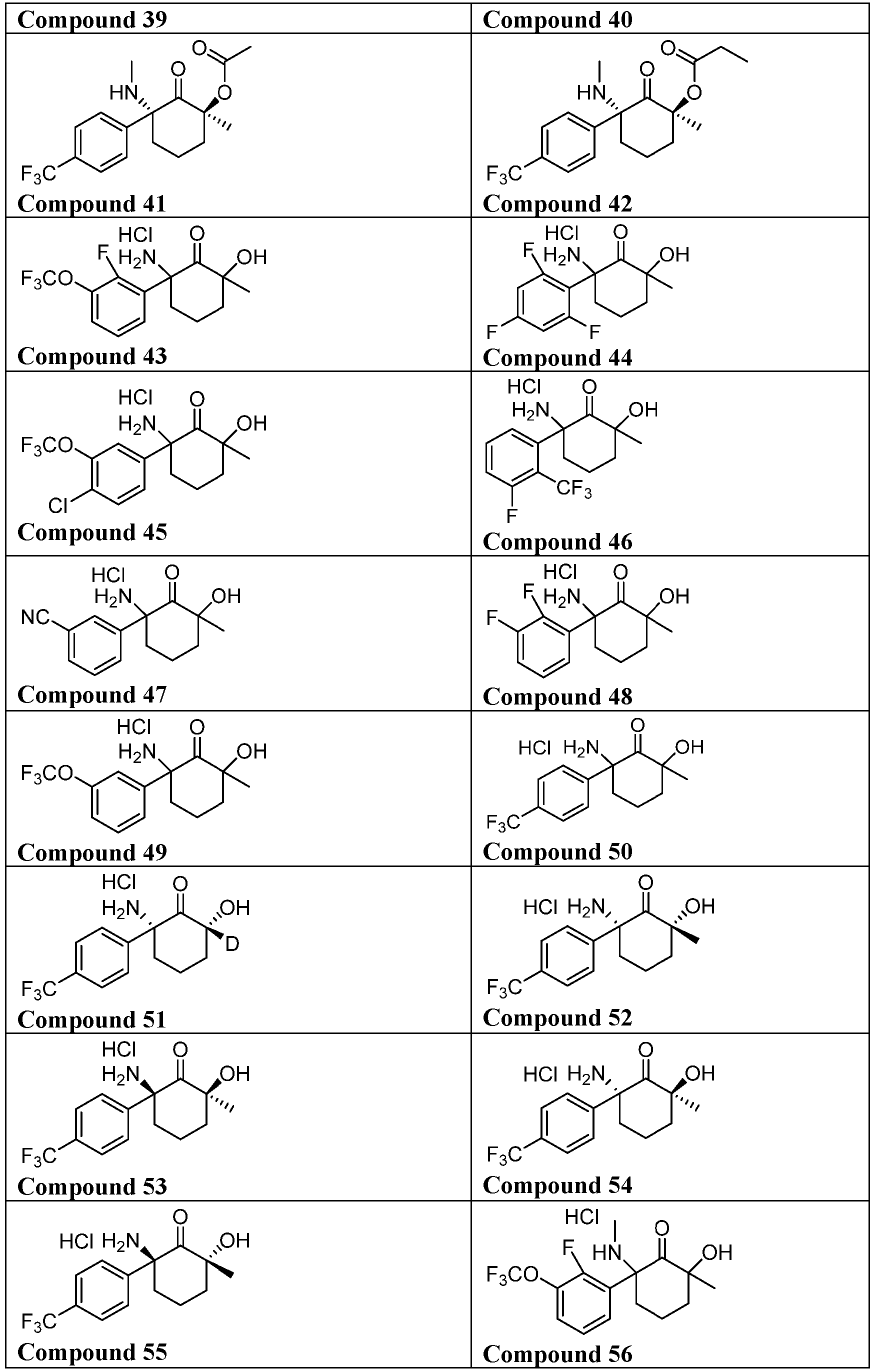

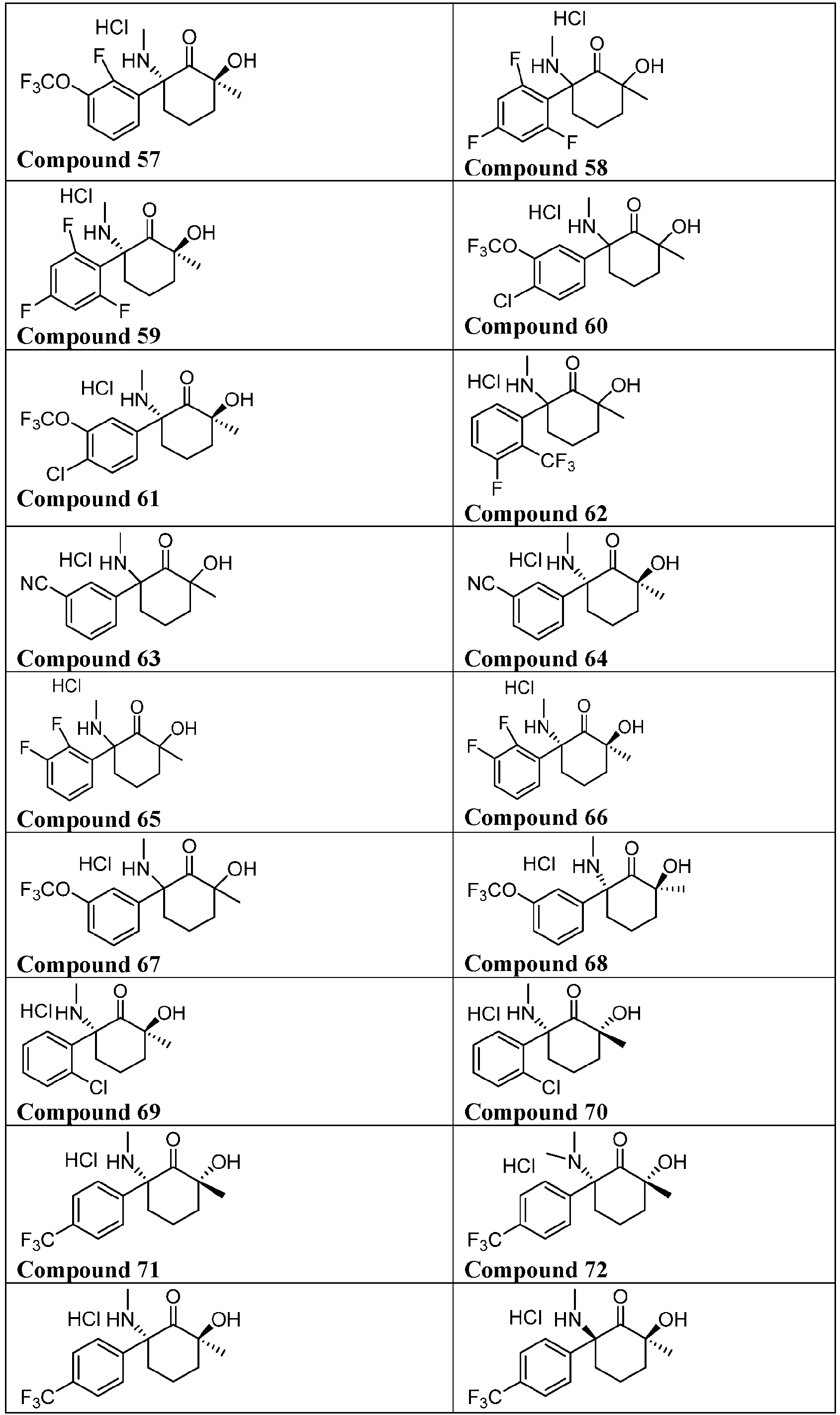

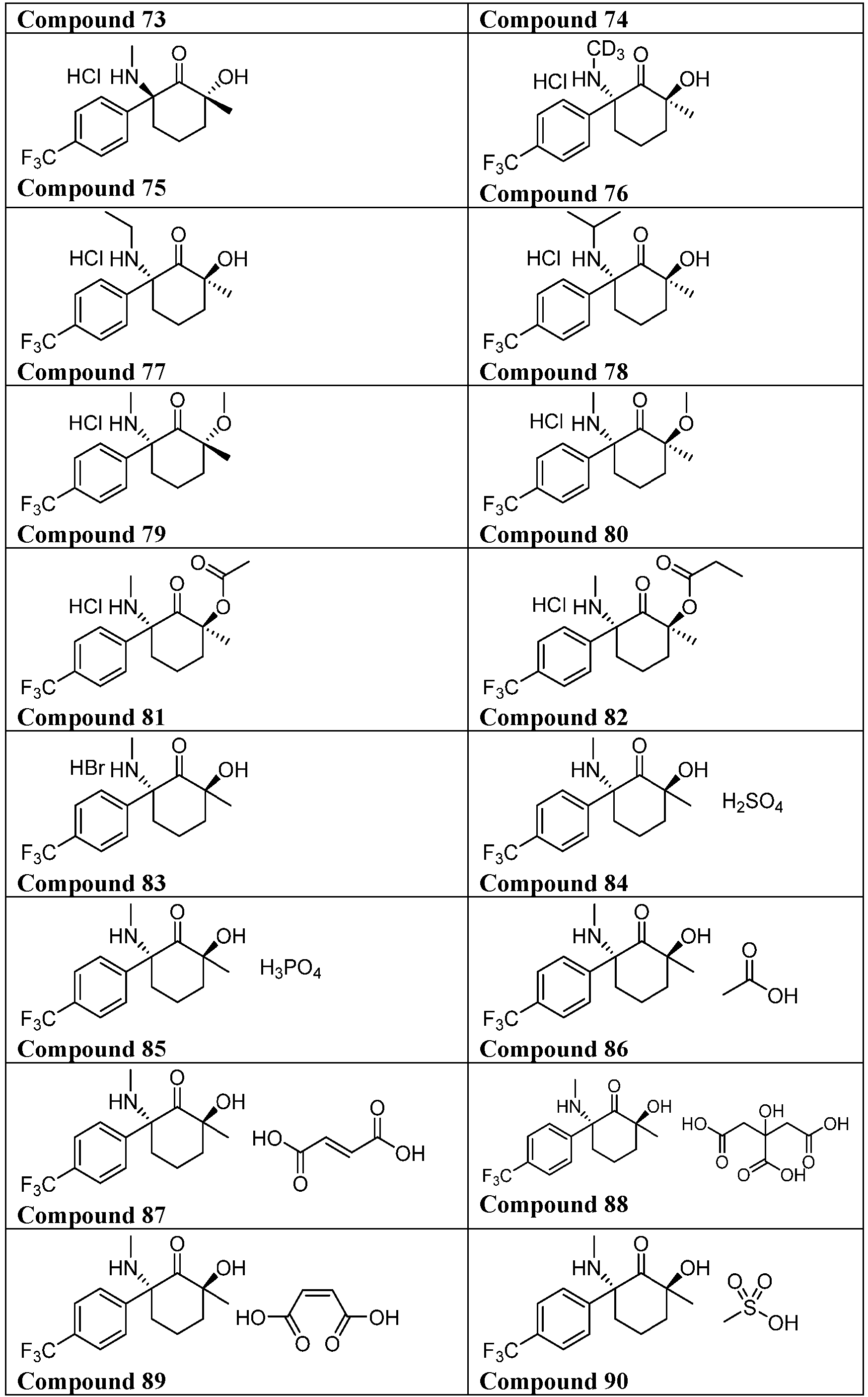

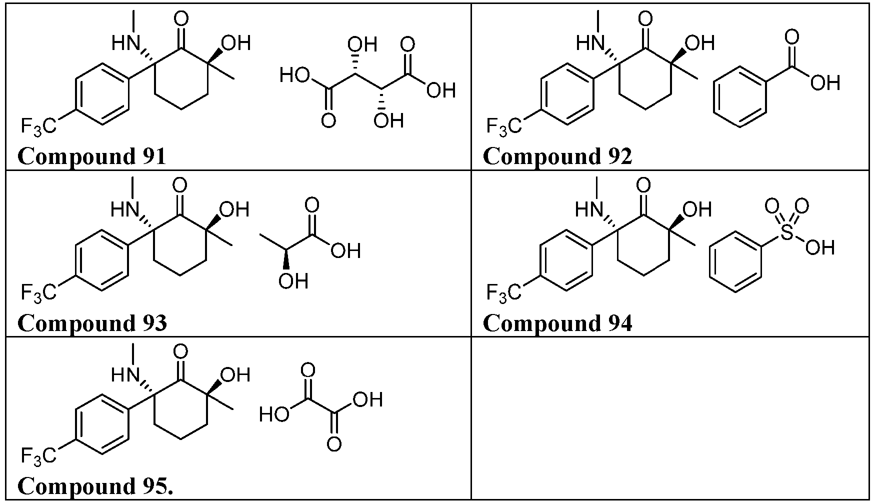

7. A pharmaceutical composition, comprising:
1) one or more safe and effective amounts of the compound of claim 1, or the tautomer, the enantiomer, the diastereoisomer, the racemate, or the mixture thereof, or the pharmaceutically acceptable salt thereof; and
2) a pharmaceutically acceptable carrier or excipient.

8. A method for the preparation of the compound of claim 1, or the tautomer, the enantiomer, the diastereoisomer, the racemate, or the mixture thereof, or the pharmaceutically acceptable salt thereof, wherein
(1) the method comprises steps of: 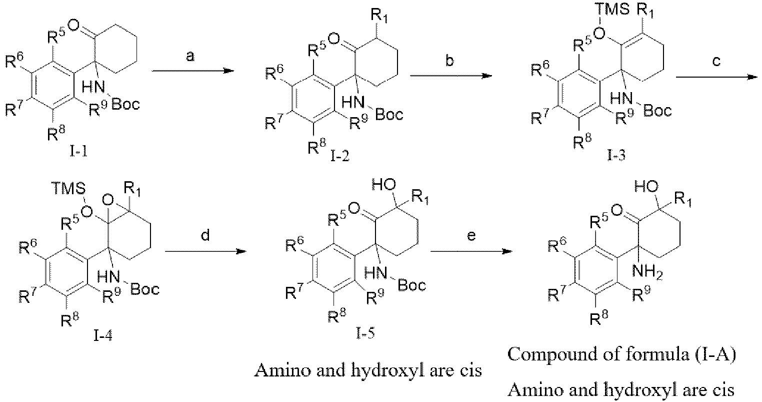 wherein, R¹, R⁵, R⁶, R⁷, R⁸ and R⁹ are as defined in claim 1;
a. in a non-protonic solvent, compound I-1 reacts with a deuterated reagent or an alkylating reagent to form compound I-2;
b. in a non-protonic solvent, compound I-2 reacts with a trimethylchlorosilane to form compound I-3;
c. in a non-protonic solvent, compound I-3 and an oxidizing agent undergo oxidation to form compound I-4;
d. in a non-protonic solvent, compound I-4 deprotects the trimethylsilyl protecting group to form compound I-5;
e. in a polar non-protonic solvent, compound I-5 deprotects the tert-butoxycarbonyl protecting group to form compound I-A;
or (2) the method comprises steps of: 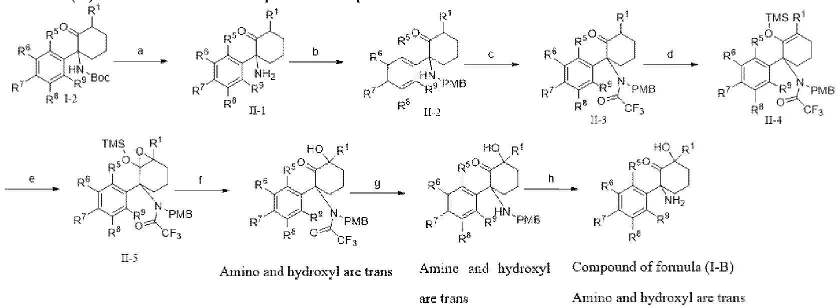 wherein, R¹, R⁵, R⁶, R⁷, R⁸ and R⁹ are as defined in claim 1;
a. in a polar non-protonic solvent, compound I-2 deprotects the tert-butoxycarbonyl protecting group to form compound II-1;
b. in a non-protonic solvent, compound II-1 reacts with p-methoxybenzyl chloride to form compound II-2;
c. in a non-protonic solvent, Compound II-2 reacts with trifluoroacetic anhydride to form Compound II-3;
d. in a non-protonic solvent, Compound II-3 reacts with trimethylchlorosilane to form Compound II-4;
e. in a non-protonic solvent, Compound II-4 and an oxidizing agent undergo oxidation to form Compound II-5;
f. in a non-protonic solvent, compound II-5 deprotects the trimethylsilyl protecting group to form compound II-6;
g. in proton solvent, compound II-6 removes trifluoroacetyl group to form compound II-7;
h. in acidic conditions, Compound II-7 removes p-methoxybenzyl to form Compound I-B;
or (3) the method comprises steps of: 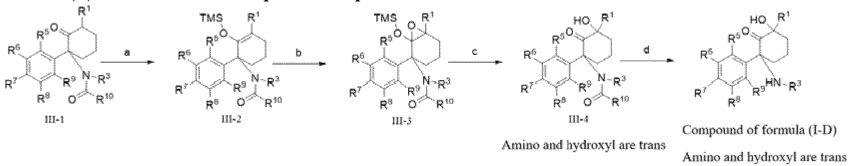 wherein, R¹, R⁵, R⁶, R⁷, R⁸ and R⁹ are as defined in claim 1, R³ is C₁-C₆ alkyl or deuterated C₁-C₆ alkyl, and R¹⁰ is C₁-C₆ alkyl, halogenated C₁-C₆ alkyl, C₁-C₆ alkoxy or benzyloxy;
a. in a non-protonic solvent, compound III-1 reacts with trimethylchlorosilane to form compound III-2;
b. in a non-protonic solvent, Compound III-2 and an oxidizing agent undergo oxidation to form Compound III-3;
c. in a non-protonic solvent, compound III-3 deprotects the trimethylsilyl protecting group to form compound III-4;
d. in a proton solvent or a non-proton solvent, Compound III-4 deprotects the R¹⁰ carbonyl protecting group to form Compound I-D;
or (4) The method comprises steps of: 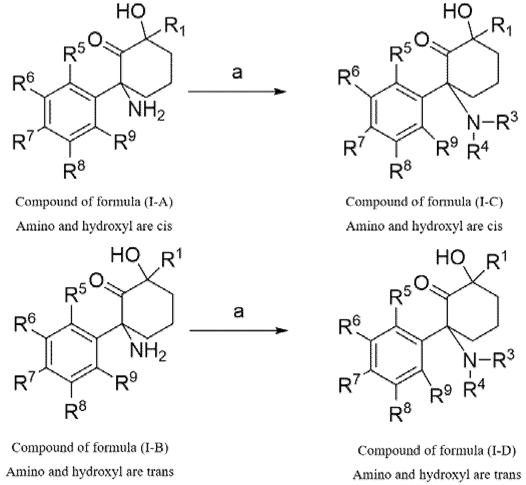 wherein, R¹, R⁵, R⁶, R⁷, R⁸ and R⁹ are as defined in claim 1, R³ and R⁴ are independently hydrogen, C₁-C₆ alkyl or deuterated C₁-C₆ alkyl, and R³ and R⁴ are not both hydrogen at the same time;
a. in a protonated solvent or a non-protonated solvent, compound I-A or I-B react with the corresponding aldehyde or ketone to form the corresponding compound I-C or I-D;
or (5) the method comprises steps of: 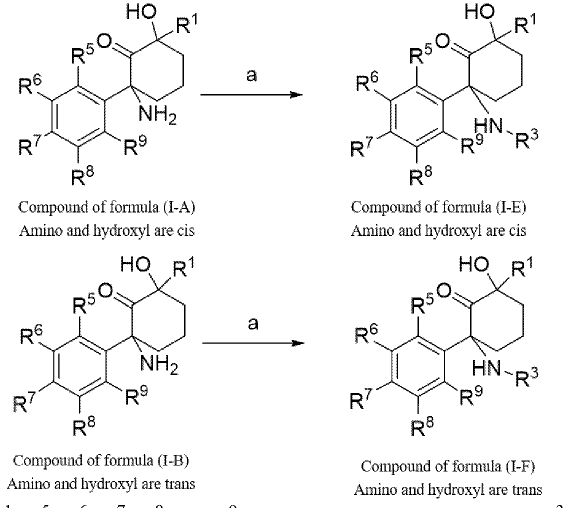 wherein, R¹, R⁵, R⁶, R⁷, R⁸ and R⁹ are as defined in claim 1 and R³ is -C=O-C₁-C₆ alkyl;
a. in a non-protonic solvent, compound I-A or I-B react with a corresponding acyl chloride or anhydride to form the corresponding compound I-E or I-F;
or (6) the method comprises steps of: 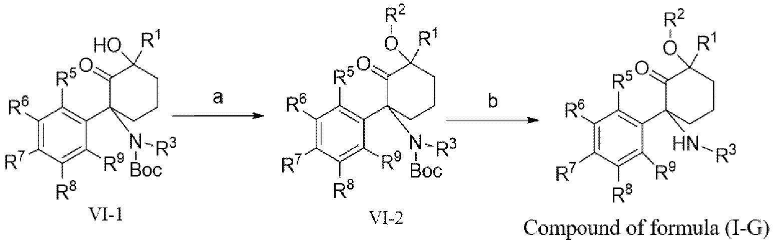 wherein, R¹, R⁵, R⁶, R⁷, R⁸ and R⁹ are as defined in claim 1; R² is C₁-C₆ alkyl, deuterated C₁-C₆ alkyl or -(C=O)-Ci-Ce alkyl; R³ is hydrogen, C₁-C₆ alkyl or deutero-C₁-C₆ alkyl;
a. in a non-protonic solvent, compound VI-1 reacts with a corresponding alkylating reagent, deuterated alkylating reagent, acyl chloride or anhydride to form the corresponding compound VI-2;
b. in a polar non-protonic solvent, compound VI-2 deprotects the tert-butoxycarbonyl protecting group to form compound I-G.

9. A use of the compound of claim 1, or the tautomer, the enantiomer, the diastereoisomer, the racemate, or the mixture thereof, or the pharmaceutically acceptable salt thereof in the preparation of a medication for the treatment of neurological related disorders.

10. The use according to claim 9, wherein the neurological related disorders are selected from the group consisting of depression, post-traumatic stress disorder, obsessive-compulsive disorder, anxiety and pain.
